# EUROPEAN PATENT SPECIFICATION

(11) **EP 0 906 448 B1**
(45) Date of publication and mention of the grant of the patent: **25.02.2009**
(21) Application number: 97920115.9
(22) Date of filing: 03.04.1997
(51) Int. Cl.: C12Q 1/00, G01N 33/53, C07K 1/00, C07K 14/00, C07K 17/00, C07H 21/02, C07H 21/04

(54) **METHODS FOR THE DIAGNOSIS AND PROGNOSIS OF CANCER**
METHODEN ZUR DIAGNOSE UND PROGNOSE VON KREBS
PROCEDES DE DIAGNOSTIC ET DE PRONOSTIC DU CANCER

(30) Priority: 05.04.1996 US 14943 P; 05.06.1996 US 19372 P; 21.06.1996 US 20196 P; 03.03.1997 US 39532 P
(43) Date of publication of application: 07.04.1999
(60) Divisional application: 08009585.4
(73) Proprietor: Giordano, Antonio, Philadelphia, Pennsylvania 19128 (US)
(72) Inventor: GIORDANO, Antonio, Philadelphia, PA 19128 (US); BALDI, Alfonso, I-80128 Naples (IT)
(74) Representative: Goulard, Sophie
(86) International application number: PCT/US1997/005598
(87) International publication number: WO 1997/038125

(56) References cited:
- EP-A- 0 390 530
- EP-A- 0 571 911
- WO-A-92/15602
- WO-A-92/21771
- WO-A-93/06244
- WO-A-94/11531
- WO-A-95/02328
- WO-A-95/24503
- CLAUDIO P P ET AL: "P120/PRB2 HAS GROWTH SUPPRESSIVE PROPORTIES SIMILAR TO YET DISTINCTIVE FROM THOSE OF RETINOBLASTOMA FAMILY MEMBERS PRB AND P107" CANCER RESEARCH, AMERICAN ASSOCIATION FOR CANCER RESEARCH, BALTIMORE, MD, US, vol. 54, no. 21, 1 November 1994 (1994-11-01), pages 5556-5560, XP002910339 ISSN: 0008-5472
- DEPASQUALE S E ET AL: "DIFFERENTIAL EXPRESSION OF THE RB2 TUMOR SUPPRESSOR GENE IN HUMAN EPITHELIAL OVARIAN CARCINOMA" LABORATORY INVESTIGATION, UNITED STATES AND CANADIAN ACADEMY OF PATHOLOGY, BALTIMORE,, US, vol. 76, no. 1, 1 March 1997 (1997-03-01), page 98A, XP008008523 ISSN: 0023-6837
- HANNON G J ET AL: "Isolation of the Rb-related p130 through its interaction with CDK2 and cyclins." GENES & DEVELOPMENT. DEC 1993, vol. 7, no. 12A, December 1993 (1993-12), pages 2378-2391, XP009034493 ISSN: 0890-9369
- HONG F D ET AL: "Structure of the human retinoblastoma gene." PROCEEDINGS OF THE NATIONAL ACADEMY OF SCIENCES OF THE UNITED STATES OF AMERICA. JUL 1989, vol. 86, no. 14, July 1989 (1989-07), pages 5502-5506, XP001182757 ISSN: 0027-8424
- XU H J ET AL: "Altered retinoblastoma protein expression and prognosis in early-stage non-small-cell lung carcinoma." JOURNAL OF THE NATIONAL CANCER INSTITUTE. 4 MAY 1994, vol. 86, no. 9, 4 May 1994 (1994-05-04), pages 695-699, XP009034424 ISSN: 0027-8874
- PICARDO A L ET AL: "Quantitative analysis of carcinoembryonic antigen, squamous cell carcinoma antigen, CA 125, and CA 50 cytosolic content in non-small cell lung cancer" CANCER 1994 UNITED STATES, vol. 73, no. 9, 1994, pages 2305-2311, XP009034800 ISSN: 0008-543X
- KHALIFA A ET AL: "The value of tumor markers in the diagnosis, staging and prognosis of lung cancer" CANCER MOLECULAR BIOLOGY 1995 EGYPT, vol. 2, no. 3, 1995, pages 541-547, XP009034802 ISSN: 1110-5313
- GENOMICS, 1994, Vol. 20, SLIGHTOM et al., "Nucleotide Sequence Analysis of 77.7 kb of the Human V beta T-Cell Receptor Gene Locus: Direct Primer-Walking Using Cosmid Template DNAs", pages 149-168.
- HUMAN MOLECULAR GENETICS, 1992, Vol. 1, No. 2, THOMAS et al., "A Polymorphic Dinucleotide Repeat in Instron 1 of the Human Tissue Plasminogen Activator Gene", page 138.
- NATURE, 14 February 1991, Vol. 349, HIRATA et al., "Cloning and Expression of cDNA for a Human Thromboyane A2 Receptor", pages 617-620.
- PROC. NATL. ACAD. SCI. U.S.A., February 1990, Vol. 87, STOPPA-LYONNET et al., "Clusters of Intragenic Alu Repeats Predispose the Human C1 Inhibitor Locus to Deleterious Rearrangements", pages 1551-1555.
- J. BIOLOGICAL CHEMISTRY, 25 February 1992, Vol. 267, No. 6, WHITEHEAD et al., "Identification of Novel Members of the Serum Amyloid A Protein Superfamily as Constitutive Apolipoproteins of High Density Lipoprotein", pages 3862-3867.
- NATURE, 03 March 1994, Vol. 368, WILSON et al., "2.2 Mb of Continuous Nucleotide Sequence from Chromosome III of C. Elegans", pages 32-38.
- J. BIOLOGICAL CHEMISTRY, 25 May 1983, Vol. 258, No. 10, WILSON et al., "Human Hypoxanthine-Guanine Phosphoribosyltransferase", pages 6458-6460.
- GENOMICS, 1994, Vol. 21, VORECHOVSKY et al., "Isolation of Cosmid and cDNA Clones in the Region Surrounding the TTK Gene at Xq21.3-q22", pages 517-524.
- GENOMICS, 1994, Vol. 22, ZHENG et al., "Development of 124 Sequence-Tagged Sites and Cytogenetic Localization of 217 Cosmids for Human Chromosome 10", pages 55-67.
- GENES AND DEVELOPMENT, 1993, Vol. 7, No. 12A, LI et al., "The Adenovirus E1A-Associated 130-kD Protein is Encoded by a Member of the Retinoblastoma Gene Family and Physically Interacts with Cyclins A and E", pages 2366-2377.
- J. NATL. CANCER INST., 04 May 1994, Vol. 86, No. 9, XU et al., "Altered Retinoblastoma Protein Expression and Prognosis in Early-Stage Non-Small-Cell Lung Carcinoma", pages 695-699.
- ONCOGENE, 1988, Vol. 2, LIFSHITZ et al., "Bcr Genes and Transcripts", pages 113-117.
- BALDI ET AL.: "Differential expression of the retinoblastoma gene family members pRb/p105, p107, and pRb2/p130 in lung cancer." CLINICAL CANCER RESEARCH, vol. 2, pages 1239-1245, XP002099965
- DJELLOUL ET AL.: "Enterocyte differentiation is compatible with SV40 large T expression and loss of p53 function in human colonic Caco-2 cells- Status of the pRb1 and pRb2 tumor suppressor gene products." FEBS LETTERS, vol. 406, no. 3, 14 April 1997 (1997-04-14), pages 234-242, XP004261925

## Description

### Reference to Government Grant

The invention described herein was supported in part by National Institutes of Health grant RO1 CA60999-01A1. The U.S. government has certain rights in the invention.

### Cross-Reference to Related Applications

This application claims priority from U.S. provisional patent application No. 60/039,532 filed March 3, 1997, U.S. Provisional Application No. 60/020,196 filed June 21, 1996, U.S. Provisional Application No. 60/019,372 filed June 5, 1996 and U.S. Provisional Application No. 60/014,943 filed April 5, 1996.

### Field of the Invention

The invention relates to methods for the identification of individuals at risk for cancer, and for the detection and evaluation of cancers.

### Background of the Invention

### A. The Rb Family of Tumor Suppressors

Many types of human cancer are believed to be caused by an imbalance of growth regulators within a cell. A decrease in negative control growth regulators and/or their deactivation can cause a cancerous condition. Alternatively, an increase in positive control growth regulators can also cause a cancerous condition.

Since the identification of the first tumor suppressor gene, much effort in cancer research has been focused on the identification of new tumor suppressor genes and their involvement in human cancer. Many types of human cancers are thought to develop by a loss of heterozygosity of putative tumor suppressor genes not yet identified (Lasko et al., Annu. Rev. Genetics, 25. 281-296 (1991)) according to Knudson's "two-hit" hypothesis (Knudson, Proc. Natl. Acad. Sci. USA, 68, 820-823 (1971)).

One of the most studied tumor suppressor genes is the retinoblastoma susceptibility gene (Rb), whose gene product (pRb, p105, or pRb/p105) has been shown to play a key role in the regulation of cell division. In interphasic cells, pRb contributes to maintaining the quiescent state of the cell by repressing transcription of genes required for the cell cycle through interaction with transcription factors, such as E2F (Wagner et al. , Nature, 352, 189-190 (1991); Nevins, Science. 258, 424-429 (1992); and Hiebert et al., Genes Develop., 6, 177-185 (1992)). The loss of this activity can induce cell transformation as evidenced by the reversion of the transformed phenotype in pRb cells after replacement of a functional pRb (Huang et al., Science 242 1563-1565 (1988); Bookstein et al., Science, 247: 712-715 (1990); and Sumegi et al., Cell Growth Differ., 1 247-250 (1990)).

Upon entrance into the cell cycle, pRb seems to be phosphorylated by cell cycle-dependent kinases (Lees et al., EMBO J. 10:4279-4290 (1991); Hu et al., Mol. Cell. Biol., 12:971-980 (1992); Hinds et al., Cell, 70:993-1006 (1992); and Matsushime et al., Nature, 35:295-300)) which is thought to permit its dissociation from transcription factors and, hence, the expression of genes required for progression through the cell cycle.

It has been found that the retinoblastoma protein family includes at least three members. Two other proteins, p107, and the recently cloned pRb2/p130. share regions of homology with pRb/p105, especially in two discontinuous domains which make up the "pocket region". Ewen et al.. Cell 66: 1155-1164 (1993); Mayol et al., Oncogene 8: 1561-2566 (1993): Li et al. , Genes Dev. 7: 2366-2377 (1993); and Hannon et al., Genes Dev. 7: 2378-2391 (1993). The pocket domain is required for binding with several viral transforming oncoproteins (Moran, Curr. Opin. Genet. Dev. 3: 63-70 (1993)).

The pRb2/p130 cDNA and putative amino acid sequence are set forth by Li *et al.* The p107 cDNA and putative amino acid sequence are set forth by Ewen *et al.* The entire disclosures of Li *et al.* and Ewen *et al.* are incorporated herein by reference.

It has been found that pRb2/p130, as well as p107 and pRb, act as negative regulators of cell cycle progression, blocking the cells in the G1 phase (Goodrich et al., Cell 67: 293-302 (1991); Zhu et al., Genes Dev. 7:1111-1125 (1993); Claudio et al., Cancer Res. 54:5556-5560 (1994); and Zhu et al., EMBO J. 14:1904-1913 (1995)). However, the three proteins exhibit different growth suppressive properties in selected cell lines, suggesting that although the different members of the retinoblastoma protein family may complement each other, they are not fully functionally redundant (Claudio *et al., supra*).

The mechanisms by which these three proteins exert their control on cell cycle progression are not fully understood but likely include complex formation and modulation of the activity of several transcription factors (Sang et al., Mol. Cell. Differ. 3:1-29 (1995)). The most studied of these complexes is the one with the E2F family of transcription factors. E2F's are heterodimeric transcription factors composed of E2F-like and DP-like subunits that regulate the expression of genes required for progression through G₀/G₁ S phase of the cell cycle (Lan Thangue, N.B., Trends Biochem. Sci. 19:108-114 (1994)).

The three proteins bind and modulate the activity of distinct E2F/DP complexes in different phases of the cell cycle (Sang *et al.. supra;* Chellapan et al., Cell 65:1053-1061 (1991); Shirodkar et al., Cell 66:157-166 (1992); Cobrinik et al., Genes Dev. 7:2392-2404 (1993); Hijmans et al., Mol. Cell. Biol. 15:3082-3089 (1995); and Vairo et al., Genes Dev. 9:869-881 (1995)). This suggests distinct roles for these related proteins in the regulation of the cell cycle.

It has been demonstrated that the growth suppressive properties of pRb2/p130 are specific for the G1 phase. D-type cyclins, as well as transcription factor E2F-1 and E1A viral oncoproteins, were able to rescue pRb2/p130-mediated G1-growth arrest in tumor cells. This suggests that, like other Rb family proteins, the phosphorylation of pRb2/p130 is controlled by the cell cycle machinery, and that pRb2/p130 may indeed be another key G1-S phase regulator. Claudio et al., Cancer Res. 56, 2003-2008 (1996).

The association of pRb with transcription factors, such as E2F, has been shown to occur by interactions at a region known as the "pocket region" (Raychaudhuri et al., Genes Develop., 5 1200-1207 (1991)). Recently, p107 has also been shown to exert such a binding profile (Cao et al., Nature, 355 176-179 (1992)). Domains A and B, along with a spacer, are believed to correspond with the "pocket region" in the pRb2/p130 gene described herein. Moreover, mutations have been found in the pocket region for several human cancers where a lack of function for the pRb protein is thought to be involved in the acquisition of the transformed phenotype (Hu et al., EMBO J., 9 1147-1153 (1990); Huang et al., Mol. Cell. Biol., 10: 3761-3769 (1990)).

The Rb, p107, and pRb2/p130 proteins may play a key role in cell cycle regulation in that all three proteins interact with several cyclin/cdk complexes. pRb can be regulated by cyclin/cdk complexes, such as cyclin A/cdk2, cyclin E/cdk2 and cyclin D/cdk4, even if stable interaction between pRb and cyclin A/cdk2 or cyclin A/cdk2 has not been found *in vivo* (MacLachlan et al., Eukaryotic Gene Exp. 5: 127-156 (1995)). On the other hand, both p107 and pRb2/p130 stably interact *in vivo* with cyclin E/cdk2 and cyclin A/cdk2 complexes (Li *et al., supra;* Ewen et al., Science 255:85-87 (1992); and Faha et al., Science 255:87-90 (1992)). These complexes may be responsible for the existence of different phosphorylated forms of pRb, p107 and pRb2/p130 in the various phases of the cell cycle (Chen et al., Cell 58:1193-1198 (1989); De Caprio et al., Proc., Natl. Acad. Sci. USA 89: 1795-1798 (1992); and Beijersbergen et al., Genes Dev. 9:1340-1353 (1993)). In that pRb's functional activities are enhanced by these phosphorylations, it is likely that pRb2/p130 is also affected in the same manner by this post-translational modification. Since pRb2/p130 demonstrates similar, even if not redundant, functional properties to pRb, it is proposed that pRb2/p130 acts, like pRb, as a tumor suppressor gene. It has also been found that pRb2/p130 maps on the long arm of chromosome 16. This finding reinforces the notion of pRb2/p130 as a tumor suppressor gene. Chromosome 16 is a region frequently reported to show loss of heterozygosity (LOH) in several human neoplasias, such as breast, ovarian, hepatocellular and prostatic carcinomas (Yeung et al., Oncogene 8:3465-3468 (1993)). Chromosome 16, and specifically pRb2/p130, has also been implicated in a rare human skin disease known as hereditary cylindromatosis (HR). HR has been reported as mapping to loci on chromosome 16q12-q13. In that the pRb2/p130 gene maps to chromosome 16q12-q13, it has been put forth as a likely candidate for the tumor suppressor gene involved with the onset of this disease. Biggs et al., Nature Genetics 11:441-4.43 (December 1995).

There is a need for improved methods for identification of individuals at risk for cancer, and for the detection and evaluation of cancers.

Because the pRb2/p130 gene is a tumor suppressor gene and because it maps to a chromosomal region known to be associated with various carcinomas, there is a need for a method to screen individuals for mutations in this gene. There is also a need to identify sequence polymorphisms in this gene. It is believed that mutations, both within the exon coding sequences and the exon-intron junctions, can occur that will affect pRb2/p130's function. Direct DNA sequence analysis of individual exons taken from genomic DNA extracted from tumors has been used successfully to identify mutations of the p53 gene in ovarian carcinomas and the Rb gene in retinoblastoma tumors. Milner et al.. Cancer Research 53: 2128-2132 (1993); Yandell et al.. N.E.J. Medicine 321:1689-1695 (1989). However, direct sequencing of exons is an undesirable approach because it is a time intensive process. An understanding of the genomic structure of the pRb2/p130 gene will enable those skilled in the art to screen a patient's DNA for polymorphisms and sequence mutations in the pRb2/p130 gene. Identification of sequence mutations will also enable the diagnosis of carriers of germline mutations of the pRb2/p130 gene and enable prenatal screening in these cases.

### B. Gynecologic Cancers

Gynecologic cancers include cancers of the uterus, ovary, cervix, vagina, vulva, and fallopian tube as well as gestational trophoblastic disease. Cancers of the uterus include endometrial carcinomas and uterine sarcomas.

Endometrial cancer is the most common malignancy of the female genital tract. Although this neoplasm is frequently diagnosed at an early stage (75 percent in stage I), approximately 20 percent of the patients will die of the disease, half of which were diagnosed at stage I (Pettersson, Annual Report On The Results Of Treatment In Gynecological Cancer, Radiumhemmet, Stockholm, vol. 22: 65-82; Braly, Gynecol Oncol 58: 145-7 (1995)). The ability to identify patients with a more aggressive disease is crucial to planning an adequate treatment for each case. With this purpose in mind, several pathologic tumor features have been considered so far, including histologic type, grade of differentiation, depth of myometrial invasion, lymph nodal metastases and extra-uterine spread (MacMahon. Gynecol Oncol 2: 122 (1974); Chambers et al. , Gynecol Oncol 27: 180-8 (1987)). Unfortunately, none of these factors allows a sufficiently accurate stratification of the patients. Such parameters have also questionable reproducibility.

There is great need for a simple laboratory test which is a consistent predictor of clinical outcome in endometrial cancer. What is needed is a prognostic method which can, at an early disease stage, identify the aggressiveness of an individual patient's disease, before initiation of therapy.

Ovarian cancer is the leading cause of gynecologic cancer death in the United States. Most ovarian malignancies are epithelial carcinomas, with a minority of tumors arising from the germ or stromal cells. In ovarian cancers, the degree of cellular differentiation (histologic grade) is an important independent predictor of both response to treatment and overall survival. Ovarian cancers frequently exhibit chromosomal alterations. The pRb2/p130 gene maps to human chromosome 16q12.2, which is one region that is frequently altered in human ovarian cancers. There is a need for improved methods of grading ovarian tumors. The improved methods would be useful in the diagnosis of disease, in selection of treatment, and as prognostic indicators.

### C. Lung Cancer

Lung cancer is the greatest single cause of cancer-related deaths in Western countries. Selecting an appropriate course of therapy for lung cancer requires an accurate determination of the cancer's malignant potential. This determination is typically made by "grading" the tumor. The grading of tumors is typically carried out by examination of the character and appearance of tumor sections by skilled pathologists. A significant problem in the use of histologic criteria when determining the prognosis and types of treatment for lung cancer is the degree of interobserver and intraobserver variability in reading the same specimens. Determinations are necessarily subjective. In addition, there is heterogeneity within the tumor itself in both primary and metastatic sites. It may become necessary to obtain the opinion of several pathologists to reach a consensus on individual tumor grade.

There is a need for a simple laboratory test which is more consistently predicative of the malignant potential of an individual patient's lung tumor than the present subjective pathological analysis of tumor samples.

Detection of latent cancers before the appearance of lung lesions would allow therapeutic intervention at the earliest stages of the disease, thereby maximizing the prospects for a positive therapeutic outcome. It would also be desirable, through a simple genetic test, to identify disease free individuals who are at risk of lung cancer. Such a screening test would be most advantageous for those individuals who, through environmental exposure to carcinogens or through family history of cancer, may be at risk for developing lung cancer.

There is a need for a simple laboratory test which can be used to augment other forms of lung cancer diagnosis and to identify individuals with latent lung cancers. There is also need for a test to screen individuals for a predisposition to lung cancer.

### Summary of the Invention

The present invention relates to the human pRb2/p130 gene and pRb2/p130 protein, and their use as molecular markers in methods for the diagnosis and prognosis of cancer and for prediction of a predisposition to cancer, wherein the cancer is a gynecologic cancer or a non-small cell lung cancer. According to a most preferred embodiment of the invention, the cancer is endometrial carcinoma, ovarian cancer, a squamous cell carcinoma of the lung, or adenocarcinoma of the lung.

It is an object of the invention to provide a method for determining a prognosis in a patient afflicted with cancer comprising determining the expression level of the pRb2/p130 gene in a sample from the patient, wherein the cancer is a gynecologic cancer or a non small cell lung cancer. A decreased level of pRb2/p130 gene expression in the sample is indicative of an unfavorable prognosis.

Another object of the invention is to provide a method for detecting or identifying a cancerous disease state in a tissue comprising determining the expression level of the pRb2/p130 gene in a sample of the tissue, wherein the cancer is a gynecologic cancer or a non small cell lung cancer. Evaluation is advantageously conducted by determining the level of pRb2/p130 expression in the sample, and comparing the expression level in the sampled tissue with the pRb2/p130 expression level in normal, non-cancerous tissue. A decreased pRb2/p130 expression level is indicative of the presence of cancer. This method may be used to detect cancer in an individual not otherwise displaying a visible lesion.

A further object of the invention is to provide a method for identifying disease free individuals at risk for cancer, or individuals at risk for the recurrence of cancer following treatment, comprising determining the level of expression of the pRb2/p130 gene in tissue sampled from an individual and comparing the pRb2/p130 expression level in the sampled tissue with a normal pRb2/p130 expression level, wherein the cancer is a gynecologic cancer or a non small cell lung cancer. A decreased level of pRb2/p130 expression is indicative of the likelihood of disease or disease recurrence. In the case of endometrial cancer, a method is provided for identifying the risk of recurrence following hysterectomy, and for evaluating the need for further treatment such as radiation therapy or chemotherapy.

Another object of the invention is to provide a method for grading a non small cell lung cancer comprising determining the level of expression of the pRb2/p130 gene in a sample of tissue from a patient suffering from cancer. The expression level in the sampled tissue is compared with the expression level in normal tissue. The degree of the decrement in expression level in the cancer sampled tissue as compared to the normal tissue is indicative of the pathological grade of the cancer. A larger decrement indicates a more aggressive disease state.

A DNA segment consisting essentially of an intron of the pRb2/p130 gene, or an at least 15 nucleotide segment thereof is disclosed.

An amplification primer of at least 15 nucleotides consisting essentially of a DNA segment having a nucleotide sequence substantially complementary to a segment of a pRb2/p130 intron exclusive of the splice signal dinucleotides of said intron is provided.

Methods for identifying polymorphisms and mutations in an exon of a human pRb2/p130 gene are provided.

Described herein is a method for amplifying and identifying polymorphisms and mutations in an exon of a human pRb2/p130 gene, which method comprises:
(a) treating, under amplification conditions, a sample of genomic DNA containing the exon with a primer pair comprising a first primer which hybridizes to the promoter region or to an intron upstream of said exon and a second primer which hybridizes to an intron or to the 3'-noncoding region, said treatment producing an amplification product containing said exon;
(b) determining the nucleotide sequence of said amplification product to provide the nucleotide sequence of said exon; and
(c) comparing the sequence of said exon obtained in step b to a sequence for the sequence of a corresponding wild type exon.

Each primer of the PCR primer pair consists of an amplification primer of at least 15 nucleotides consisting essentially of a DNA segment from the promoter region, from a pRb2/p130 intron exclusive of the splice signal dinucleotides, or from the 3'-noncoding region.

The amplification primer described above has a nucleotide sequence substantially complementary to the 3'-noncoding region, the promoter region given as SEQ ID NO:113, or an intron having a nucleotide sequence selected from the group consisting of SEQ ID NO:48, SEQ ID NO:49, SEQ ID NO:50, SEQ ID NO:51, SEQ ID NO:52, SEQ ID NO:53, SEQ ID NO:54, SEQ ID NO:55, SEQ ID NO:56, SEQ ID NO:57, SEQ ID NO:58, SEQ ID NO:59, SEQ ID NO:60, SEQ ID NO:61, SEQ ID NO:62. SEQ ID NO:63, SEQ ID NO:64, SEQ ID NO:65. SEQ ID NO:66 SEQ ID NO:67. and SEQ ID NO:68.

The amplification primer as described above has a nucleotide sequence selected from the group consisting of SEQ ID NO:69, SEQ ID NO:70, SEQ ID NO:71, SEQ ID NO:72. SEQ ID NO:73. SEQ ID NO:74, SEQ ID NO:75, SEQ ID NO:76, SEQ ID NO:77. SEQ ID NO:78, SEQ ID NO:79, SEQ ID NO:80, SEQ ID NO:81, SEQ ID NO:82, SEQ ID NO:83, SEQ ID NO:84, SEQ ID NO:85, SEQ ID NO:86, SEQ ID NO:87, SEQ ID NO:88, SEQ ID NO:89, SEQ ID NO:90, SEQ ID NO::91, SEQ ID NO:92, SEQ ID NO:93, SEQ ID NO:94, SEQ ID NO:95, SEQ ID NO:96, SEQ ID NO:97, SEQ ID NO:98, SEQ ID NO:99, SEQ ID NO: 100, SEQ ID NO:101, SEQ ID NO:102, SEQ ID NO:103, SEQ ID NO:104, SEQ ID NO:105, SEQ ID NO:106, SEQ ID NO:107, SEQ ID NO:108, SEQ ID NO:109, SEQ ID NO:110, SEQ ID NO:111, and SEQ ID NO:112.

Furthermore disclosed is a method for identifying polymorphisms and mutations in an exon of a human pRb2/p130 gene, which method comprises:
(a) forming a polymerase chain reaction admixture by combining in a polymerase chain reaction buffer, a sample of genomic DNA containing said exon, a primer pair comprising a first primer which hybridizes to the promoter region or to an intron upstream of said exon and a second primer which hybridizes to the 3'-noncoding region or to an intron downstream of said exon, a mixture of one or more deoxynucleotide triphosphates, and a compound capable of radioactively labeling said primer pair, and a DNA polymerase;
(b) subjecting said admixture to a plurality of polymerase chain reaction thermocycles to produce a pRb2/p130 amplification product;
(c) denaturing said pRb2/p130 amplification product:
(d) electrophoretically separating said denatured pRb2/p130 amplification product:
(e) exposing the electrophoretically separated product of step d to a film to produce a photographic image; and
(e) comparing the mobility of the bands in said photographic image of said pRb2/p130 amplification product to a electrophoretically separated amplification product for a corresponding wild type exon.

The present disclosure includes a method for identifying mutations in a human chromosomal sample containing an exon of a human pRb2/p130 gene, which method comprises:
(a) forming an admixture by combining in a buffer, a chromosomal sample containing said exon, a primer pair comprising a first primer which hybridizes to the promoter region or to an intron upstream of said exon and a second primer which hybridizes to the 3'-noncoding region or to an intron downstream of said exon, a mixture of one or more deoxynucleotide triphosphates including at least one deoxynucleotide triphosphate that is labeled, and a DNA polymerase;
(b) subjecting said admixture to a temperature and time sufficient to produce a pRb2/p130 amplification product; and
(c) visualizing said pRb2/p130 amplification product with a fluorochrome conjugate specific to said label; and
(d) comparing the visualized pRb2/p 130 amplification product obtained in step a to a visualized amplification product for a corresponding wild type exon.

These and other objects will be apparent to those skilled in the art from the following discussion.

### Description of the Figures

Figure 1 is a plot of the probability of survival of 100 patients with endometrial carcinoma (all stages) who were characterized as having either pRb2/p130-positive or pRb2/p130-negative tumors.
Figure 2 is a plot of the probability of survival in the same 100 patients with endometrial carcinoma, as stratified by stage and pRb2/p130 expression.
Figure 3A is a schematic representation of the human pRb2/p130 gene. Exons are represented by open rectangles, while the introns are represented by hatched vertical bars. Exons 10-13, 14-16, and 17-20 represent domain A, a spacer, and domain B, respectively.
Figure 3B is a schematic representation of the human pRb2/p130 genomic clones derived from the P1 and X phage libraries.

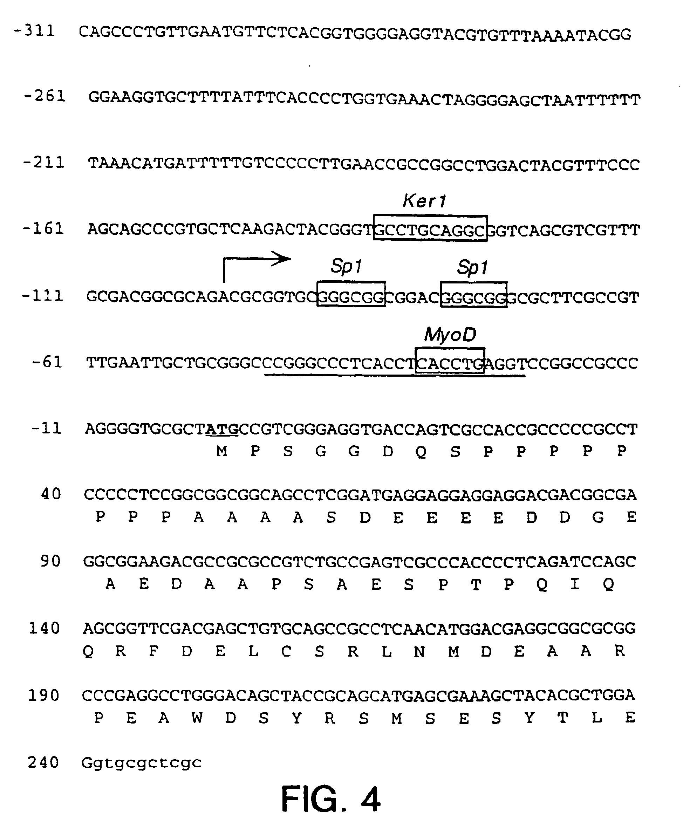

Figure 4 is the nucleotide sequence (SEQ ID NO:4) of the 5' end and 5' upstream region of the human pRb2/p130 gene showing the transcription start site (→) and the sequence complementary to a primer utilized for a primer extension analysis (underlined). Position + 1 is assigned to the A of the ATG translation start codon (bold and underlined). The sequences corresponding to the Sp1 factor recognition motif are boxed. Also boxed are the sequence motifs corresponding to the MyoD and Ker1 transcription factors. The nucleotides beginning at position 1 through position 240 correspond to exon 1 of pRb2/p130. The lowercase letters beginning at position 241 represent the first ten nucleotides of intron 1.
Figure 5 shows the products of a primer extension experiment done to identify the transcription start site for the human pRb2/p130 gene. Cytoplasmatic RNA was hybridized overnight to an oligonucleotide complementary to the twenty four nucleotides beginning at position -22 of Figure 4 (SEQ ID NO:4). Lane M contains molecular-weight markers (φχ174 DNA/Hae III. Promega). Lanes 1 and 2 contain the primer-extended product of pRb2/p130 from HeLa cells and tRNA as template, respectively.
Figure 6 illustrates two alleles containing exon 20 of the pRb2/p130 gene in the nucleus of a peripheral blood lymphocyte visualized through the use of the PRINS technique.

### Detailed Description of the Invention

### A. Abbreviations and Definitions

### 1. Abbreviations

| | |
|---|---|
| bp | base pairs |
| BSA | Bovine Serum Albumin |
| dATP | deoxyadenosine triphosphate |
| dCTP | deoxycytidine triphosphate |
| dGTP | deoxyguanosine triphosphate |
| DIG DNA | Digoxigenin-labeled DNA |
| DIG-dUTP | Digoxigenin-deoxyuridine triphosphate |
| DNA | deoxyribonucleic acid |
| dTTP | deoxythymine triphosphate |
| EDTA | ethylene diaminetetratetic acid |
| FITC | fluorescein isothiocyanate |
| PCR | polymerase chain reaction |
| PHA | phytohemagglutinin |
| PRINS | oligonucleotide-PRimed *IN Situ* synthesis |
| RNA | ribonucleic acid |
| SDS | sodium dodecyl sulfate |
| SSC | standard saline citrate |
| SSCP | single-strand conformation polymorphism |
| TBE | buffer mixture of 0.09 M tris, 0.09 M boric acid, and 2.5 mM EDTA |

### 2. Definitions

**"Allele"** refers to one or more alternative forms of a gene occupying a given locus on a chromosome.
**"Affected tissue"** means tissue which, through visual or other examination, is believed to contain a purported cancerous or precancerous lesion.
**"Amplification product"** refers to a nucleic acid segment produced by amplification procedures such as PCR, SSCP, and PRINS, which product is complementary to the template segment amplified.
**"Downstream"** identifies sequences which are located in the direction of expression, i.e., on the 3'-side of a given site in a nucleic acid.
**"Endometrial cancer"** or **"endometrial carcinoma"** means a polypoid growth arising in the endometrium.
**"Expression",** with respect to the pRb2/p130 gene, means the realization of genetic information encoded in the gene to produce a functional RNA or protein. The term is thus used in its broadest sense, unless indicated to the contrary, to include either transcription or translation.
**"Expression level",** with respect to the pRb2/p130 gene, means not only an absolute expression level, but also a relative expression level as determined by comparison with a standard level of pRb2/p130 expression.
**"Genomic DNA"** refers to all of the DNA sequences composing the genome of a cell or organism. In the invention described herein it includes the exons, introns, and regulatory elements for the pRb2/p130 gene.
**"Grading",** with respect to a tumor sample, means a classification of the perceived degree of malignancy. In grading tumor samples, a pathologist or other observer evaluates the degree of differentiation (e. g. grade 1, well differentiated, grade 2, moderately differentiated, grade 3, poorly differentiated) of the tissue.
**"Gynecologic cancer"** means a tumor arising in the uterus, ovary, cervix, vagina, vulva, or fallopian tube, as well as gestational trophoblastic disease.
**"Hybridization"** means the Watson-Crick base-pairing of essentially complementary nucleotide sequences (polymers of nucleic acids) to form a double-stranded molecule.
**"3'-noncoding region"** means those nucleic acid sequences downstream of the termination codon.
**"Non-small cell lung cancer"** (NSCLC) means all forms of lung cancer except small cell lung cancer (SCLC). In particular, by non-small cell lung cancer is meant the group of lung cancers including squamous cell carcinomas, adenocarcinomas, bronchiolo-alveolar carcinomas and large cell carcinomas.
**"Polymorphic"** refers to the simultaneous occurrence in the population of genomes showing allelic variations. As used herein the term encompasses alleles producing different phenotypes, as well as proteins for which amino acid variants exist in a population, but for which the variants do not destroy the protein's function.
**"Primer"** refers to an oligonucleotide which contains a free 3' hydroxyl group that forms base pairs with a complementary template strand and is capable of acting as the starting point for nucleic acid synthesis by a polymerase. The primer can be single-stranded or double-stranded, however, if in double-stranded form, the primer is first treated in such a way so as to separate it from its complementary strand.
**"pRb2/p130 gene"** means the gene which encodes the pRb2/p130 protein, the cDNA of which is set forth as SEQ ID NO:1, and all allelic variations and mutants thereof.
**"pRb2/p130 intron"** as used herein means a wild type intron segment of the pRb2/p130 gene, as well as any allelic variations thereof.
"pRb2/p130 protein" means the translation product of the pRb2/p130 gene, including all allelic variations and mutants thereof. The pRb2/p130 amino acid sequence is set forth as SEQ ID NO:2.
**"Prognosis"** is used according to its ordinary medical meaning, that is, the prospect of recovery from a disease.
**"Splice junction"** or "exon-intron junction" refers to the nucleotide sequence immediately surrounding an exon-intron boundary of a nuclear gene. As used herein the term includes the sites of breakage and reunion in the mechanism of RNA splicing.
**"Splice signal dinucleotide"** refers to the first two nucleotides (5'-terminal) or the last two nucleotides (3'-terminal) of an intron. In highly conserved genes the 5'-terminal dinucleotide is GT and the 3'-terminal dinucleotide is AG. Alternatively, the 5'-terminal dinucleotide and the 3'-terminal dinucleotide are referred to as the "donor" and "acceptor" sites, respectively.
**"Substantially complementary nucleotide sequence"** means, as between two nucleotide sequences, a relationship such that the sequences demonstrate sufficient Watson-Crick base-pair matching to-enable formation of a hybrid duplex under hybridization conditions. It is not required, however, that the base-pair matchings be exact.
Downstream" identifies sequences which are located in the direction of expression. i.e., on the 3'-side of a given site in a nucleic acid.
**"Upstream"** identifies sequences which are located in the direction opposite from expression, i.e. on the 5'-side of a given site in a nucleic acid.

The present invention provides methods for the identification of individuals at risk for cancer, and for the detection and evaluation of cancers, wherein the cancer is a gynecologic cancer or a non-small cell lung cancer. These methods are of two basic types: methods based on determination of pRb2/p130 expression levels, and methods based on determination of the genomic structure of pRb2/p130.

### B. Methods Based on Determination of pRb2/p130 Expression Levels

The present invention provides improved methods, based on pRb2/p130 expression levels, for the diagnosis and prognosis of gynecologic cancers and non-small cell lung cancers. Among the gynecologic cancers to which these methods may be applied are ovarian cancer and endometrial cancer.

### 1. Gynecologic Cancers

Early ovarian cancer is frequently asymptomatic, or produces only mild symptoms which might be ignored by the patient. The majority of ovarian tumors have spread beyond the ovary, and frequently beyond the pelvis, at the time of diagnosis. Improved methods for the diagnosis and prognosis of ovarian cancer will be useful in treatment selection, and should have a favorable effect on patient outcomes. The present invention rests on the discovery that in ovarian cancer tissue, there is a correlation between the expression of pRb2/p130 and tumor grade.

Endometrial cancer often follows a favorable course, however a considerable proportion of these cases behave poorly and ultimately die of the disease. Currently used surgical-pathologic parameters do not always allow the identification of this subset of patients.

According to the F.I.G.O. criteria for staging in endometrial cancer, surgical procedure should always include peritoneal washing, abdominal hysterectomy, bilateral salpingo-oophorectomy and systematic pelvic and paraaortic lymphadenectomy. Indeed, this operation is often unnecessarily "radical" and potentially dangerous to patients with tumors limited to the uterine corpus. This observation becomes more relevant if it is considered that patients with endometrial cancer very often present also cardiovascular disease, diabetes mellitus, hypertension and severe obesity (Wingo et al., Am J Obstet Gynecol 152:803-8 (1985), which are known risk factors for morbidity from abdominal surgery (DiSaia et al., "Adenocarcinoma Of The Uterus" In: Clinical Gynecologic Oncology, St. Louis: Mosby-Year Book, p. 156-93 (1993). On the other hand, in the obese or patients at high surgical risk total hysterectomy can be easily and safely performed by the vaginal technique (Massi et al., Am J Obstet Gynecol 174:1320-6 (1996); Pitkin, Obster Gynecol 49:567-9 (1977); Peters et al., Am J Obstet Gynecol 146:285-90 (1983)). With this in view, the relative pRb2/p130 expression, assayed according to the present invention may be used in the selection of candidates for a less aggressive surgical treatment, without decreasing their chance of cure, as well as being helpful for the identification of high risk patients, to whom every surgical effort should be attempted and post-surgical treatment given.

Normal cells of the endometrium express a relatively high level of pRb2/p130 protein. The present invention rests on the discovery of a highly statistical inverse correlation between the expression of pRb2/p130 in tissues from endometrial cancer patients and the eventual clinical outcome following treatment. Decreased levels of pRb2/p130 are significantly associated with a poor survival. The study results reported herein indicate that the risk of dying of endometrial carcinoma is increased almost fivefold in patients whose tumors were pRb2/p130 negative, regardless of the tumor stage or grade of differentiation.

Tissue with the greatest malignant potential expresses little or no pRb2/p130. Accordingly, a sample is contacted with an antibody specific for pRb2/p130 protein. In the case of endometrial cancer, the sample may typically comprise endometrial tissue, and may specifically comprise an endometrial tumor. The amount of antibody bound by the sample may be determined relative to the amount of antibody bound by a sample of normal endometrial tissue. The difference in the amount of antibody bound by the normal and test samples is indicative of the patient's prognosis. The endometrial carcinoma study described in Example 1 concurrently tested a known molecular prognostic indicator, *i.e.*, DNA index, various classic clinical-pathologic parameters and pRb2/p130 expression. Decreased levels of pRb2/p130 were significantly associated with a poorer survival. The expression of pRb2/p130 thus represents an independent predictor of clinical outcome in endometrial carcinoma. Well known risk factors, such as F.I.G.O. stage and tumor ploidy were also confirmed as independent prognosticators, although of minor strength. The pRb2/p130 expression was significantly correlated with tumor ploidy and patient age, in that pRb2/p130 negativity was associated with aneuploidy (P=0.001) and with age > 65 years (P =0.008), in accordance with the known negative impact of such features on survival in endometrial cancer (DiSaia et al., Am J Obstet Gynecol 151:1009-15 (1985); Susini et al., Am J Obstet Gynecol 170:527-34 (1994); Massi et al., Am J Obstet Gynecol 174:1320-6 (1996)). However, it is noteworthy that tumor ploidy resisted as an independent prognostic variable by multivariate analysis. Stratification by pRb2/p130 status and ploidy allowed identification of patient subgroups with significant differences in survival (data not shown). A trend toward correlation was also found between pRb2/p130 status and another major prognostic indicator such as grade of differentiation, where pRb2/p130 negativity was more frequent among moderately and poorly differentiated tumors (P=0.06). Furthermore, concerning grade of differentiation, stratification by pRb2/p130 status revealed significant differences in survival within each grade group (data not shown). Expression of pRb2/p130 was not correlated with tumor stage; pRb2/p130 negative tumors were equally distributed among different tumor stages, thus indicating that this feature is typical of certain tumors, from their onset in early stages.

Thus, the pRb2/p130 expression level may serve as a convenient molecular marker to replace or augment conventional prognostic techniques. An important advantage of the use of pRb2/p130 expression over classical surgical pathologic parameters as a prognostic factor is that the former can be determined at the time of the initial diagnosis, before any therapy is initiated. For patients not previously treated by radiotherapy or chemotherapy, low levels of pRb2/p130 can be used to identify tumors with a tendency to behave aggressively.

An early accurate determination of the aggressiveness of disease in an individual patient is a necessary part of designing a course of treatment. In cases where the test method of the invention identifies a poor prognosis, adjuvant therapy, such as radiation therapy or chemotherapy, may be initiated. This more aggressive treatment should increase the patient's chance of survival. The pRb2/p130 expression level, even in early stages of the disease, is reflective of the malignant potential of the patient's carcinoma and the aggressiveness of the ensuing disease course. This form of "molecular based" prognosis can be evaluated more consistently than conventional prognostic factors which are based upon subjective evaluations of histological type, grade of differentiation, depth of myometrial invasion, degree of lymph nodal metastases, extra-uterine spread, and the other factors upon which endometrial carcinoma prognoses are presently based.

### 2. Lung Cancer

In the case of lung cancer, a sample of lung tissue is removed from an individual by conventional biopsy techniques which are well-known to those skilled in the art. The sample is generally collected by needle biopsy. See, for example, Cancer: Principles & Practice of Oncology, V. T. DeVita, Jr. et al., eds. 3rd edition (1989), J. B. Lippincott Co., Philadelphia, PA, p. 616-619, incorporated herein by reference (transcarinal needle biopsy and transthoracic percutaneous fine-needle aspiration biopsy). For identification of lung lesions as comprising NSCLC, the sample is taken from the disease lesion. The disease lesion is first located by x-ray or other conventional lung lesion imaging techniques known to those skilled in the art. For testing for latent NSCLC or NSCLC predisposition, the tissue sample may be taken from any site in the lung. Tissue with the greatest malignant potential expresses little or no pRb2/p130. Normal lung tissue cells express a high level of pRb2/p130 protein. The pRb2/p130 expression level in the cells of the patient lung tumor tissue can be compared with the level in normal lung tissue of the same patient, or with the level in the lung tissue of a normal control group.

Non-small cell lung cancer (NSCLC) includes squamous cell carcinomas, adenocarcinomas, bronchiolo-alveolar carcinomas and large cell carcinomas. A highly significant statistical inverse correlation has been established between the expression of pRb2/p130 in tissues from non-small cell lung cancers and the tissues' pathological grading by skilled pathologists.

Thus, the pRb2/p130 expression level may serve as a convenient molecular marker to replace or augment conventional tumor grading. Accurate tumor grading is a necessary part of designing a course of treatment for the individual patient. Grading is reflective of the malignant potential of the tumor in question and thus the aggressiveness of the ensuing disease course. The generation of vital tumor grade information is made easier, by relying on pRb2/p130 as a molecular surrogate for more subjective observations concerning tumor histology. This form of "molecular-based" grading can be performed more consistently than conventional pathological grading which is based upon subjective evaluations by expert pathologists. pRb2/p130 expression levels may also serve as a convenient molecular marker for the presence of active or latent NSCLC, or predisposition to NSCLC.

Lung lesions may be identified as non-small cell lung carcinomas (NSCLCs) by showing a decrement in the expression of pRb2/p130 in the lesion compared to the level of pRb2/p130 in normal, non-cancerous control lung tissue. Similarly, the level of pRb2/p130 expression in lung tissue of individuals with no apparent lung lesion but other symptoms of lung cancer, or in disease-free individuals, indicates latent NSCLC or risk of NSCLC, respectively. Early diagnosis of NSCLC, even before the appearance of visible lung lesions, will permit earlier initiation of treatment and increased survival.

According to the practice of the invention, an at least about one-third decrement in pRb2/p130 expression level in an affected lung tissue sample, in comparison with normal controls, indicates that the lesion is an NSCLC. Similarly, a pRb2/p130 expression decrement of about one-third or greater in lung tissue of patients who are free of lung lesions but manifest other potential lung cancer symptoms such as sputum cytology irregularities, coughing or bronchitis, is indicative of pre-lesion NSCLC. An about one-third or greater pRb2/p130 expression decrement in lung tissue of otherwise healthy individuals manifesting no symptoms of lung cancer is believed indicative of a risk of future NSCLC. Decrements in pRb2/p130 expression of about one-half or greater are even more indicative of NSCLC disease or NSCLC predisposition.

According to one aspect of the invention, individuals who are disease free are evaluated for risk in contracting NSCLC. The test method may be used to identify individuals at risk of developing NSCLC from among populations exposed to environmental carcinogens, *e.g.* asbestos workers, miners, textile workers, tobacco smokers and the like, and from among families having a history of NSCLC or other forms of cancer.

### 3. Methods for Determining Expression Levels

According to the practice of the present invention, a sample of affected tissue is removed from a cancer patient by conventional biopsy techniques which are well-known to those skilled in the art. The sample is preferably obtained from the patient prior to initiation of radiotherapy or chemotherapy. The sample is then prepared for a determination of pRb2/p130 expression level.

Determining the relative level of expression of the pRb2/p130 gene in the tissue sample comprises determining the relative number of pRb2/p130 RNA transcripts, particularly mRNA transcripts in the sample tissue, or determining the relative level of the corresponding pRb2/p130 protein in the sample tissue. Preferably, the relative level of pRb2/p130 protein in the sample tissue is determined by an immunoassay whereby an antibody which binds pRb2/p130 protein is contacted with the sample tissue. The relative pRb2/p130 expression level in cells of the sampled tumor is conveniently determined with respect to one or more standards. The standards may comprise, for example, a zero expression level on the one hand and the expression level of the gene in normal tissue of the same patient, or the expression level in the tissue of a normal control group on the other hand. The standard may also comprise the pRb2/p130 expression level in a standard cell line. The size of the decrement in pRb2/p130 expression in comparison to normal expression levels is indicative of the future clinical outcome following treatment.

Methods of determining the level of mRNA transcripts of a particular gene in cells of a tissue of interest are well-known to those skilled in the art. According to one such method, total cellular RNA is purified from the effected cells by homogenization in the presence of nucleic acid extraction buffer, followed by centrifugation. Nucleic acids are precipitated, and DNA is removed by treatment with DNase and precipitation. The RNA molecules are then separated by gel electrophoresis on agarose gels according to standard techniques, and transferred to nitrocellulose filters by, *e.g.,* the so-called "Northern" blotting technique. The RNA is immobilized on the filters by heating. Detection and quantification of specific RNA is accomplished using appropriately labelled DNA or RNA probes complementary to the RNA in question. See Molecular Cloning: A Laboratory Manual, J. Sambrook et al. , eds., 2nd edition, Cold Spring Harbor Laboratory Press, 1989, Chapter 7.

In addition to blotting techniques, the mRNA assay test may be carried out according to the technique of *in situ* hybridization. The latter technique requires fewer tumor cells than the Northern blotting technique. Also known as "cytological hybridization", the *in situ* technique involves depositing whole cells onto a microscope cover slip and probing the nucleic acid content of the cell with a solution containing radioactive or otherwise labelled cDNA or cRNA probes. The practice of the *in situ* hybridization technique is described in more detail in U.S. Patent 5,427,916.

The nucleic acid probes for the above RNA hybridization methods can be designed based upon the published pRb2/p130 cDNA sequence of Li et al.. Genes Dev. 7: 2366-2377 (1993). The nucleotide sequence is reproduced herein as SEQ ID NO:1. The translation initiation codon comprises nucleotides 70-72 of SEQ ID NO:1. The translation termination codon comprises nucleotides 3487-3489.

Either method of RNA hybridization, blot hybridization or *in situ* hybridization, can provide a quantitative result for the presence of the target RNA transcript in the RNA donor cells. Methods for preparation of labeled DNA and RNA probes, and the conditions for hybridization thereof to target nucleotide sequences, are described in *Molecular Cloning, supra,* Chapters 10 and 11.

The nucleic acid probe may be labeled with, *e.g.*, a radionuclide such as ³²P, ¹⁴C, or ³⁵S; a heavy metal; or a ligand capable of functioning as a specific binding pair member for a labelled ligand, such as a labelled antibody, a fluorescent molecule, a chemolescent molecule, an enzyme or the like.

Probes may be labelled to high specific activity by either the nick translation method or Rigby et al. , J. Mol. Biol. 113: 237-251 (1977) or by the random priming method, Fienberg et al., Anal. Biochem. 132: 6-13 (1983). The latter is the method of choice for synthesizing ³²P-labelled probes of high specific activity from single-stranded DNA or from RNA templates. Both methods are well-known to those skilled in the art and will not be repeated herein. By replacing preexisting nucleotides with highly radioactive nucleotides, it is possible to prepare ³²P-labelled DNA probes with a specific activity well in excess of 10⁸ cpm/microgram according to the nick translation method. Autoradiographic detection of hybridization may then be performed by exposing filters on photographic film. Densitometric scanning of the filters provides an accurate measurement of mRNA transcripts.

Where radionuclide labelling is not practical, the random-primer method may be used to incorporate the dTTP analogue 5-(N-(N-biotinyl-epsilon-aminocaproyl)-3-aminoallyl)deoxyuridine triphosphate into the probe molecule. The thus biotinylated probe oligonucleotide can be detected by reaction with biotin binding proteins such as avidin, streptavidin, or anti-biotin antibodies coupled with fluorescent dyes or enzymes producing color reactions.

The relative number of pRb2/p130 transcripts may also be determined by reverse transcription of mRNA followed by amplification in a polymerase chain reaction (RT-PCR), and comparison with a standard. The methods for RT-PCR and variations thereon are well known to those of ordinary skill in the art.

The level of pRb2/p130 expression in cells of the patient tissue can be determined by assaying the amount of the corresponding pRb2/p130 protein. A variety of methods for measuring expression of the pRb2/p130 protein exist, including Western blotting and immunohistochemical staining. Western blots are run by spreading a protein sample on a gel, using an SDS gel, blotting the gel with a cellulose nitrate filter, and probing the filters with labeled antibodies. With immunohistochemical staining techniques, a cell sample is prepared, typically by dehydration and fixation, followed by reaction with labeled antibodies specific for the gene product coupled, where the labels are usually visually detectable, such as enzymatic labels, florescent labels, luminescent labels, and the like.

Tissue samples can be obtained from patients and the samples are embedded then cut to e.g. 3-5 µm, fixed, mounted and dried according to conventional tissue mounting techniques. The fixing agent may advantageously comprise formalin. The embedding agent for mounting the specimen may comprise, *e.g.*, paraffin. The samples may be stored in this condition. Following deparaffinization and rehydration, the samples are contacted with an immunoreagent comprising an antibody specific for pRb2/p130. The antibody may comprise a polyclonal or monoclonal antibody. The antibody may comprise an intact antibody, or fragments thereof capable of specifically binding pRb2/p130 protein. Such fragments include, but are not limited to, Fab and F(ab')₂ fragments. As used herein, the term "antibody" includes both polyclonal and monoclonal antibodies. The term "antibody" means not only intact antibody molecules, but also includes fragments thereof which retain antigen binding ability.

Appropriate polyclonal antisera may be prepared by immunizing appropriate host animals with pRb2/p130 protein and collecting and purifying the antisera according to conventional techniques known to those skilled in the art. Monoclonal antibody may be prepared by following the classical technique of Kohler and Milstein, Nature 254:493-497 (1975), as further elaborated in later works such as Monoclonal Antibodies, Hybridomas: A New Dimension in Biological Analysis, R. H. Kennet et al., eds., Plenum Press, New York and London (1980).

Substantially pure pRb2/p130 for use as an immunogen for raising polyclonal or monoclonal antibodies may be conveniently prepared by recombinant DNA methods. According to one such method, pRb2/p130 is prepared in the form of a bacterially expressed glutathione S-transferase (GST) fusion protein. Such fusion proteins may be prepared using commercially available expression systems, following standard expression protocols, e.g., "Expression and Purification of Glutathione-S-Transferase Fusion Proteins", Supplement 10, unit 16.7, in Current Protocols in Molecular Biology (1990). Also see Smith and Johnson, Gene 67: 34-40 (1988); Frangioni and Neel, Anal. Biochem. 210: 179-187 (1993). Briefly, DNA encoding for pRb2/p130 is subcloned into a pGEX2T vector in the correct reading frame and introduced into *E. coli* cells. Transformants are selected on LB/ampicillin plates; the plates are incubated 12 to 15 hours at 37°C. Transformants are grown in isopropyl-β-D-thiogalactoside to induce expression of pRb2/p130-GST fusion protein. The cells are harvested from the liquid cultures by centrifugation. The bacterial pellet is resuspended and the cell pellet sonicated to lyse the cells. The lysate is then contacted with glutathione-agarose beads. The beads are collected by centrifugation and the fusion protein eluted. The GST carrier is then removed by treatment of the fusion protein with thrombin cleavage buffer. The released pRb2/p130 protein is recovered.

As an alternative to immunization with the complete pRb2/p130 molecule, antibody against pRb2/p130 can be raised by immunizing appropriate hosts with immunogenic fragments of the whole protein, particularly peptides corresponding to the carboxy terminus of the molecule.

The antibody either directly or indirectly bears a detectable label. The detectable label may be attached to the primary anti-pRb2/p130 antibody directly. More conveniently, the detectable label is attached to a secondary antibody, *e.g.*, goat anti-rabbit IgG, which binds the primary antibody. The label may advantageously comprise, for example, a radionuclide in the case of a radioimmunoassay; a fluorescent moiety in the case of an immunofluorescent assay; a chemiluminescent moiety in the case of a chemiluminescent assay; or an enzyme which cleaves a chromogenic substrate, in the case of an enzyme-linked immunosorbent assay.

Most preferably, the detectable label comprises an avidin-biotin-peroxidase complex (ABC) which has surplus biotin-binding capacity. The secondary antibody is biotinylated. To locate pRb2/p130 antigen in the tissue section under analysis, the section is treated with primary antiserum against pRb2/p130, washed, and then treated with the secondary antiserum. The subsequent addition of ABC localizes peroxidase at the site of the specific antigen, since the ABC adheres non-specifically to biotin. Peroxidase (and hence antigen) is detected by incubating the section with *e.g.* H₂O; and diaminobenzidine (which results in the antigenic site being stained brown) or H₂O₂ and 4-chloro-1-naphthol (resulting in a blue stain).

The ABC method can be used for paraffin-embedded sections, frozen sections, and smears. Endogenous (tissue or cell) peroxidase may be quenched *e.g.* with H₂O₂ in methanol.

The level of pRb2/p130 expression in tumor samples may be compared on a relative basis to the expression in normal tissue samples by comparing the stain intensities, or comparing the number of stained cells. The lower the stain intensity with respect to the normal controls, or the lower the stained cell count in a tissue section having approximately the same number of cells as the control section, the lower the expression of the pRb2/p130 gene, and hence the higher the expected malignant potential of the sample.

In the examples which follow, a polyclonal antibody raised against pRb2/p130, designated ADL1 was utilized. The specificity of the antibody has been confirmed by Western blot analysis, (Pertile et al., Cell Growth & Diff 6:1659-64 (1995); Claudio et al., Cancer Res 56:2003-8 (1996)), as well as by immunoprecipitation of the antibody with the *in vitro* translated forms of the cDNAs coding for pRb2/p130 and for the other retinoblastoma related proteins, pRb/p105 and p107. The ADL1 antibody was able to immunoprecipitate only the *in vitro* translated form of the pRb2/p130 protein (Baldi et al., Clin Cancer Res 2:1239-45 (1996).

### C. Methods Based on Determination of the Genomic Structure of pRB2/p130

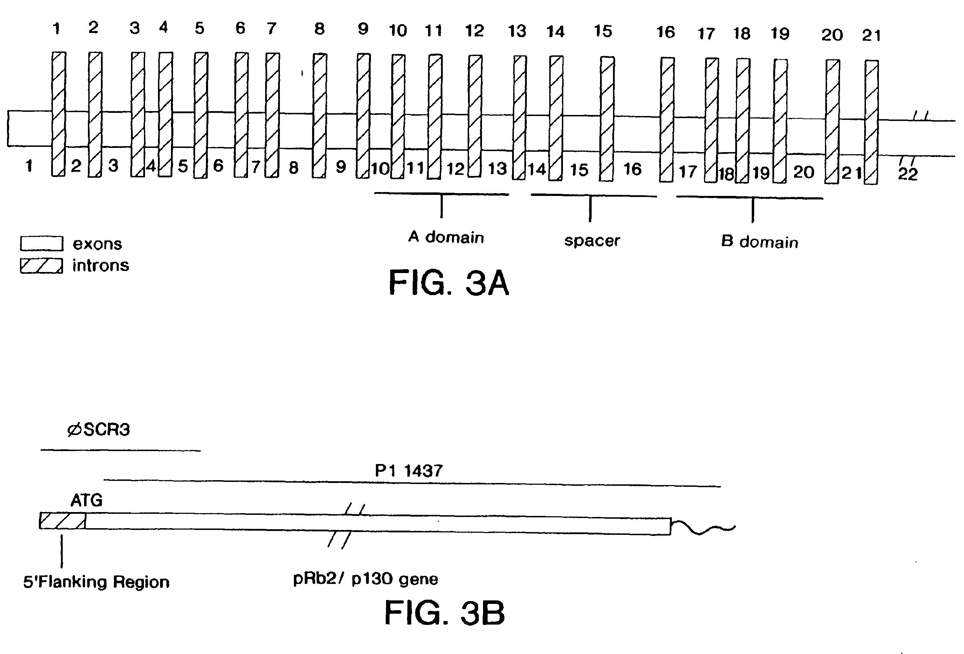

The genomic structure of the human pRb2/p130 gene is described herein. The pRb2/p130 genomic DNA has been cloned and sequenced. The pRb2/p130 gene has been mapped to the long arm of chromosome 16, an area previously reported to show loss of heterozygosity (LOH) for human neoplasias. The putative promoter for pRb2/p130 has been identified, cloned and sequenced. The complete intron-exon organization of the gene has been elucidated. The pRb2/p130 gene contains 22 exons and 21 introns, spanning over 50 kb of genomic DNA. The length of the individual exons ranges from 65 bp to 1517 bp, while the length of individual introns ranges from 82 bp to 9837 bp. The organization of these exons and introns are shown in Figure 3A. The location and size of each exon and intron of pRb2/p130, as well as the nucleotide sequences at the exon-intron junctions are shown below in Table 7. (SEQ ID NOS:6-47). The exon sequences are shown in upper case letters, while the intron sequences are in lower case letters. The superscript numbers correspond to the nucleotide positions of the exon-intron boundaries on SEQ ID NO:1.

All the exons were completely sequenced and no discrepancies were found in comparing the genomic sequence of the exons and the cDNA sequence previously reported. Li, Y. et al., Genes 7:2366-2377 (1993). The exon-intron boundaries were determined by comparing the sequence of the genomic DNA described herein to the published cDNA sequence of Li *et al., supra.* The exon-intron boundaries were identified as the positions where the genomic DNA sequence diverged from that of the cDNA.

With the exception of exon 22, the largest of all the exons (1517 bp in length), the exons found were relatively small, with the shortest, exons 4 and 7, comprising only 65 nucleotides each. Exons 10 through 20 code for the region of the pRb2/p130 protein which form the "pocket region". Exons 10 through 13 and 17 through 20 translate to Domain A and Domain B, respectively. Exons 14, 15, and 16 code for the region of the pRb2/p130 protein, known as the "spacer." The spacer lies between Domains A and B.

The introns have been completely sequenced. The shortest intron, intron 16, lying between exons 16 and 17, is only 82 bp in length, whereas the largest intron, intron 21, spans 9837 bp. Intron 21 is located between exons 21 and 22. The complete sequences for the introns are given as SEQ ID NOS: 48-68. All of the intron sequences of pRb2/p130 conform to the GT-AG rule found to be characteristic of other human genes. Breathnach, R. et al. , Annu. Rev. Biochem. 50: 349-383 (1981). This rule identifies the generic sequence of an intron as GT... ...AG. Introns having this generic form are characterized as conforming to the GT - AG rule. The two dinucleotides, GT and AG, known as the "splice signal dinucleotides," act as signals for splicing out the introns during the processing of the pRb2/p130 mRNA. Point mutations in splice signal dinucleotides have been associated with aberrant splicing in other genes *in vivo* and *in vitro. See generally,* Genes V, B. Lewin. Oxford University Press, pp. 913-916, New York (1994) and Yandell *et al., supra* at p. 1694. Thus, it is important to identify any mutations to the splice signal dinucleotides or other sequences that are excluded from the RNA transcript during splicing.

The pRb2/p130 genomic structure and intron sequences described herein may be used to delineate mutations and rearrangements associated with tumor formation. The genomic structure and intron sequences herein may also be used to screen for naturally occurring polymorphisms at the nucleotide level. Knowledge of a specific single polymorphism can be used to eliminate a mutation in pRb2/p130 as a causative factor in a tumor if the purported mutation displays the same pattern as the polymorphism. Knowledge of polymorphisms in pRb2/p130 can be used to determine the genetic linkage of an identical mutation, and in turn, the tracing of parental origin and family histories without the need for time for time intensive sequencing if mutation is of germline origin. These polymorphisms can then be utilized for the development of diagnostic approaches for human neoplasias. However, it should be noted that not all polymorphisms are of equal utility in these applications. It is preferable to seek out mutations in the exons, as these mutations are most likely to lead to tumor development. Further, because the coding regions of the gene are generally more stable and less likely to mutate over time, it follows that polymorphisms in the exon region are typically less common. The detection of a polymorphism in the exon region of pRb2/p130 would enable screening of both genomic DNA and cDNA.

In the examples that follow, several screening methods are exemplified to identify pRb2/p130 mutations and polymorphisms.

### 1. Transcriptional Control of pRb2/p130

There is evidence that tumor suppressor gene products directly interact with transcription factors, such as MyoD, which regulate not only cell growth, but also cell differentiation. Sang *et al. , supra* at p. 8. Mutations in the sequence region motifs for these transcription factors would be expected to effect the function of the tumor suppressor genes. Accordingly, in addition to identifying the genomic structure of the pRb2/p130 gene, additional experiments were conducted to define the 5'-flanking promoter sequence of pRb2/p130. Part of the putative promotor sequence for pRb2/p130, along with the entire sequence of the first exon and the beginning of the first intron is shown in Figure 4 (SEQ ID NO:4). The full sequence for the putative promoter region is given in SEQ ID NO:113.

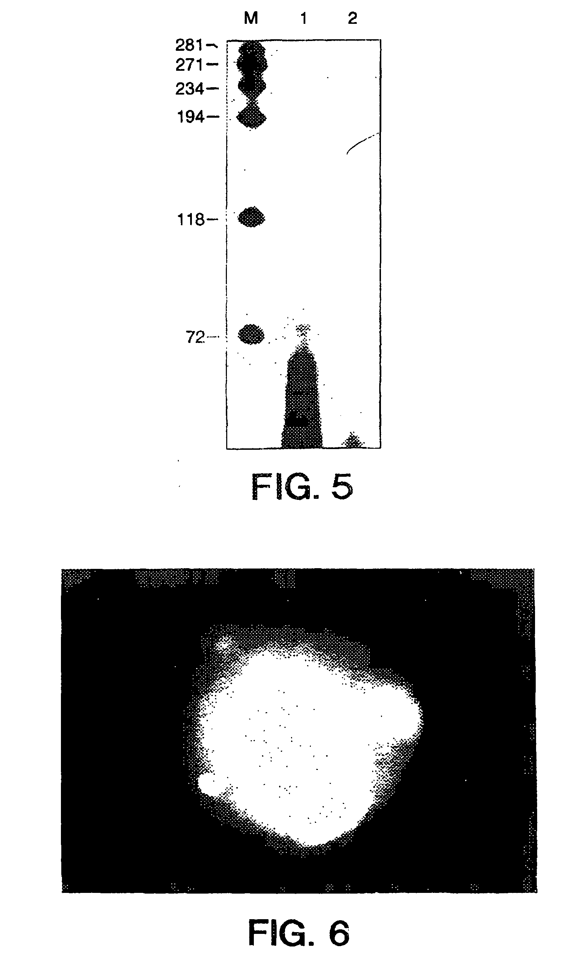

To characterize the pRb2/p130 promoter, a primer extension analysis was performed to locate the transcription initiation site. The protocol for the prime-extension analysis is given in the examples that follow. A twenty four nucleotide segment (SEQ ID NO:114) containing the antisense-strand sequence 26 to 50 nucleotides upstream from the putative ATG codon (*See* Fig. 4) was end-labeled and used as a primer for an extension reaction on cyctoplasmatic RNA from HeLa cells. As shown in Fig. 5, a major extended fragment of 78 bp was detected (lane 1) from the primer extension done with HeLa cells as the template. The additional bands detected by the primer extension analysis could represent additional initiation sites. This finding (lane 1) is consistent with a transcription initiation site 99 nucleotides upstream of the start codon. On the contrary, there was no primer extension product observed when tRNA was used as a template (lane 2). The probable position of the identified transcription initiation site within the promoter sequence is indicated by the arrow in Fig. 4. The primer extension analysis was repeated three times, and similar results were produced in each instance.

The putative transcription factor-binding sites were identified by their similarity to consensus sequences for known transcription factor-binding sites by use of the SIGNAL SCAN program. A description of this program is included in the examples that follow. The most recognizable sequence motifs are for the transcription factors Sp1 (two sites), Ker1 and MyoD. Fig. 4 shows the location of these motifs. Ker1 is involved in keratinocyte-specific transcription, while MyoD is involved in myogenesis. Leask et al., Genes Dev. 4: 1985-1998 (1990); Weintraub, H., Cell 75: 1241-1244 (1993). The presence in the promoter region for pRb2/p130 of these sequence motifs supports a hypothesis of an involvement of this gene in the complex pathways regulating differentiation of specific cell systems.

### 2. Detection of Mutations in pRb2/p130

A method for amplifying the genomic DNA of pRb2/p130 and for screening polymorphisms and mutations are provided therein. The assay methods described herein can be used to diagnose and characterize certain cancers or to identify a heterozygous carrier state. While examples of methods for amplifying and detecting mutations in pRb2/p130 are given, the invention is not limited to the specific methods exemplified. Other means of amplification and identification that rely on the use of the genomic DNA sequence for pRb2/p130 and/or the use of the primers described herein are also contemplated by this invention.

Generally, the methods described herein involve preparing a nucleic acid sample for screening and then assaying the sample for mutations in one or more alleles. The nuclei acid sample is obtained from cells. Cellular sources of genomic DNA include cultured cell lines, or isolated cells or cell types obtained from tissue (or whole organs or entire organisms). Preferably, the cell source is peripheral blood lymphocytes. Methods of DNA extraction from blood and tissue samples are known to those skilled in the art. *See, for example,* Blin et al., Nuc. Acids Res. 3:2303-2308 (1976); and Sambrook et al., Molecular Cloning:A Laboratory Manual, Second Edition, pp. 9.16-9.23, Cold Spring Harbor Laboratory Press, Cold Spring Harbor, NY (1989). If the patient sample to be screened is in the form of double-stranded genomic DNA. it is first denatured using methods known to those skilled in the art. Denaturation can be carried out either by melting or subjecting the strands to agents that destabilize the hydrogen bonds, such as alkaline solutions and concentrated solutions of formamide or urea.

Prior to screening the genomic DNA sample, the pRb2/p130 genomic DNA sample can be amplified by use of the polymerase chain reaction (PCR), using a primer pair, a buffer mixture, and an enzyme capable of promoting chain elongation. Methods of conducting PCR are well known to those skilled in the art. *See,* for example, Beutler *et al.,* U.S. Patent No. 5,234,811, or Templeton, N.S., Diag. Mol. Path. 1(1):58-72 (1992). The amplification product produced from PCR can then be used to screen for mutations using the techniques known as Single Strand Conformational Polymorphism (SSCP) or Primed *In-Situ* DNA synthesis (PRINS). Of course, mutations can also be identified through the more laborious task of sequencing the gene isolates of a patient and comparing the sequence to that for the corresponding wild type pRb2/p130 segment.

PCR is carried out by thermocycling, *i.e*., repeated cycles of heating and cooling the PCR reaction mixture, within a temperature range whose lower end is 37°C to 55°C and upper end is around 90°C to 100°C. The specific temperature range chosen is dependent upon the enzyme chosen and the specificity or stringency required. Lower end temperatures are typically used for annealing in amplifications in which high specificity is not required and conversely, higher end temperatures are used where greater stringency is necessary. An example of the latter is when the goal is to amplify one specific target DNA from genomic DNA. A higher annealing temperature will produce fewer DNA segments that are not of the desired sequence. Preferably, for the invention described herein, the annealing temperature is between 50°C and 65°C. Most preferably, the annealing temperature is 55°C.

The PCR is generally performed in a buffered aqueous solution, *i.e*. , a PCR buffer, preferably at a pH of 7-9, most preferably about 8. Typically, a molar excess of the primar is mixed with the buffer containing the template strand. For genomic DNA, this ratio is typically 10⁶:1 (primer: template). The PCR buffer also contains the deoxynucleotide triphosphates (dATP, dCTP, dGTP, and dTTP) and a polymerase. Polymerases suitable for use in PCR include, but are not limited to, *E*. *coli* DNA polymerase I, the Klenow fragment of *E. coli* DNA polymerase I, T4 DNA polymerase, T7 DNA polymerase, *Taq* DNA polymerase (*Thermus aquaticus* DNA polymerase I), and other heat-stable enzymes which will facilitate the formation of amplification products.

The primers used herein can be naturally occurring oligonucleotides purified from a nucleic acid restriction digest or produced synthetically using any suitable method, which methods are known to those skilled in the art. The primers used herein can be synthesized using automated methods.

Because a mutation can occur in both the exon itself and the splice junction, it is necessary to design primers that will ensure that the entire exon region to be analyzed is amplified. To amplify the entire exon, the oligonucleotide primer for any given exon must be designed such that it includes a portion of the complementary sequence for the promoter region, for the 3'-noncoding region, or for the introns flanking the exon to be amplified, provided however that the primer sequence should not include the sequence for the splice signal dinucleotides. It is important to exclude the complementary sequence for the splice signal dinucleotides from the primer in order to ensure that the entire region, including the splice signal dinucleotide, is amplified. Including the complementary sequences to the splice signal dinucleotides could result in an amplification product that "plasters over" the splice junction and masks any potential mutation that could occur therein. It should be noted, however, that the introns flanking the exon are not limited to the introns immediately adjacent to the exon to be amplified. The oligonucleotide primer can be designed such that it includes a portion of the complementary sequence for the introns upstream or downstream from the exon to exon to be amplified. In the latter instance, the amplification product produced would include more than one exon. Preferably at least 20 to 25 nucleotides of the sequence for each flanking intron are included in the primer sequence.

The primers used herein are selected to be substantially complementary to each strand of the pRb2/p130 segment to be amplified. There must be sufficient base-pair matching to enable formation of a hybrid duplex under hybridization conditions. It is not required, however, that the base-pair matchings be exact. Therefore, the primer sequence may or may not reflect the exact sequence of the pRb2/p130 segment to be amplified. Non-complementary bases or longer sequences can be interspersed into the primer, provided the primer sequence retains sufficient complementarity with the segment to be amplified and thereby form an amplification product.

The primers must be sufficiently long to prime the synthesis of amplification products in the presence of a polymerizing agent. The exact length of the primer to be used is dependent on many factors including, but not limited to, temperature and the source of the primer. Preferably the primer is comprised of 15 to 30 nucleotides, more preferably 18 to 27 nucleotides, and most preferably 24 to 25 nucleotides. Shorter primers generally require cooler annealing temperatures with which to form a stable hybrid complex with the template.

Primer pairs are usually the same length, however, the length of some primers was altered to obtain primer pairs with identical annealing temperatures. Primers of less than 15 bp are generally considered to generate non-specific amplification products.

SSCP can be used to analyze polymorphisms and mutations in the exons of pRb2/p130. SSCP has the advantages over direct sequencing in that it is simple, fast, and efficient. The analysis is performed according to the method of Orita et al. , Genomics 5:874-879 (1989), the entire disclosure of which is incorporated herein by reference. The target sequence is amplified and labeled simultaneously by the use of PCR with radioactively labeled primers or deoxynucleotides. Neither *in situ* hybridization nor the use of restriction enzymes is necessary for SSCP.

SSCP detects sequence changes, including single-base substitutions (point mutations), as shifts in the electrophoretic mobility of a molecule within a gel matrix. A single nucleotide difference between two similar sequences is sufficient to alter the folded structure of one relative to the other. This conformational change is detected by the appearance of a band shift in the tumor DNA, when compared with the banding pattern for a corresponding wild type DNA segment. Single base pair mutations can be detected following SSCP analysis of PCR products up to about 400 bp. PCR products larger than this size must first be digested with a restriction enzyme to produce smaller fragments.

Sequence mutations in pRb2/p130 can be detected utilizing the PRINS technique. The PRINS method represents a versatile technique, which combines the accuracy of molecular and cytogenetic techniques, to provide a physical localization of the genes in nuclei and chromosomes. *See* Cinti et al., Nuc. Acids Res. Vol 21, No. 24: 5799-5800 (1993). The PRINS technique is based on the sequence specific annealing of unlabeled oligodeoxynucleotides *in situ.* The oligodeoxynucleotides operate as a primer for *in situ* chain elongation catalyzed by *Taq* I polymerase. Labeled nucleotides, labeled with a substance such as biotin or Digoxigenin, act as substrate for chain elongation. The labeled DNA chain is visualized by exposure to a fluorochrome-conjugated antibody specific for the label substance. Preferably, the label is Digoxigenin and the fluorochrome conjugated antibody is anti-Digoxigenin-FITC. This results in the incorporation of a number of labeled nucleotides far greater than the number of nucleotides in the primer itself. Additionally, the specificity of the hybridization is not vulnerable to the problems that arise when labeled nucleotides are placed in the primer. The bound label will only be found in those places where the primer is annealed and elongated.

Neither the SSCP nor the PRINS technique will characterize the specific nature of the polymorphism or mutation detected. If a band shift is detected through use of SSCP analysis, one must still sequence the sample segment and compare the sequence to that of the corresponding wild type pRb2/p130 segment. Similarly, if the absence of one or both of the alleles for a given exon segment is detected by the PRINS technique, the sequence of the segment must be determined and compared to the nucleotide sequence for the corresponding wild type in order to determine the exact location and nature of the mutation, i. e. , point mutation, deletion or insertion. The PRINS technique is not capable of detecting polymorphisms.

Protocols for the use of the SSCP analysis and the PRINS technique are included in the examples that follow.

The PRINS method of detecting mutations in the pRb2/p130 gene may be practiced in kit form. In such an embodiment, a carrier is compartmentalized to receive one or more containers, such as vials or test tubes, in close confinement. A first container may contain one or more subcontainers, segments or divisions to hold a DNA sample for drying, dehydrating or denaturing. A second container may contain the PRINS reaction mixture, which mixture is comprised of a PCR buffer, a DIG DNA labeling mixture, a polymerase such as Taq I DNA polymerase, and the primers designed in accordance with this invention (see Example 7, Table 8). The DIG DNA labeling mixture is comprised of a mixture of labeled and unlabeled deoxynucleotides. Preferably, the labeled nucleotides are labeled with either biotin or Digoxigenin. More preferably, the label is Digoxigenin. A third container may contain a fluorochrome conjugated antibody specific to the label. The fluorochrome conjugated antibody specific for Digoxigenin is anti-Digoxigenin-FITC. Suitable conjugated fluorochromes for biotin include avidin-FITC or avidin Texas Red. The fourth container may contain a staining compound, preferably Propidium Iodide (PI). The kit may further contain appropriate washing and dilution solutions.

### Examples

The following examples are illustrative only, and do not limit the scope of the invention.

### Example 1

### Expression of pRb2/p130 in Endometrial Carcinoma

### A. Patients and Tumors

Between September 1988 and December 1994, 196 patients with previously untreated endometrial carcinoma were seen at the Department of Obstetrics and Gynecology, University of Florence, Italy. To avoid concern for the possibility radiation affecting molecular analyses, the patients who received preoperative irradiation were excluded. In 175 cases surgery was the first treatment. Paraffin-embedded tissue blocks containing the most representative portion of the tumor were available in 104 of these cases; four patients were lost to follow up, leaving a total of 100 patients. Patients' ages ranged from 46 to 84 years with a median age of 64 years. Histologic slides were reviewed to assess histologic type, grade of differentiation and depth of myometrial invasion. The stage was evaluated by microscopic analysis of the surgical specimen according to the 1988 International Federation of Gynecology and Obstetrics (FIGO) classification (Gynecol Oncol 35: 125 (1988). Table 1 summarizes the clinical and pathological characteristics of the study group.

### B. Surgical Treatment

Surgical treatment included total hysterectomy in 95 cases and extended hysterectomy in five cases. Bilateral salpingo-oophorectomy was always associated. Pelvic and paraaortic lymphadenectomy were performed at the surgeon's discretion, but not systematically. Overall. 43 patients underwent lymphadenectomy. The omentum was removed when appropriate (four cases).

**Table 1. Clinical And Pathological Features Of 100 Patients In Which pRb2/p130 Expression Was Tested.**

| **Feature** | **Number of Patients** |
|---|---|
| Age | |
| < 65 yr | 52 |
| ≥ 65 yr | 48 |

| FIGO stage | |
|---|---|
| I | 68 |
| II | 15 |
| III | 14 |
| IV | 3 |

| Histologic type | |
|---|---|
| Adenocarcinoma | 74 |
| Adenosquamous | 17 |
| Adenoacanthoma | 4 |
| Papillary serous | 4 |
| Clear cell | 1 |

| Grade of differentiation | |
|---|---|
| Well differentiated | 44 |
| Moderately differentiated | 26 |
| Poorly differentiated | 25 |
| Not evaluable | 5 |
| Depth of myometrial invasion | |
| ≤ 50% | 41 |
| > 50 % | 59 |
| Adjuvant treatment | |
| None | 57 |
| Radiotherapy | 37 |
| Chemotherapy | 6 |

### C. Tumor Specimen Collection

For all 100 patients, a tumor specimen was taken fresh from a site regarded to be representative of the lesion immediately after hysterectomy. Each tumor sample was later divided into two parts: one for flow cytometry and the other for histological analysis.

### D. Adjuvant Therapy

Forty-three of the 100 patients received adjuvant treatment. Of the 43 patients receiving adjuvant treatment, 37 received radiotherapy and 6 received chemotherapy. Poor grade of differentiation, deep myometrial invasion (> 50 percent) and tumor outside the uterine corpus (stage > I) were the major criteria for receiving adjuvant treatment. The irradiated patients (37 patients) received 56Gy on the whole pelvis. Chemotherapy (six patients) was given, when possible, in cases with more advanced disease (stage III-IV). The chemotherapy regimen included cisplatin (60 mg per square meter of body surface area) in combination with cyclophosphamide (600 mg per square meter of body surface area) and epirubicin (60 mg per square meter of body surface area), every 21 days, for six cycles.

### E. Follow-up And Evaluation Of Results

After completing the treatment, patients were seen every three months for the first two years, every four months during the third and fourth years, and every six months thereafter. Recurrence was considered as any documented relapse of the tumor either in the pelvis or systemic. Disease-free interval was calculated from the date of the operation. Patients with residual disease after surgery or who recurred within three months from the date of the operation were not considered free of disease and therefore excluded from the disease-free analysis, but not from the actuarial survival calculation. Patients with deaths from causes other than endometrial cancer were considered as lost to follow-up and therefore their survival times were censored at the date of death. Follow-up data were available for all 100 patients, with a median of 48 months (range 20 to 86 months). Disease-free interval and actuarial survival were the end-points of the study.

### F. Flow Cytometric Analysis Of DNA Index

For flow cytometry, a suspension of tumor cells was obtained by mincing the sample with a lancet and scissors in phosphate-buffered saline. The cell suspension was filtered by a 50 micrometer mesh of polyacrylamide, fixed in 70 percent ethanol, and stored at -4°C until assayed. Prior to DNA analysis the ethanol was removed by centriguation (1500 revolutions/min for ten minutes); the pellet was then resuspended and washed twice in phosphate-buffered saline. The RNA was removed by digestion with ribonuclease (Serva, 0.1 mg/ml in phosphate-buffered saline) for 30 minutes at 37°C. the nuclei were washed in phosphate-buffered saline, and DNA was stained with 40 mg propidium iodide (Becton Dickinson) and 1 gm sodium citrate per liter in distilled water. Human female lymphocytes were added to the samples before enzymatic treatment and staining, and they were used as the DNA diploid standard. The DNA analyses were performed with an Elite flow cytometer (Coulter Corporation, Hialeah, Fla.) provided with a 15 mW Argon laser, at a wavelength of 488 mm. Data were expressed as DNA histograms. The DNA ploidy was given by the DNA index, defined as a proportion of the modal DNA values of the tumor G₀ and G₁ cells (peak channel) to the DNA content of the diploid standard. The histograms were based on measurement of more than 10,000 cells and resulted, in general, in a good resolution with a coefficient of variation of three to six percent. Calculation of DNA index was done by processing each histogram in the computer-assisted program Multicycle Autofit, version 2.00 (Phoenix Flow Systems, San Diego, CA).

All cases with DNA index value of 1 (±0.04) were classified as diploid and others as aneuploid.

### G. Antibody

Rabbit polyclonal immune serum, designated ADL1, was prepared against pRb2/p130 according to the procedure of Harlow and Lane, *Antibodies: A Laboratory Manual,* Cold Spring Laboratory Press (1988), Chapter 5, the disclosure of which is incorporated herein by reference. Rabbits were immunized with a conjugate comprising the peptide Glu-Asn-His-Ser-Ala-Leu-Leu-Arg-Arg-Leu-Gln-Asp-Val-Ala-Asn-Asp-Arg-Gly-Ser-His-Cys (SEQ ID NO:3) coupled to keyhole limpet hemocyanin (KLH). The peptide corresponds to the carboxy terminus of the pRb2/p130 protein. Briefly, rabbits were immunized with the SEQ ID NO:3-KLH conjugate by subcutaneous injection once every two weeks until a total of three injections were given. The initial injection (primary immunization) comprised 1 mg SEQ ID NO:3-KLH conjugate in 500 µl PBS, plus 500 µl of complete Freund's adjuvant. The second and third injections (boosts) comprised 500 µg of the conjugate in 500 µl PBS, plus 500 µl of complete Freund's adjuvant. The rabbits were bled after the third injection. Subsequent boosts, with the same composition as the second and third injections, were given once a month.

### H. Immunohistochemical Analysis

Sections of each tumor specimen were cut to 5-micrometer, mounted on glass and dried overnight at 37 °C. All sections were then deparaffinized in xylene, rehydrated through graded alcohol series and washed in phosphate-buffered saline. This buffer was used for all subsequent washes and for the dilution of the antibodies. Sections were quenched in 0.5 percent hydrogen peroxide and blocked with diluted ten percent normal goat anti-rabbit serum. Slides were then incubated for one hour at room temperature with the ADL1 immune serum at a dilution of 1:1000, then incubated with diluted goat anti-rabbit biotinylated antibody (Vector, Burlingame, Calif.) for 30 minutes at room temperature. After washing in phosphate-buffered saline, the slides were processed by the ABC method (Vector) for 30 minutes at room temperature. Diaminobenzidine (Sigma, St. Louis) was used as the final chromogen, and hematoxylin as the nuclear counterstain. Negative controls for each tissue section consisted of substitution of the primary antibody with the corresponding pre-immune serum. Moreover, preincubation of the antibody with an excess of the corresponding immunizing antigen, blocked the immunocytochemical reaction, thus confirming the specificity of the ADL1 antibody for pRb2/p130 (data not shown).

All the samples were processed under the same conditions. In each experiment, normal uterine tissue was also included as a control. The results of pRb2/p130 immunostaining were independently interpreted by three observers who had no previous knowledge of the clinical outcome of each patient. The level of concordance, expressed as the percentage of agreement between the observers was 90 percent (90 of 100 specimens). In the remaining specimens the score was obtained from the opinions of the two investigators in agreement. The results were expressed as percentage of positive cells. In each tumor sample, at least 20 high power fields were randomly chosen and 2.000 cells were counted. The pRb2/p130 immunostaining was mostly nuclear, but a few specimens also exhibited cytoplasmatic staining. This pattern of immunoreactivity could be referred to microstructural alterations caused by the fixing and embedding procedures, or might reflect differences in the levels of expression and in the localization of this antigen during the various phases of the cell cycle, as has already been shown at the molecular level (Claudio et al., Cancer Res 56: 2003-8 (1996).

### I. Cellular Reactivity Cutoff Point

To evaluate the prognostic value of pRb2/p130 expression, the patients' disease-free and actuarial survival durations were compared after dividing them into two groups using different cutoff points of percent pRb2/p130 positivity. The P values were significant for poor disease-free and actuarial survival when a cutoff point of 40 percent or fewer reactive cells was used (P = 0.003 and P < 0.001, respectively). The level of significance decreased to P = 0.02 and P = 0.01, respectively, with a cutoff point of 50 percent positivity and became insignificant with a cutoff point of 60 percent or higher positivity. Consequently, subsequent survival analyses were carried out using a 40 percent reactivity cutoff point. A similar approach to identify optimal cutoff points has been used in immunohistochemical studies utilizing p53 expression and *bcl*-2 expression (Shim et al. , J Natl Cancer Inst 88: 519-29 (1996); Silvestrini et al., J Clin Oncol 14: 1604-10 (1996)).

### J. Statistical Analysis

Fisher's exact test was used to evaluate the association between pRb2/p130 expression and the other prognostic variables (Fienberg, The Analysis Of Cross-Classified Categorical Data, MIT Press, Cambridge, Mass.: Zelterman et al., "Contingency Tables In Medical Studies". NEJM Books 293-310 (1992)). Disease-free interval and actuarial survival were calculated according to the Kaplan-Meier method (Kaplan et al., Am Stat Assoc 53: 457-81 (1958)) and evaluated by the log-rank test (Miller, Survival Analysis. pp. 44-102. John Wiley, New York (1981)). Univariate Cox analysis was used to assess the effect of each prognostic variable on disease-free interval and survival. A multivariate analysis (Cox proportional-hazards regression, with forward selection of variables) (Cox, J R Stat Soc 34: 187-220 (1972)) was performed to estimate which of the possible risk factors yielded independent prognostic information. Data analysis was performed with the SPSS statistical package, release 5.0.1 (SPSS Inc., Chicago, IL).

### K. Results

A brown stain indicated the presence of pRb2/p130 in tumor cells. The specimens were characterized as having no detectable staining, staining in only a few positive cells (about ten percent), staining in more than 40 percent of the cells, or intense staining in the majority of cells. Tumors with immunostaining in more than 40 percent of cells were considered to be positive for pRb2/p130.

In normal uterine samples, strong immunoreactivity was detected for pRb2/p130 in all endometrial and endocervical epithelial cells. Of the 100 endometrial adenocarcinomas examined, five showed immunoreactivity for pRb2/p130 in 20 percent or fewer cells, 15 had reactivity in 30 percent of the cells and nine had staining in 40 percent of the cells. These 29 tumors (29 percent) were considered pRb2/p130 negative. The remaining 71 tumors were scored as 50 percent positivity in 11 cases, as 60 percent positivity in 49 cases and with staining in over 70 percent of the cells in four cases. These 71 tumors (71 percent) were considered pRb2/p130 positive.

The DNA index values showed a diploid type in 73 cases and an aneuploid type in 27 cases. The DNA index of the aneuploid tumors was hypodiploid in one case, hypertetraploid in four cases; the remaining 22 cases had a modal DNA content in the diploid to tetraploid range (1 < DNA index < 2).

### L. Association Of pRb2/p130 Expression With Clinical And Pathological Features.

The expression of pRb2/p130 was inversely correlated with patients' age: in patients younger than 65 years pRb2/p130 negative tumors were nine of 52 (17.3 percent) in contrast with 20 of 48 in patients aged 65 years or older (41.6 percent) (P = 0.008). Immunostaining for pRb2/p130 was more frequently negative among patients with aneuploid tumors (13 of 27; 48.1 percent) than among those with a diploid pattern (16 of 73; 21.9 percent) (P = 0.001). Tumors negative for pRb2/p130 were more frequent among patients with poorly or moderately differentiated carcinomas, but this association was not statistically significant (P = 0.06). The level of expression of pRb2/p130 did not differ significantly between patients with tumors limited to the uterine corpus (stage I) and those in whom the tumor had spreads outside the corpus uteri (stage > I), (P = 0.4). No significant difference in the incidence of pRb2/p130 negativity was found among the histologic types, nor among patients with different degrees of myometrial infiltration.

Expression of pRb2/p130, tumor ploidy, FIGO stage and grade of differentiation were significantly correlated with disease-free interval and actuarial survival, by Univariate Cox analysis, as shown in Table 2. Other clinico-pathological features, including age, histologic type and depth of myometrial invasion were not associated with the outcome (data not shown).

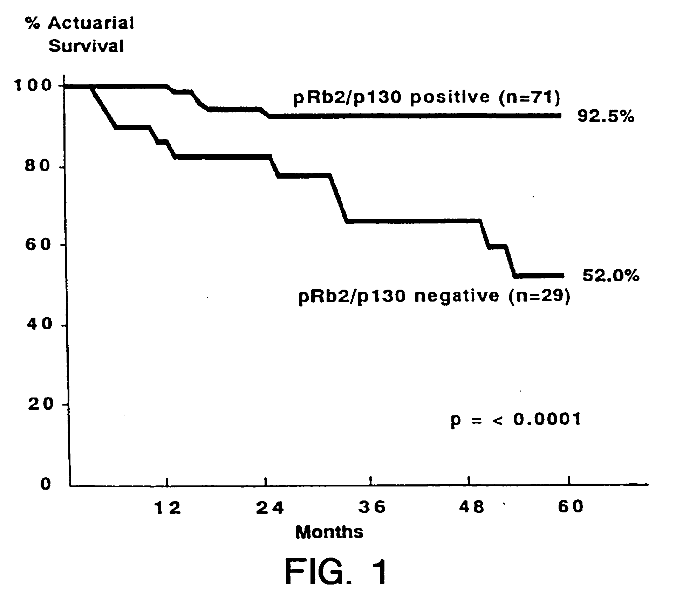

As shown in Figure 1, patients with pRb2/p130 negative tumors had a significantly reduced disease-free interval and survival (P=0.001 and P < 0.0001, respectively); the five-year survival probability was 52.0 percent in patients with such tumors, in contrast with 92.5 percent in patients with pRb2/p130 positive tumors.

**Table 2. Significant Predictors Of Clinical Outcome In 100 Patients With Endometrial Carcinoma, According To Cox Univariate Analysis For Disease-free Interval And Actuarial Survival.**

| **Variable** | **Recurrence Rate Ratio** | **95% Confidence Interval** | **P Value** | **Death Rate Ratio** | **95% CI+** | **P Value** |
|---|---|---|---|---|---|---|
| **pRb2/p130** | | | | | | |
| positive | 1 | | | 1 | | |
| negative | 4.83 | 1.70 - 13.64 | 0.003 | 6.68 | 2.32 - 19.27 | <0.0001 |
| **FIGO stage** | | | | | | |
| 1 | 1 | | | | 1 | |
| > 1 | 5.42 | 1.86 - 15.77 | 0.002 | 5.08 | 1.78 - 14.51 | 0.002 |
| **Ploidy status** | | | | | | |
| diploid | 1 | | | 1 | | |
| aneuploid | 3.43 | 1.24 - 9.51 | 0.01 | 5.94 | 2.14 - 16.42 | <0.001 |
| **Grade of differentiation** (1 = well differentiated, 2= moderately differentiated, 3 = poorly differentiated) | | | | | | |
| 1 | 1 | | | 1 | | |
| 2 | 7.73 | 1.54-38.67 | 0.01 | 13.88 | 1.65 - 116.27 | 0.01 |
| 3 | 7.45 | 1.43 - 38.78 | 0.01 | 18.36 | 2.23 - 151.10 | 0.007 |

Table 3 shows the results of Cox proportional-hazards regression analysis in which the response to pRb2/p130 immunostaining, tumor ploidy, FIGO stage and grade of differentiation were tested simultaneously to estimate the rate ratios for the occurrence of death from disease in patients with endometrial cancer. Negative immunostaining for pRb2/p130 resulted as the strongest independent predictor of poor outcome. Patents with pRb2/p130 negative tumors had a significantly higher rate ratio for dying due to disease (4.91) than patients with pRb2/p130 positive tumors. Multivariate analysis revealed that tumor spread outside the corpus uteri (stage > I) and aneuploidy were also associated with a higher probability of death from disease, whereas grade of differentiation yielded no independent prognostic information. By the combined use of pRb2/p130 expression and FIGO stage, a more accurate definition of risk of death was possible.

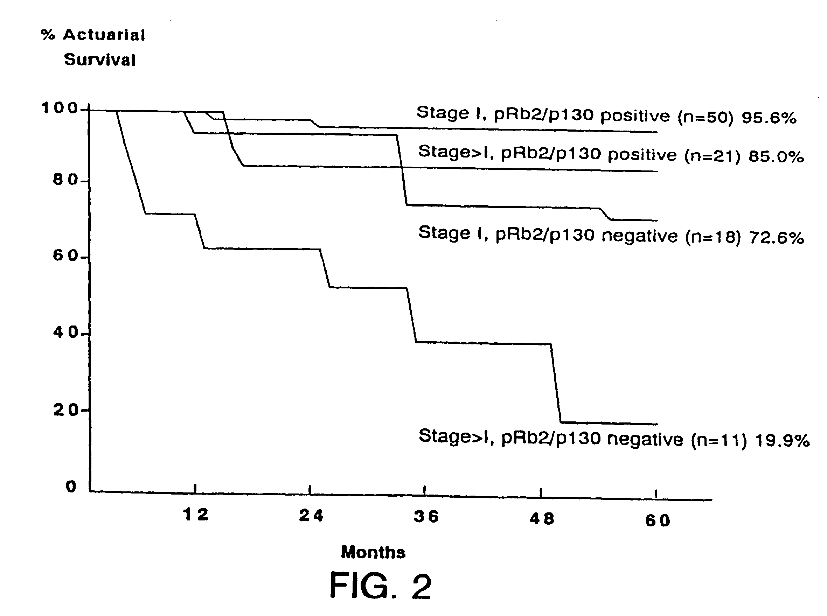

Figure 2 presents Kaplan Meier survival estimates according to these stratified risk groups. The following is the comparison between the groups by the log-rank test:
Stage I, pRb2/p130-Positive versus Stage > I, pRb2/p130-Positive: difference not significant;
Stage I, pRb2/p130-Positive versus Stage I, pRb2/p130-Negative: P = 0.01;
Stage I, pRb2/p130-Negative versus Stage > I, pRb2/p130-Negative: P = 0.005;
Stage > I, pRb2/p130-Positive versus Stage > I, pRb2/p130-Negative: P = 0.003;
Stage > I, pRb2/p130-Positive versus Stage I, pRb2/p130-Negative: difference not significant.

**Table 3. Results Of Cox Proportional-Hazards Regression Analysis For Survival Data.**

| **Variable** | **Rate Ratio** | **95% Confidence Interval** | **P Value*** |
|---|---|---|---|
| pRb2/p 130 | | | |
| positive | 1 | | |
| negative | 4.91 | 1.66 - 14.54 | 0.004 |
| FIGO stage | | | |
| I | 1 | | |
| > I | 4.18 | 1.43- 12.23 | 0.009 |
| Ploidy status | | | |
| Diploid | 1 | | |
| Aneuploid | 3.36 | 1.17 - 9.62 | 0.02 |

| | | | |
|---|---|---|---|
| * Chi-square of the model, P < 0.001 | | | |

### Example 2

### Expression of pRb2/p130 in Ovarian Cancer

### A. Tumors

Sixty archived (formalin fixed and paraffin-embedded) epithelial carcinoma specimens were obtained from the Department of Pathology at Pennsylvania Hospital. The specimens included Grade 1, Grade 2, and Grade 3 tumors.

### B. Immunohistochemistry

Immunohistochemical staining was performed using an automated immunostainer (Ventana ES, Ventana Medical Systems, Tucson. AZ) and a Peroxidase-DAB immunodetection kit (Ventana Medical Systems). Five micron sections were cut from each tumor specimen. The sections were mounted on slides and air-dried. The sections were deparaffinized in xylene and hydrated through a graded alcohol series into water. A polyclonal anti-RB2 primary antibody was applied at a dilution of 1:500 for 30 minutes at 37°C. The slides were then incubated with a biotinylated goat anti-rabbit antibody for 30 minutes. The slides were then incubated with a horseradish peroxidase conjugated-avidin. Hydrogen peroxide was used as the oxidizing substrate, and diaminobenzidine (DAB) was used as the chromagen. The slides were counterstained with hematoxylin, dehydrated, and mounted. The intensity of pRb2/p130 immunostaining was evaluated.

### C. Results

The preliminary results are shown in Table 4. These results suggest that as the grade of tumor increases, less expression of the pRb2/p130 protein is detected. The pRb2/p130 expression level may therefore be useful in grading and as a prognostic indicator in human epithelial ovarian cancer.

**Table 4. Immunohistochemical Detection Of pRb2/p130 In Human Epithelial Ovarian Carcinoma Specimens**

| **Grade of Tumor** | **Intensity of Immunostaining** | | | |
|---|---|---|---|---|
| | Negative | + | + + | + + + |
| Grade 1 | 20 % | 40% | 40 % | 0% |
| Grade 2 | 50 % | 33% | 17 % | 0% |
| Grade 3 | 37 % | 26% | 23 % | 14% |

### Example 3

### Expression of pRb2/p130 in Lung Cancer, Series I

### A. Antibody Against pRb2/p130

The rabbit polyclonal immune serum designated ADL1. as described in Example 1G, was used in these studies.

### B. Antibody Against p107

Rabbit polyclonal immune serum was prepared against p107 (ADL2) by immunizing rabbits with a bacterially expressed GST-p107 fusion protein. Expression of the fusion protein was performed according to the procedure reported by Smith and Johnson, Gene 67:31-40 (1988) and Frangioni and Neel, Anal. Biochem. 210:179-187 (1993). Rabbits were immunized with the fusion protein by subcutaneous injection once every two weeks until a total of three injections were given. The initial injection (primary immunization) comprised 500 µg protein in 500 µl PBS, plus 500 µl of incomplete Freund's adjuvant. The second and third injections (boosts) comprised 100 µg of the protein in 500 µl PBS, plus 500 µl of incomplete Freund's adjuvant. The rabbits were bled after the third injection. Subsequent boosts, with the same composition as the second and third injections, were given once a month.

### C. Antibody Against pRb/p105

An anti-pRb/p105 monoclonal antibody (XZ 77), prepared as described by Hu et al., Mol. Cell. Biol. 11:5792-5799 (1991), was used in these studies.

### D. Tissue Samples

Lung tissue specimens from 51 patients with surgically resected lung cancer were obtained from patients who had not received chemo- or radiotherapy prior to surgical resection. The samples consisted of 39 squamous cell carcinomas and 12 adenocarcinomas. Histological diagnosis and grading were performed by a skilled lung pathologist. Samples were graded on the scale of 1-2-3 with "3" representing the most malignant disease and "1" representing the least malignant disease. Normal lung tissue samples containing the stratified columnar epithelia of trachea, bronchi and adjacent glands were obtained either from biopsy or autopsy performed within 10 hours of the patient's death.

### E. Immunohistochemistry

Sections from each lung tissue specimen were cut at 3-5 µm, mounted on glass and dried overnight at 37°C. All sections were then deparaffinized in xylene, rehydrated through a graded alcohol series and washed in phosphate-buffered saline (PBS). The same buffer was used for all subsequent washes and for dilution of antibodies.

Tissue sections for pRb2/p130 and p 107 detection were sequentially quenched in 0.5 % hydrogen peroxide and blocked with diluted 10 % normal goat anti-rabbit serum (Vector Laboratories). The slides were incubated for 1 hour at room temperature with the rabbit polyclonal immune serum (ADL1) raised against pRb21p130 at a dilution of 1:2000, or the ADL2 antibody against p107 at a dilution of 1:500. The slides were then incubated with diluted goat anti-rabbit biotinylated antibody (Vector Laboratories) for 30 minutes at room temperature.

Sections for pRb/p105 detection were heated twice in a microwave oven for 5 min each at 700 W in citrate buffer (pH6), were quenched sequentially in 0.5 % hydrogen peroxide, and were blocked with diluted 10% normal horse anti-mouse serum (Vector Laboratories, Inc.) The monoclonal mouse anti-human pRb/p105 antibody XZ77 (at a dilution of 1:500) was added and incubated for 120 min. at room temperature. After being washed in PBS. the slides were incubated with diluted horse anti-mouse biotinylated antibody (Vector Laboratories. Inc.) for 30 min. at room temperature.

Slides were processed by the so-called "ABC" method according to the instructions of the biotinylated antibody manufacturer (Vector Laboratories) for 30 minutes at room temperature. Diaminobenzidine was used as the final chromagen, and hematoxylin as a nuclear counterstain. Negative controls for each tissue section consisted of substitution of the primary antibody with pre-immune serum for ADL1 and ADL2, or leaving out the primary antibody for XZ77.

Three pathologists scored the expression of pRb21p130 protein as the percentage of positively stained nuclei on a scale of 0-1-2-3: 0 = undetectable level of expression; 1 = low expression level (1-30% cells stained positive); 2 = medium expression level (30-60% cells stained positive); 3 = high expression level (60-100% cells stained positive). The normal lung tissue samples comprising the stratified epithelia of the trachea, bronchi and adjacent glands were strongly stained, indicating a high expression level.

### F. Results

The results are shown in Table 5.

**TABLE 5**

| **Sample No.** | **Type** | **Grading** | **pRb2/p130 Level** | **p107 Level** | **pRb/p105 Level** |
|---|---|---|---|---|---|
| 1 | squamous | 3 | 0 | 2 | 3 |
| 2 | squamous | 2 | 3 | 1 | 3 |
| 3 | squamous | 1 | 3 | 1 | 3 |
| 4 | squamous | 1 | 3 | 1 | 3 |
| 5 | squamous | 2 | 2 | 1 | 2 |
| 6 | squamous | 2 | 3 | 1 | 2 |
| 7 | squamous | 3 | 1 | 1 | 3 |
| 8 | squamous | 2 | 3 | 1 | 2 |
| 9 | squamous | 2 | 1 | 1 | 2 |
| 10 | squamous | 2 | 3 | 1 | 1 |
| 11 | squamous | 2 | 3 | 1 | 2 |
| 12 | squamous | 1 | 3 | 1 | 3 |
| 13 | squamous | 3 | 1 | 1 | 1 |
| 14 | squamous | 1 | 3 | 1 | 3 |
| 15 | squamous | 3 | 0 | 2 | 3 |
| 16 | squamous | 2 | 2 | 1 | 2 |
| 17 | squamous | 2 | 3 | 1 | 2 |
| 18 | squamous | 2 | 1 | 1 | 2 |
| 19 | squamous | 1 | 3 | 1 | 3 |
| 20 | squamous | 3 | 1 | 1 | 1 |
| 21 | squamous | 2 | 3 | 1 | 2 |
| 22 | squamous | 3 | 2 | 1 | 3 |
| 23 | squamous | 2 | 3 | 1 | 3 |
| 24 | squamous | 2 | 3 | 1 | 1 |
| 25 | squamous | 2 | 3 | 1 | 2 |
| 26 | squamous | 1 | 3 | 1 | 3 |
| 27 | squamous | 3 | 1 | 2 | 3 |
| 28 | squamous | 2 | 3 | 1 | 3 |
| 29 | squamous | 1 | 3 | 1 | 3 |
| 30 | squamous | 1 | 3 | 1 | 3 |
| 31 | squamous | 2 | 2 | 1 | 2 |
| 32 | squamous | 2 | 3 | 1 | 2 |
| 33 | squamous | 3 | 3 | 1 | 3 |
| 34 | squamous | 2 | 3 | 1 | 2 |
| 35 | squamous | 2 | 0 | 1 | 2 |
| 36 | squamous | 2 | 3 | 1 | 1 |
| 37 | squamous | 2 | 3 | 1 | 2 |
| 38 | squamous | 1 | 3 | 1 | 3 |
| 39 | squamous | 3 | 1 | 1 | 0 |
| 40 | adenocarcinoma | 3 | 0 | 2 | 2 |
| 41 | adenocarcinoma | 1 | 2 | 1 | 2 |
| 42 | adenocarcinoma | 2 | 1 | 2 | 1 |
| 43 | adenocarcinoma | 2 | 1 | 1 | 2 |
| 44 | adenocarcinoma | 2 | 0 | 2 | 1 |
| 45 | adenocarcinoma | 2 | 1 | 1 | 2 |
| 46 | adenocarcinoma | 1 | 2 | 1 | 2 |
| 47 | adenocarcinoma | 3 | 0 | 2 | 2 |
| 48 | adenocarcinoma | 1 | 2 | 1 | 2 |
| 49 | adenocarcinoma | 3 | 0 | 2 | 2 |
| 50 | adenocarcinoma | 2 | 1 | 2 | 1 |
| 51 | adenocarcinoma | 2 | 0 | 1 | 2 |

### Statistical Analysis

The data from Table 5 were analyzed using the Jonkheere-Terpstra test and STATXACT statistical software (Cytel Software Corp., Cambridge, MA) determine whether there is a relationship between tissue grade and protein expression level.

A statistically significant inverse relationship was found between the pathological grading and the expression of pRb2/p130 in squamous cell carcinomas (p< .0001) and adenocarcinomas (p<.004).

Although a statistically significant inverse relationship was found between pathological grading and the expression of pRb/p105 in squamous cell carcinomas (p=0.004), no such relationship was found between pRb/p105 expression and grading of adenocarcinomas.

### Example 4

### Expression of pRb2/p130 in Lung Cancer, Series II

### A. Lung Cancer Specimens

One hundred and fifty eight lung cancer specimens were obtained from patients that underwent a surgical resection (lobectomy or pneumonectomy) in the Departments of Thoracic Surgery of the V. Monaldi Hospital and of the II University of Naples (Italy) between January 1995 and April 1996. Specimens were obtained only from patients who had not received chemo- or radiotherapy prior to surgical resection.

The histological diagnoses and classifications of the tumors were based on the WHO criteria, and the postsurgical pathologic TNM stage was determined using the guidelines of the American Joint Committee on Cancer.

The routine histopathological evaluation of the 158 tumor specimens analyzed was performed independently of the pRb2/p130 immunostaining. Thirty two tumors were adenocarcinomas. 118 were squamous carcinomas, 4 were carcinoids and 4 were small cell lung cancers. Eighty seven tumors (55.1%) were classified as stage I, 43 tumors (27.1%) were classified as stage II and 28 tumors (17.7%) were classified as stage IIIa. The adenocarcinomas and squamous carcinomas were classified by grade, as shown in Table 6.

### B. Immunohistochemistry

Sections of each specimen were cut at 3-5 µm, mounted on glass and dried overnight at 37°C. All the sections were then deparaffinized in xylene, rehydrated through a graded alcohol series and washed in PBS. This buffer was used for all subsequent washes and for the dilution of the antibodies. Sections were heated twice in a microwave oven for five minutes each at 700 W in citrate buffer (pH 6), sequentially quenched in 0.5% hydrogen peroxide and blocked with diluted 10% normal goat anti-rabbit serum. Slides were then incubated for one hour at room temperature with rabbit polyclonal immune serum raised against pRb2/p130 at a dilution ranging from 1:500 to 1:1500, then incubated with diluted goat anti-rabbit biotinylated antibody (Vector Laboratories) for 30 minutes at room temperature. After washings in PBS, the slides were processed by the ABC method (Vector Laboratories) for 30 minutes at room temperature. Diaminobenzidine was used as the final chromogen, and hematoxylin as the nuclear counterstain. Negative controls for each tissue section were obtained by substituting the primary antibody with pre-immune serum.

All samples were processed under the same conditions. Three pathologists (A. Baldi, G.G. Giordano and F. Baldi) evaluated the staining pattern of the protein separately and scored it for the percentage of positive nuclei: score 1, less than 10% of positive cells (low to undetectable level of expression); score 2, from 10% to 50% of positive cells (medium level of expression); score 3, more than 50% of positive cells (high level of expression). The level of concordance, expressed as the percentage of agreement between the observers was 90% (142 of 158 specimens). In the remaining specimens the score was obtained from the opinions of the two investigators in agreement. At least 20 high power fields were chosen randomly and 2000 cells were counted. This coded score was preferred to facilitate the statistical analyses.

### C. Statistical Analysis

Statistical analyses, using the chi square test, were performed to evaluate the significance of associations between the different variables of the considered tumors (histological type and grading, evidence of metastasis, pRb2/p130 expression levels). A p value <.05 was considered statistically significant.

### D. Results

pRb20/p130 immunostaining was mostly nuclear, but some specimens clearly exhibited cytoplasmatic staining with a low to absent background.

Immunohistochemical staining patterns of the tumors can be summarized as follows: 50 specimens (31.6%) showed low to undetectable levels of pRb2/p130 (score 1), 73 specimens (46.2%) exhibited medium pRb2/p130 expression levels, while high levels of expression were detected in 35 specimens (22.2%). The small number of small cell lung cancers and carcinoids included in this study did not allow statistical analysis in these histological groups. All the SCLCs specimens exhibited low to undetectable pRb2/p130 expression levels, while a high level of expression of this protein was recognized in all carcinoids.

Statistical analyses revealed that pRb2/p130 expression did not correlate with tumor stage or with TNM status (p = n.s.). However, a negative significant relationship was found between pRb21p130 expression level and the histological grading (p < .0001). The correlation between histological grade and pRb2/p130 expression is shown in Table 6.

**TABLE 6**

| | | | **pRb2/p130 Level** | | |
|---|---|---|---|---|---|
| **Type** | **Grade** | **No.** | **1** | **2** | **3** |
| Squamous | 1 | 13 | 2 | 0 | 11 |
| Squamous | 2 | 42 | 8 | 28 | 6 |
| Squamous | 3 | 63 | 30 | 27 | 6 |
| Adenocarcinoma | 1 | 8 | 0 | 2 | 6 |
| Adenocarcinoma | 2 | 27 | 4 | 16 | 2 |
| Adenocarcinoma | 3 | 2 | 2 | 0 | 0 |

The mean follow-up period was too short to allow a detailed analysis of the disease free and the overall survival time of the patients. However, in looking at the development of metastasis in the patients, we found a significant inverse relationship between metastasis and the expression of pRb2/p130 (p < .0001).

### Example 5

### Isolation and Characterization of Genomic Clones

### A. Isolation of Genomic Clones

To isolate the entire human pRb2/p130 gene, a human P1 genomic library (Genome System Inc., St. Louis. MO) was screened by using two primers made from the published cDNA sequence, Li et al., Genes Dev. 7:2366-2377 (1993). The sequences for the primers used to isolate the genomic clones are GTATACCATTTAGCAGCTGTCCGCC (SEQ ID NO:116) and the complement to the sequence GTGTGCCATTTATGTGATGGCAAAG (SEQ ID NO: 115).

One of the clones identified upon screening the P1 genomic library (clone no. 1437, Fig. 3B) was confirmed by Southern blot hybridization to contain a part of the pRb2/p130 gene. To obtain the additional 5' flanking sequence of the pRb2/p130 gene containing the putative promoter region, a human placenta genomic DNA phage library (EMBL3 SP6/T7) from Clontech, Palo Alto, CA was screened with a cDNA probe according to the method of Sambrook et al., Molecular Cloning:A Laboratory Manual, Second Edition, pp. 12.30-12.38, Cold Spring Harbor Laboratory Press, Cold Spring Harbor, NY (1989). The cDNA probe, labeled with [γ-³²P], corresponded to the first 430 bp after the start codon of the published cDNA sequence, Li et al., *supra.* Of the two positive clones obtained, one, identified as øSCR3 (Fig. 3B), was determined to contain the 5' flanking region of the pRb2/p130 gene.

### B. Identification of Exon/Intron Boundaries

To precisely characterize the position of the exons and the exon/intron boundaries in the genomic DNA, a set of oligonucleotide primers were used to sequence the genomic DNA clones. The primers were synthesized based upon the cDNA nucleotide sequence of pRb2/p130 such that they annealed to the genomic DNA at roughly 150 bp intervals. The exon/intron boundaries were identified from those positions in which the genomic DNA sequence differed from that of the published cDNA sequence.

### C. Sequencing of Clones

Sequencing of the recombinant clones was carried out in part by automated DNA sequencing using the dideoxy terminator reaction chemistry for sequence analysis on the Applied Biosystem Model 373A DNA sequencer and, in part, by using a dsDNA Cycle Sequencing System kit purchased from GIBCO BRL, Gaithersburg, MD, according to the instructions of the manufacturer.

### D. Synthesis of Oligonucleotide Primers

All oligonucleotide primers used herein were synthesized using Applied Biosystems DNA-RNA synthesizer Model 394, using beta-cyanoethyl phosphoramidite chemistry.

### E. Results of the Genomic Clones Characterization

The human pRb2/p130 gene consists of 22 exons and 21 introns and spans more than 50 kb of genomic DNA. The organization of these exons and introns are shown approximately to scale in Figure 3A. The location and size of each exon and intron of pRb2/p130, as well as the nucleotide sequences at the exon-intron boundaries are shown in Table 7 (SEQ ID No:6-47). The exons range in size from 65 to 1517 bp in length. The introns, which range in size from 82-9837 bp in length, have been completely sequenced. The nucleotide sequences are given as SEQ ID NOS:48-68.

### Example 6

### Characterization of Transcriptional Control Elements

### A. Cell Culture and RNA Extraction

The human HeLa (cervix epithelioid carcinoma) cell line was obtained from the American Type Culture Collection and maintained in culture in Dulbecco's modified Eagle medium (DHEM) with 10% fetal calf serum (PCS) at 37°C in a 10% CO₂-containing atmosphere. Cytoplasmatic RNA was extracted utilizing the RNAzol B method (CINNA/BIOTECX, Friendswood, TX).

**TABLE 7**

| Exon-Intron Boundaries of the Human pRb2/p130 Gene | | | |
|---|---|---|---|
| Exon No. (bp) | 5' Donor sequence | 3' Acceptor sequence | Intron No. (bp) |
| 1(240) | ACGCTGGAG³⁰⁹gtgcgctcgc (SEQ ID NO:6) | tcttttacag³¹⁰GGAAATGAT (SEQ ID NO:7) | 1(4220) (SEQ ID NO:66) |
| 2(131) | AGAGCAGAG⁴⁴⁰gtaactatgt (SEQ ID NO:8) | ttaataccag⁴⁴¹CTTAATCGA (SEQ ID NO:9) | 2(3507) (SEQ ID NO:67) |
| 3(201) | GAAACAGCG⁶⁴¹gtaggttttc (SEQ ID NO: 10) | tcccccaaag⁶⁴²GCGACAGCC (SEQ ID NO: 11) | 3(3865) (SEQ ID NO:48) |
| 4(65) | ATGCAAAAG⁷⁰⁶gtaagaaaat (SEQ ID NO: 12) | aatcctgcag⁷⁰⁷GTAATTTCC (SEQ ID NO: 13) | 4(4576) (SEQ ID NO:49) |
| 5(129) | ATTTTAAAG⁸³⁵gtaggtttgt (SEQ ID NO: 14) | acaccatag⁸³⁶GCTTATCTG (SEQ ID NO: 15) | 5(1618) (SEQ ID NO:50) |
| 6(161) | GAAAAAAAG⁹⁹⁶gtttgtaagt (SEQ ID NO:16) | ttcatcatag⁹⁹⁷CTCCTTAAG (SEQ ID NO:17) | 6(92) (SEQ ID NO:51) |
| 7(65) | AGAGAGTTT¹⁰⁶¹gtgagtactt (SEQ ID NO: 18) | ttcctatag¹⁰⁶²TAAAGCCAT (SEQ ID NO: 19) | 7(889) (SEQ ID NO:52) |
| 8(187) | TTTGACAAG¹²⁴⁸gtgagtttag (SEQ ID NO:20) | ttttctttag¹²⁴⁹TCCAAAGCA (SEQ ID NO:21) | 8(4586) (SEQ ID NO:53) |
| 9(167) | GATTCTCAG¹⁴¹⁵gttagtttga (SEQ ID NO:22) | ccttttttag¹⁴¹⁶GACATGTTC (SEQ ID NO:23) | 9(2127) (SEQ ID NO:54) |
| 10(90) | GTGCTAAAG¹⁵²⁵gtaattgtgc (SEQ ID NO:24) | atttctacag¹⁵²⁶AAATTGCCA (SEQ ID NO:25) | 10(716) (SEQ ID NO:55) |
| 11 (104) | GATTTATCT¹⁶²⁹gtgagtaaaa (SEQ ID NO:26) | attttatag¹⁶³⁰GGTATTCTG (SEQ ID NO:27) | 11 (837) (SEQ ID NO:56) |
| 12(138) | TTTTATAAG¹⁷⁶⁷gtatttccca (SEQ ID NO:28) | tttatttcag¹⁷⁶⁸GTGATAGAA (SEQ ID NO:29) | 12(1081) (SEQ ID NO:57) |
| 13(165) | TGTGAAGAG¹⁹³²gtgaaaatca (SEQ ID NO:30) | tcttcatag¹⁹³³GTCATGCCA (SEQ ID NO:31) | 13(1455) (SEQ ID NO:58) |
| 14(112) | TTGGAAGGA²⁰¹⁴gtaagtttaa (SEQ ID NO:32) | ttgacccctag²⁰⁴⁵GCATAACAT (SEQ ID NO:33) | 14(2741) (SEQ ID NO:59) |
| 15(270) | CTGTGCAAG²³¹⁴gtaaggaagg (SEQ ID NO:34) | ctgtcactag²³¹⁵GTATTGCCA (SEQ ID NO:35) | 15(197) (SEQ ID NO:60) |
| 16(281) | TTTAGAAAG²⁵⁹⁵gtaatttttc (SEQ ID NO:36) | tatctcctag²⁵⁹⁶GTATACCAT (SEQ ID NO:37) | 16(82) (SEQ ID NO:61) |
| 17(177) | ATGGCAAAG²⁷⁷²gtgagtacca (SEQ ID NO:38) | gtttgccag²⁷⁷³GTCACAAAA (SEQ ID NO:39) | 17(1079) (SEQ ID NO:62) |
| 18(72) | CGGAGCCAG²⁸⁴⁴gtaactacat (SEQ ID NO:40) | ttctctaaag²⁸⁴⁵GTGTATAGA (SEQ ID NO:41) | 18(659) (SEQ ID NO:63) |
| 19(107) | AAGATAGAA²⁹⁵⁰gtgggatctt (SEQ ID NO:42) | ctggctgcag²⁹⁵¹CCAGTAGAG (SEQ ID NO:43) | 19(572) (SEQ ID NO:64) |
| 20(202) | CAGGCAAAT³¹⁵³gtaagtatga (SEQ ID NO:44) | tttttaaacag³¹⁵⁴ATGGGATGC (SEQ ID NO:45) | 20(901) (SEQ ID NO:65) |
| 21(165) | CCTTCAAAG³³¹⁸gtgagcctaa (SEQ ID NO:46) | cccaccatag³³¹⁹AGACTGAGA (SEQ ID NO:47) | 21(9837) (SEQ ID NO:68) |
| 22(1517) | to the polyadenylation signal | | |

### B. Primer Extension Analysis

To characterize the pRb2/p130 promoter, a primer extension analysis was performed to locate the transcription initiation site. The primer for this analysis was an oligonucleotide, 5'ACCTCAGGTGAGGTGAGGGCCCGG 3' (SEQ ID NO:114), complementary to the pRb2/p130 genomic DNA sequence starting at position -22 (*See* Fig. 4, SEQ ID NO:4). The primer was end labeled with [γ³²P]ATP and hybridized overnight with 20 µg of HeLa cytoplasmatic RNA at 42°C. The primer-annealed RNA was converted into cDNA by avian myeloblastosis virus reverse transcriptase in the presence of 2 mM deoxynucleotides at 42°C for 45 minutes. The cDNA product was then analyzed on 7% sequencing gel containing 8 M urea. The position of the transcription start site was mapped from the length of the resulting extension product.

### C. SIGNAL SCAN Program

Several of the transcription factor-binding motifs were identified through the use of SIGNAL SCAN VERSION 4.0. SIGNAL SCAN is a computer program that was developed by Advanced Biosciences Computing Center at the University of Minnesota, St. Paul, MN. This program aids molecular biologists in finding potential transcription factor binding sites and other elements in a DNA sequence. A complete description of the program can be found in Prestridge, D.S., CABIOS 7: 203-206 (1991), the entire disclosure of which is incorporated herein as if set forth at length.

SIGNAL SCAN finds sequence homologies between published signal sequences and an unknown sequence. A signal, as defined herein, is any short DNA sequence that may have known significance. Most of the known signals represent transcriptional elements. The program does not interpret the significance of the identified homologies; interpretation of the significance of sequences identified is left up to the user. The significance of the signal elements varies with the signal length, with matches to short segments having a higher probability of random occurrence.

### D. Results of the Primer Extension Analysis And SIGNAL SCAN

Figure 5 shows the results of the primer extension analysis done to locate the transcription initiation site for pRb2/p130. A major extended fragment of 78 bp was detected (lane 1) from the primer extension done with HeLa Cells as the template. The probable position of the identified transcription start site is indicated by the arrow in Fig. 4. Putative transcription factor-binding sites were identified by their similarity to consensus sequences for known transcription factor-binding sites. The sequence motifs corresponding to Sp1, Ker1, and MyoD are also indicated in Fig. 4.

### Example 7

### Detection of Heterozygous Mutations By PCR

### A. Preparation of Genomic DNA

The genomic DNA used herein was obtained from human peripheral blood lymphocytes. The samples were prepared by the methods of Sambrook et al., Molecular Cloning:A Laboratory Manual, Second Edition, pp. 9.16-9.23, Cold Spring Harbor Laboratory Press, Cold Spring Harbor, NY (1989).

### B. Synthesis Of PCR Primers

The PCR primers used herein were synthesized as described in Example 5D. The specific primer sequences used and their annealing temperatures are given in Table 8. as SEQ ID NOS:69 to 112.

**Table 8**

| Exon Amplified | Sequence Of Primer (5'-3') | Annealing Temperature (°C) | Size Of PCR Product (bp) |
|---|---|---|---|
| Exon 1 | TTCGCCGTTTGAATTGCTGC (SEQ ID NO:93) | 55 | 359 |
| Exon 1 (rev) | ACCGGTTCACACCAACTAGG (SEQ ID NO:94) | | |
| Exon 2 | GAGATAGGGTCATCATTGAAAC (SEQ ID NO:95) | 55 | 206 |
| Exon 2(rev) | CATTAGCCATACTCTACTTGT (SEQ ID NO:96) | | |
| Exon 3 | GCTAATTTAACTCTGTAACTGC (SEQ ID NO:97) | 55 | 327 |
| Exon 3(rev) | CACTGCAGCACAGACTAATGTGT (SEQ ID NO:98) | | |
| Exon 4 | TCTCTCCCTTTAACTGTGGGTTT (SEQ ID NO:99) | 55 | 245 |
| Exon 4(rev) | GGAGTTGACGAGATTAATACCTG (SEQ ID NO: 100) | | |
| Exon 5 | CTCTGTAACTGCTTATAATCCTG (SEQ ID NO:69) | 55 | 235 |
| Exon 5(rev) | CTAGGAAACCTGTACAACTCC (SEQ ID NO:70) | | |
| Exon 6 | GGCTTATTGTGTGCTGATATC (SEQ ID NO:71) | 55 | 289 |
| Exon 6(rev) | AGAGATCCTTAAGTCGTCATG (SEQ ID NO:72) | | |
| Exon 7 | CATGACGACTTAAGGATCTCTT (SEQ ID NO:101) | 55 | 196 |
| Exon 7(rev) | CTCAGTTTCCAGAGTACAAAC (SEQ ID NO:102) | | |
| Exon 8 | CAGTTTCTGTGAGAGAGTACA (SEQ ID NO:73) | 55 | 283 |
| Exon 8(rev) | GGCTTACCTGCTCCTGTATTT (SEQ ID NO:74) | | |
| Exon 9 | GTGAATTAAAGTCTTTCTGGCC (SEQ ID NO: 103) | 55 | 277 |
| Exon 9(rev) | ATCTTAGAAAGCAGACAGGGC (SEQ ID NO: 104) | | |
| Exon 10 | GAGACATTTTATCCCCTTGTG (SEQ ID NO: 105) | 55 | 289 |
| Exon 10(rev) | TCCATGCCTCCAGTCTAAAGT (SEQ ID NO:106) | | |
| Exon 11 | GAGGAGGAATGGGCCTTTATT (SEQ ID NO:75) | 55 | 244 |
| Exon 11(rev) | AACCCACAGAATAGGGCAGGA (SEQ ID NO:76) | | |
| Exon 12 | CACTTAAGTTGCACTGGGTA (SEQ ID NO: 107) | 55 | 273 |
| Exon 12(rev) | CAACAGGAAGTTGGTCTCATC (SEQ ID NO: 108) | | |
| Exon 13 | TAAAAGGAAGAGCGGCTGTTT (SEQ ID NO: 109) | 55 | 378 |
| Exon 13(rev) | TTAAACCTAACTGCCACCCTC (SEQ ID NO:110) | | |
| Exon 14 | GGATACTGGCATTCTGTGTAAC (SEQ ID NO:77) | 55 | 197 |
| Exon 14(rev) | ATTTCCAGATAGTAAGCCCCA (SEQ ID NO:78) | | |
| Exon 15 | AGCTTGGACGGAAGTCAGATC (SEQ ID NO:79) | 55 | 413 |
| Exon 15(rev) | TCTAGCCAAACCTCGGGTAAC (SEQ ID NO:80) | | |
| Exon 16 | AATTGTAAACCTCTGCCC (SEQ ID NO:81) | 55 | 394 |
| Exon 16(rev) | ATTTCCCAAGCTCATGCT (SEQ ID NO:82) | | |
| Exon 17 | AGCATGAGCTTGGGAAAT (SEQ ID NO:83) | 55 | 277 |
| Exon 17(rev) | TGAAGACCTATCTTTGCC (SEQ ID NO:84) | | |
| Exon 18 | GTTCACAGAGCTCCTCACACT (SEQ ID NO:85) | 55 | 230 |
| Exon 18(rev) | AGGCCACAGAGTCAACTATGG (SEQ ID NO:86) | | |
| Exon 19 | AGGTCCTATCACCAAGGGTGT (SEQ ID NO:87) | 55 | 250 |
| Exon 19(rev) | GCTTAGTTACTTCTTCAAGGC (SEQ ID NO:88) | | |
| Exon 20 | GTAGCTGTTCCCTTTCTCCTA (SEQ ID NO:89) | 55 | 364 |
| Exon 20(rev) | CCTCAACACTCATGAGAGTGA (SEQ ID NO:90) | | |
| Exon 21 | TGGTTTAGCACACCTCTTCAC (SEQ ID NO:91) | 55 | 325 |
| Exon 21(rev) | GCTTAGCACAAACCCTGTTTC (SEQ ID NO:92) | | |
| Exon 22 | CTGAGCTATGTGCATTTGCA (SEQ ID NO:111) | 55 | 232 |
| Exon 22(rev) | AAGGCTGCTGCTAAACAGAT (SEQ ID NO: 112) | | |

### C. PCR Amplification

The sample DNA was amplified in a Perkin-Elmer Cetus thermocycler. The PCR was performed in a 100 µl reaction volume using 2.5 units of recombinant *Taq* DNA-polymerase and 40 ng of genomic DNA. The reaction mixture was prepared according to the recommendations given in the Gene Amp DNA Amplification kit (Perkin-Elmer Cetus). The reaction mixture consisted of 50 mM/l KCl, 10mM/l Tris-HCl (pH 8.3), 1.5 mM MgCl. 200 µM each deoxynucleotide triphosphate and 1 µM of each primer. Thirty five (35) PCR cycles were carried out, with each cycle consisting of an initial denaturation step at 95°C for one minute, one minute at the annealing temperature (55°C), an extension step at 72°C for one minute, and followed by a final incubation period at 72°C for seven minutes. Suitable annealing temperatures are shown in Table 8 for each of the primers designed in accordance with this invention. Minor adjustments in the annealing temperatures may be made to accommodate other primers designed in accordance with this invention.

### D. Amplification Products of PCR

The size of the amplification products produced by PCR are shown in Table 8 above. The lengths of the PCR products ranged from 196 bp to 413 bp.

### E. Sequencing of PCR Products

Sequencing of the amplification products of pRb2/p130 can be conducted according to the method set forth in Example 5C above. Sequencing can also be performed by the chain termination technique described by Sanger et al., Proc. Nat'l. Acad. Sci., U.S.A. 74:5463-5467 (1977) or Sambrook et al., Molecular Cloning: A Laboratory Manual, Second Edition, pp. 13.42-13.77, Cold Spring Harbor Laboratory Press, Cold Spring Harbor, NY (1989) with appropriate primers based on the pRb2/p130 genomic sequence described herein.

### Example 8

### Detecting Mutations By SSCP Analysis

### A. General Methods

The SSCP analysis was performed according to the methods of Orita et al., Genomics 5: 874-879 (1989) and Hogg et al., Oncogene 7: 1445-1451 (1992), each of which is incorporated herein by reference. For the SSCP analysis, amplification of the individual exons was, in some experiments, performed as described in Example 7 with the exception that 1 µCi of (³²P]dCTP (3000 Ci mmol⁻¹) was added to the mixture in order to obtain a labeled product. A 10% aliquot of the PCR-amplified product was diluted with a mixture of 10-20 µl of 0.1 % SDS and 10 mM EDTA. Following a 1:1 dilution with 95% formamide, 2mM EDTA, 0.05% bromophenol blue, and 0.05% xylene cyanol loading solution (United States Biochemicals. OH), the diluted sample was run on a 6% non-denaturing gel. The DNA was electrophoresed in TBE (0.09 M Tris base, 0.09 M boric acid and 2.5 mM EDTA) running buffer at constant wattage at room temperature. The gel was dried on filter paper and exposed to X-ray film for 12 to 72 hours without an intensifying screen.

Polymorphisms and mutations were detected by observing a shift in the electrophoretic mobility pattern of the denatured PCR-amplified product relative to a corresponding wild type sample or normal tissue sample from the same patient. Once a band shift was identified, the segment was sequenced to confirm the exact nature of the polymorphism or mutation.

### B. Detection Of pRb2/p130 Gene Mutations In the CCRF-CEM Cell Line

DNA was extracted from the CCRF-CEM line (human lymphoblastoid cells), and amplified. For the amplification, 50 µl of the PCR reaction mix containing 4 ng of genomic DNA, 0.2 mM of each deoxynucleotide triphosphates, 2 U of Taq polymerase and 0.4 µM of each primer were used. Fifty-Five cycles of denaturation (95°C, 1 minute), annealing (55°C, 1 minute) and extension (72°C, 1 minute) were carried out in a thermal cycler. The SSCP analysis was performed using an MDE mutation detection kit (AT Biochem). The PCR products were heated to 95°C for two minutes and placed directly on ice for several minutes. The samples were run through the MDE gel at 8 Watts constant power for eight hours at room temperature, in 0.6X TBE running buffer. The gel was stained for 15 minutes at room temperature in a 1 µg/ml ethidium bromide solution, made in 0.6X TBE buffer, and placed on a UV-transilluminator to visualize the bands. Exon 20 showed a different migration relative to the control, suggesting the presence of mutations.

The sequences of the PCR products were determined by automated DNA sequencing, using dideoxy-terminator reaction chemistry. Two point mutations were identified: ACC to GCC at position 2950 of SEQ ID NO: 1, resulting in a threonine to alanine substitution; and CCT to CGT at position 3029 of SEQ ID NO:1, resulting in a proline to arginine substitution.

### C. Detection of pRb2/p130 Gene Mutations in Other Cell Lines

Using the SSCP and DNA sequencing methods described above, mutations in the pRb2/p130 gene were identified in the following human tumor cell lines:
Jurkat cell line (human leukemia, T-cell lymphoblast): point mutations in exon 22;
K562 cell line (human chronic myelogenous leukemia, erythroblastoid cells): point mutations in exon 22, deletion in exon 21;
Molt-4 cell line (human T-cell leukemia, peripheral blood lymphoblast): point mutations in exon 21, mutation(s) in exon 22;
Daudi cell line (human thyroid lymphoma, lymphoblast B cell): point mutations and insertion in exon 19, point mutations and insertions in exon 21, mutations(s) in exon 22;
Cem cell line (lymphoblastoid cell line, T-lymphocytes): mutation(s) in exon 20, point mutations and insertions in exon 22;
Saos-2 cell line (human primary osteogenic sarcoma): point mutations and insertions in exon 21, point mutations and insertion in exon 22;
U2-Os cell line (human primary osteogenic sarcoma): point mutations in exons 19 and 21, point mutation and insertion in exon 22:
MG63 cell line (human osteosarcoma): point mutations in exon 19;
Hos cell line (human osteogenic sarcoma. TE85): point mutations in exon 19; insertions in exon 22;
U1752 cell line (human lung tumor): point mutations in exon 19, point mutations and insertion in exon 21, point mutation and insertion in exon 22;
H69 cell line (human lung tumor): point mutations in exon 21, point mutations and insertions in exon 22;
H82 cell line (human lung tumor): point mutations in exon 21; and
Hone cell line (human nasopharyngeal carcinoma): mutations and insertion in exon 21, mutation(s) in exon 22.

### D. Detection of pRb2/p130 Gene Mutations in Primary Tumors

Using the SSCP and DNA sequencing methods described above, mutations in the pRb2/p130 gene were identified in the following primary human tumors:

13 NPC primary tumor (human nasopharyngeal carcinoma): point mutations in exon 21, point mutation and insertions in exon 22; and

5 NPC primary tumor (human nasopharyngeal carcinoma): point mutations and insertion in exon 22.

### Example 9

### Detecting Mutations By The PRINS Technique

The PRINS technique was performed according to the method of Cinti et al., Nuc. Acids Res. Vol. 21, No. 24: 5799-5800 (1993) using human peripheral lymphocytes as the source of genomic DNA. The oligonucleotide primers were designed such that they included portions of the introns flanking exon 20. The sequences of the primers utilized to amplify exon 20 are listed in Table 8 above (SEQ ID NOS:89 and 90).

Human fixed metaphase chromosomes or interphase nuclei from PHA stimulated peripheral blood lymphocytes were spread onto glass slides and allowed to air dry for ten days. The DNA was dehydrated in an ethanol series (70%, 90 % , and 100%) and then denatured by heating to 94 ° C for 5 minutes. Using a reaction mixture containing 200 pmol of each oligonucleotide primer, 5 µl of 10 X PCR Buffer II (AmpliTaq, Perkin-Elmer), 2 µl DIG DNA labeling mixture (1 mM dATP, 1mM dCTP, 1mM dGTP, 0.65 mM dTTP, 0.35 mM DIG-dUTP, Boehringer-Mannheim) and 2 Units of Taq I DNA polymerase (AmpliTaq, Perkin-Elmer), the samples were incubated for 10 minutes at 55°C and for 30 minutes at 72°C. Suitable annealing temperatures for other primers designed in accordance with this invention are shown in Table 8. The samples were then washed two times in 2 X SSC (pH 7.0) and in 4 X SSC (pH 7.0) for 5 minutes at room temperature. The DNA samples were then placed in a solution of 4 X SSC and 0.5 % Bovine Serum Albumin (BSA) (pH 7.0), incubated at room temperature for 45 minutes with anti-Digoxigenin-FITC (Boehringer-Mannheim), and diluted 1:100 in 4 X SSC and 0.5 % BSA (pH 7.0). After washing the samples in 4 X SSC and 0.05% Triton X-100, the samples were counterstained with 1 µg/ml Propidium Iodide (PI).

The slides were examined under a Confocal Laser Scanning Microscope (CLSM Sarastro, Molecular Dynamics). The FITC and PI signals were detected simultaneously, independently elaborated and the final projections were superimposed with a Silicon Graphic Computer Personal IRIS-4D/20 workstation.

Figure 6 shows the results of a PRINS reaction on normal human interphase nuclei. The bright spots correspond to a DNA segment containing exon 20 of pRb21p130. This individual is homozygous for the presence of exon 20 of pRb2/p130. Had there been a mutation in exon 20 of this individual, either one or both of these areas would have been diminished in intensity or not visible in its entirety. To determine the exact nature of this mutation, the patient's pRb2/p130 DNA segment would be sequenced by methods known to those skilled in the art and compared to a wild type sample of pRb2/p130 DNA.

All the references discussed herein are incorporated by reference. Some or all of the reagents, compositions, and supplies needed to carry out the methods, procedures, and techniques disclosed herein may be provided in the form of a kit. Such kits are another embodiment of the present invention.

One skilled in the art will readily appreciate that the present invention is well adapted to carry out the ends and advantages mentioned, as well as those inherent therein. The nucleic acids, compositions, methods, procedures, and techniques described herein are presented as representative of the preferred embodiments, and are intended to be exemplary and not limitations on the scope of the invention.

## Claims

1. A method for determining a prognosis in a patient afflicted with cancer comprising determining the expression level of the pRb2/p130 gene in a sample from a patient, a decreased level of pRb2/p130 expression being indicative of an unfavorable prognosis, wherein the cancer is a gynecologic cancer or a non small cell lung cancer.

2. The method according to claim 1 wherein determining the expression level of the pRb2/p130 gene comprises determining the relative number of RNA transcripts of the gene, relative to a standard number of said transcripts.

3. The method according to claim 1 wherein determining the expression level of the pRb2/p130 gene comprises determining the relative level of the pRb2/p130 protein, relative to a standard level of said protein.

4. The method according to claim 3 wherein the level of the pRb2/p130 protein is determined by contacting the sample with an antibody which binds the pRb2/p130 protein.

5. The method according to claim 1 wherein the sample is obtained from the patient prior to treatment of the patient with radiotherapy or chemotherapy.

6. The method according to claim 1 wherein the gynecologic cancer is endometrial carcinoma or ovarian cancer.

7. The method according to claim 6 wherein in the case of endometrial carcinoma, the sample comprises endometrial tissue.

8. The method according to claim 7 wherein the endometrial tissue comprises a tumor.

9. A method for detection of a cancerous disease state in a tissue of a patient comprising determining the expression level of the pRb2/p130 gene in a sample of the tissue, a decreased level of pRb2/p130 expression, compared with (1) a zero expression level of pRb2/p130 , (2) the expression level of pRb2/p130 gene in normal tissue of the same patient, (3) the expression level in the tissue of a normal control group, or (4) the expression level in a standard cell line, being indicative of the presence of cancer, wherein the cancer is a gynecologic cancer or a non small cell lung cancer.

10. The method according to claim 9 wherein determining the expression level of the pRb2/p130 gene comprises determining the relative number of RNA transcripts of the gene, relative to a standard number of said transcripts.

11. The method according to claim 9 wherein determining the expression level of the pRb2/p130 gene comprises determining the relative level of the pRb2/p130 protein, relative to a standard level of said protein.

12. The method according to claim 11 wherein the level of the pRb2/p130 protein is determined by contacting the sample with an antibody which binds the pRb2/p130 protein.

13. The method according to claim 12 wherein the gynecologic cancer is endometrial carcinoma or ovarian cancer.

14. A method for identifying individuals at risk for cancer, or individuals at risk for the recurrence of cancer after treatment, comprising:
determining the level of expression of pRb2/p130 in tissue sampled from an individual; and
comparing the pRb2/p130 expression level in the sampled tissue with a normal pRb2/p130 expression level,
a decrease in the pRb2/p130 expression level being indicative of the risk for cancer, or indicative for the risk of recurrence of cancer after treatment,
wherein the cancer is a gynecologic cancer or a non small cell lung cancer.

15. The method according to claim 14 wherein determining the expression level of the pRb2/p130 gene comprises determining the relative number of RNA transcripts of the gene, relative to a standard number of said transcripts.

16. The method according to claim 14 wherein determining the expression level of the pRb2/p130 gene comprises determining the relative level of the pRb2/p130 protein, relative to a standard level of said protein.

17. The method according to claim 16 wherein the level of the pRb2/p130 protein is determined by contacting the sample with an antibody which binds the pRb2/p130 protein.

18. The method according to claim 14 wherein the gynecologic cancer is endometrial carcinoma or ovarian cancer.

19. A method for grading a cancer comprising:
determining the level of expression of the pRb2/p130 gene in a sample of tissue from a patient suffering from cancer, the level of expression being indicative of the grade of the cancer, wherein the cancer is a non small cell lung cancer.

20. The method according to claim 19 wherein determining the level of expression of the pRb2/p130 gene comprises determining the relative number of RNA transcripts of the gene in the sampled tissue, relative to a standard number of said transcripts.

21. The method according to claim 19 wherein determining the level of expression of the pRb2/p130 gene comprises determining the relative level of the corresponding pRb2/p130 protein in the sampled tissue, relative to a standard level of said protein.

22. The method according to claim 21 wherein the level of the protein in the sampled tissue is determined by an immunoassay whereby an antibody which binds said pRb2/p130 protein is contacted with said sampled tissue.

23. The method according to claim 19 wherein the non small cell lung cancer is a squamous cell carcinoma or an adenocarcinoma.

## Patentansprüche

1. Verfahren zur Bestimmung der Prognose für einen Patienten, der an Krebs leidet, umfassend eine Bestimmung des Expressionslevels des pRb2/p130-Gens in einer Probe von einem Patienten, wobei ein verringerter Level der pRb2/p130-Expressionindikativ für eine ungünstige Prognose ist, wobei der Krebs ein gynäkologischer Krebs oder ein nicht-kleinzelliger Lungenkrebs ist.

2. Verfahren gemäß Anspruch 1, wobei eine Bestimmung des Expressionslevels des pRb2/p130-Gens eine Bestimmung der relativen Anzahl von RNA-Transkripten des Gens im Vergleich zu einer Standardanzahl der Transkripte umfasst.

3. Verfahren gemäß Anspruch 1, wobei eine Bestimmung des Expressionslevels des pRb2/p130-Gens eine Bestimmung des relativen Levels des pRb2/p130-Proteins im Vergleich zu einem Standardlevel des Proteins umfasst.

4. Verfahren gemäß Anspruch 3, wobei der Level des pRb2/p130-Proteins bestimmt wird, indem die Probe mit einem Antikörper, welcher das pRb2/p130-Protein bindet, in Kontakt gebracht wird.

5. Verfahren gemäß Anspruch 1, wobei die Probe von einem Patienten vor Behandlung des Patienten mit Radiotherapie oder Chemotherapie erhalten wird.

6. Verfahren gemäß Anspruch 1, wobei der gynäkologische Krebs Endometriumkarzinom oder Ovarialkarzinom ist.

7. Verfahren gemäß Anspruch 6, wobei die Probe im Fall von Endometriumkarzinom Endometriumgewebe umfasst.

8. Verfahren gemäß Anspruch 7, wobei das Endometriumgewebe einen Tumor umfasst.

9. Verfahren zur Detektion eines Krebserkrankungszustandes in einem Gewebe eines Patienten, umfassend eine Bestimmung des Expressionslevels des pRb2/p130-Gens in einer Probe des Gewebes, wobei ein verringerter Level der pRb2/p130-Expression im Vergleich zu (1) einem 0-Expressionslevel von pRb2/p130, (2) dem Expressionslevel von pRb2/p130-Gen in normalem Gewebe des selben Patienten, (3) dem Expressionslevel im Gewebe einer normalen Kontrollgruppe oder (4) dem Expressionslevel in einer Standardzelllinie für das Vorliegen von Krebs indikativ ist, wobei der Krebs ein gynäkologischer Krebs oder ein nicht-kleinzelliger Lungenkrebs ist.

10. Verfahren gemäß Anspruch 9, wobei eine Bestimmung des Expressionslevels des pRb2/p130-Gens eine Bestimmung der relativen Anzahl von RNA-Transkripten des Gens im Vergleich zu einer Standardanzahl der Transkripte umfasst.

11. Verfahren gemäß Anspruch 9, wobei eine Bestimmung des Expressionslevels des pRb2/p130-Gens eine Bestimmung des relativen Levels des pRb2/p130-Proteins im Vergleich zu einem Standardlevel des Proteins umfasst.

12. Verfahren gemäß Anspruch 11, wobei der Level des pRb2/p130-Proteins bestimmt wird, indem die Probe mit einem Antikörper, welcher das pRb2/p130-Protein bindet, in Kontakt gebracht wird.

13. Verfahren gemäß Anspruch 12, wobei der gynäkologische Krebs Endometriumkarzinom oder Ovarialkarzinom ist.

14. Verfahren zur Identifizierung von Individuen mit Krebsrisiko oder von Individuen mit dem Risiko für das Wiederauftreten von Krebs nach Behandlung, umfassend:
Bestimmung des Expressionslevels von pRb2/p130 in Gewebe, das als Probe von einem Individuum entnommen wurde, und
Vergleichen des pRb2/p130-Expressionslevels in dem als Probe genommenen Gewebe mit einem normalen pRb2/p130-Expressionslevel,
wobei eine Verringerung im pRb2/p130-Expressionslevels für das Krebsrisiko indikativ ist oder für das Risiko eines Wiederauftretens von Krebs nach Behandlung indikativ ist,
wobei der Krebs ein gynäkologischer Krebs oder ein nicht-kleinzelliger Lungenkrebs ist.

15. Verfahren gemäß Anspruch 14, wobei eine Bestimmung des Expressionslevels des pRb2/p130-Gens eine Bestimmung der relativen Anzahl von RNA-Transkripten des Gens im Vergleich zu einer Standardanzahl der Transkripte umfasst.

16. Verfahren gemäß Anspruch 14, wobei eine Bestimmung des Expressionslevels des pRb2/p130-Gens eine Bestimmung des relativen Levels des pRb2/p130-Proteins im Vergleich zu einem Standardlevel des Proteins umfasst.

17. Verfahren gemäß Anspruch 16, wobei der Level des pRb2/p130-Proteins bestimmt wird, indem die Probe mit einem Antikörper, welcher das pRb2/p130-Protein bindet, in Kontakt gebracht wird.

18. Verfahren gemäß Anspruch 14, wobei der gynäkologische Krebs Endometriumkarzinom oder Ovarialkarzinom ist.

19. Verfahren zur Graduierung eines Krebses, umfassend:
Bestimmung des Expressionslevels des pRb2/p130-Gens in einer Gewebeprobe von einem Patienten, der an Krebs leidet, wobei der Expressionslevel für den Grad des Krebses indikativ ist,
wobei der Krebs ein nicht-kleinzelliger Lungenkrebs ist.

20. Verfahren gemäß Anspruch 19, wobei eine Bestimmung des Expressionslevels des pRb2/p130-Gens eine Bestimmung der relativen Anzahl von RNA-Transkripten des Gens in dem als Probe entnommenen Gewebe im Vergleich zu einer Standardanzahl der Transkripte umfasst.

21. Verfahren gemäß Anspruch 19, wobei eine Bestimmung des Expressionslevels des pRb2/p130-Gens eine Bestimmung des relativen Levels des entsprechenden pRb2/p130-Proteins in dem als Probe entnommenen Gewebe im Vergleich zu einem Standardlevel des Proteins umfasst.

22. Verfahren gemäß Anspruch 21, wobei der Level des Proteins in dem als Probe entnommenen Gewebe durch einen Immunoassay bestimmt wird, wobei ein Antikörper, welcher an das pRb2/p130-Protein bindet, mit dem als Probe entnommenen Gewebe in Kontakt gebracht wird.

23. Verfahren gemäß Anspruch 19, wobei der nichtkleinzellige Lungenkrebs ein Plattenepithelkarzinom oder ein Adenokarzinom ist.

## Revendications

1. Procédé de détermination d'un pronostic sur un patient souffrant d'un cancer, comprenant la détermination du niveau d'expression du gène pRb2/p130 dans un échantillon provenant d'un patient, un niveau diminué d'expression pRb2/p130 étant indicatif d'un pronostic défavorable, dans lequel le cancer est un cancer gynécologique ou un cancer bronchique non à petites cellules.

2. Procédé selon la revendication 1, dans lequel la détermination du niveau d'expression du gène pRb2/p130 comprend la détermination du nombre relatif de transcrits d'ARN du gène, relativement à un nombre standard desdits transcrits.

3. Procédé selon la revendication 1, dans lequel la détermination du niveau d'expression du gène pRb2/p130 comprend la détermination du niveau relatif de la protéine pRb2/p130, relativement à un niveau standard de ladite protéine.

4. Procédé selon la revendication 3, dans lequel le niveau de protéine pRb2/p130 est déterminé en faisant entrer en contact l'échantillon avec un anticorps qui lie la protéine pRb2/p130.

5. Procédé selon la revendication 1, dans lequel l'échantillon est obtenu sur le patient avant le traitement dudit patient par radiothérapie ou chimiothérapie.

6. Procédé selon la revendication 1, dans lequel le cancer gynécologique est un cancer de l'endomètre ou un cancer des ovaires.

7. Procédé selon la revendication 6, dans lequel dans le cas d'un cancer de l'endomètre, l'échantillon comprend du tissu endométrial.

8. Procédé selon la revendication 7, dans lequel le tissu endométrial comprend une tumeur.

9. Procédé de détection d'un état pathologique cancéreux dans le tissu d'un patient comprenant la détermination du niveau d'expression du gène pRb2/p130 dans un échantillon du tissu, un niveau diminué d'expression pRb2/p130, par rapport (1) au niveau d'expression zéro de pRb2/p130, (2) au niveau d'expression du gène pRb2/p130 dans le tissu normal du même patient, (3) au niveau d'expression dans le tissu d'un groupe de contrôle normal, ou (4) au niveau d'expression dans une ligne cellulaire standard, étant indicatif de la présence de cancer, dans lequel le cancer est un cancer gynécologique ou un cancer bronchique non à petites cellules.

10. Procédé selon la revendication 9, dans lequel la détermination du niveau d'expression du gène pRb2/p130 comprend la détermination du nombre relatif de transcrits d'ARN du gène, relativement à un nombre standard desdits transcrits.

11. Procédé selon la revendication 9, dans lequel la détermination du niveau d'expression du gène pRb2/p130 comprend la détermination du niveau relatif de la protéine pRb2/p130, relativement à un niveau standard de ladite protéine.

12. Procédé selon la revendication 11, dans lequel le niveau de protéine pRb2/p130 est déterminé en mettant en contact l'échantillon avec un anticorps qui lie la protéine pRb2/p130.

13. Procédé selon la revendication 12, dans lequel le cancer gynécologique est un cancer de l'endomètre ou un cancer des ovaires.

14. Procédé permettant d'identifier les individus présentant un risque de cancer, ou les individus à risque pour la rechute du cancer après traitement, comprenant :
la détermination du niveau d'expression de pRb2/p130 dans le tissu prélevé sur un individu ; et
la comparaison du niveau d'expression pRb2/p130 dans le tissu prélevé avec un niveau d'expression pRb2/p130 normal ;
une diminution du niveau d'expression pRb2/p130 étant indicative du risque de cancer, ou indicative du risque de rechute du cancer après traitement,
dans lequel le cancer est un cancer gynécologique ou un cancer bronchique non à petites cellules.

15. Procédé selon la revendication 14, dans lequel la détermination du niveau d'expression du gène pRb2/p130 comprend la détermination du nombre relatif de transcrits d'ARN du gène, relativement à un nombre standard desdits transcrits.

16. Procédé selon la revendication 14, dans lequel la détermination du niveau d'expression du gène pRb2/p130 comprend la détermination du niveau relatif de la protéine pRb2/p130, relatif à un niveau standard de ladite protéine.

17. Procédé selon la revendication 16, dans lequel le niveau de la protéine pRb2/p130 est déterminé en mettant en contact l'échantillon avec un anticorps qui lie la protéine pRb2/p130.

18. Procédé selon la revendication 14, dans lequel le cancer gynécologique est un cancer de l'endomètre ou un cancer des ovaires.

19. Procédé de classement d'un cancer, comprenant :
la détermination du niveau d'expression du gène pRb2/p130 dans un échantillon de tissu d'un patient souffrant d'un cancer, le niveau d'expression étant indicatif de l'évolution du cancer, dans lequel le cancer est un cancer bronchique non à petites cellules.

20. Procédé selon la revendication 19, dans lequel la détermination du niveau d'expression du gène pRb2/p130 comprend la détermination du nombre relatif de transcrits d'ARN du gène dans le tissu prélevé, relativement à un nombre standard desdits transcrits.

21. Procédé selon la revendication 19, dans lequel la détermination du niveau d'expression du gène pRb2/p130 comprend la détermination du niveau relatif de la protéine pRb2/p130 correspondante dans le tissu prélevé, relativement à un niveau standard de ladite protéine.

22. Procédé selon la revendication 21, dans lequel le niveau de protéine dans le tissu prélevé est déterminé par un essai immunologique, moyennant quoi un anticorps qui lie ladite protéine pRb2/p130 est mis en contact avec ledit tissu prélevé.

23. Procédé selon la revendication 19, dans lequel le cancer bronchique non à petites cellules est un cancer des cellules d'épithélium malpinghien ou un adénocarcinome.


### SEQUENCE LISTING

(1) GENERAL INFORMATION:
   (i) APPLICANT: Thomas Jefferson University
      INVENTORS: Giordano, Antonio
      Baldi, Alphonso
   (ii) TITLE OF INVENTION: METHODS FOR THE DIAGNOSIS AND PROGNOSIS OF CANCER
   (iii) NUMBER OF SEQUENCES: 116
   (iv) CORRESPONDENCE ADDRESS:
      (A) ADDRESSEE: SEIDEL, GONDA, LAVORGNA & MONACO, P.C.
      (B) STREET: Suite 1800 Two Penn Center Plaza
      (C) CITY: Philadelphia
      (D) STATE: PA
      (E) COUNTRY: USA
      (F) ZIP: 19102
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.30
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Monaco, Daniel A
      (B) REGISTRATION NUMBER: 30,480
      (C) REFERENCE/DOCKET NUMBER: 8321-13 pc
   (ix) TELECOMMUNICATION INFORMATION:
      (A) TELEPHONE: (215) 568-8383
      (B) TELEFAX: (215) 568-5549
(2) INFORMATION FOR SEQ ID NO:1:
   (i) SEQUENCE CHARACTERISTICS:
      (A) LENGTH: 4853 base pairs
      (B) TYPE: nucleic acid
      (C) STRANDEDNESS: double
      (D) TOPOLOGY: linear
   (ii) MOLECULE TYPE: cDNA
   (ix) FEATURE:
      (A) NAME/KEY: CDS
      (B) LOCATION: 70..3489
   (xi) SEQUENCE DESCRIPTION: SEQ ID NO:1:
   TTCGCCGTTT GAATTGCTGC GGGCCCGGGC CCTCACCTCA CCTGAGGTCC GGCCGCCCAG 60
   GGGTGCGCT ATG CCG TCG GGA GGT GAC CAG TCG CCA CCG CCC CCG CCT        108
             Met Pro Ser Gly Gly Asp Gln Ser Pro Pro Pro Pro Pro
               1               5                  10
   CCC CCT CCG GCG GCG GCA GCC TCG GAT GAG GAG GAG GAG GAC GAC GGC      156
   Pro Pro Pro Ala Ala Ala Ala Ser Asp Glu Glu Glu Glu Asp Asp Gly
       15                  20                  25
   GAG GCG GAA GAC GCC GCG CCG TCT GCC GAG TCG CCC ACC CCT CAG ATC      204
   Glu Ala Glu Asp Ala Ala Pro Ser Ala Glu Ser Pro Thr Pro Gln Ile
   30                  35                  40                  45
   CAG CAG CGG TTC GAC GAG CTG TGC AGC CGC CTC AAC ATG GAC GAG GCG      252
   Gln Gln Arg Phe Asp Glu Leu Cys Ser Arg Leu Asn Met Asp Glu Ala
                   50                  55                  60
   GCG CGG CCC GAG GCC TGG GAC AGC TAC CGC AGC ATG AGC GAA AGC TAC      300
   Ala Arg Pro Glu Ala Trp Asp Ser Tyr Arg Ser Met Ser Glu Ser Tyr
               65                  70                  75
   ACG CTG GAG GGA AAT GAT CTT CAT TGG TTA GCA TGT GCC TTA TAT GTG      348
   Thr Leu Glu Gly Asn Asp Leu His Trp Leu Ala Cys Ala Leu Tyr Val
           80                  85                  90
   GCT TGC AGA AAA TCT GTT CCA ACT GTA AGC AAA GGG ACA GTG GAA GGA      396
   Ala Cys Arg Lys Ser Val Pro Thr Val Ser Lys Gly Thr Val Glu Gly
       95                  100                 105
   AAC TAT GTA TCT TTA ACT AGA ATC CTG AAA TGT TCA GAG CAG AGC TTA      444
   Asn Tyr Val Ser Leu Thr Arg Ile Leu Lys Cys Ser Glu Gln Ser Leu
   110                 115                 120                 125
   ATC GAA TTT TTT AAT AAG ATG AAG AAG TGG GAA GAC ATG GCA AAT CTA      492
   Ile Glu Phe Phe Asn Lys Met Lys Lys Trp Glu Asp Met Ala Asn Leu
                   130                 135                 140
   CCC CCA CAT TTC AGA GAA CGT ACT GAG AGA TTA GAA AGA AAC TTC ACT      540
   Pro Pro His Phe Arg Glu Arg Thr Glu Arg Leu Glu Arg Asn Phe Thr
               145                 150                 155
   GTT TCT GCT GTA ATT TTT AAG AAA TAT GAA CCC ATT TTT CAG GAC ATC      588
   Val Ser Ala Val Ile Phe Lys Lys Tyr Glu Pro Ile Phe Gln Asp Ile
           160                 165                 170
   TTT AAA TAC CCT CAA GAG GAG CAA CCT CGT CAG CAG CGA GGA AGG AAA      636
   Phe Lys Tyr Pro Gln Glu Glu Gln Pro Arg Gln Gln Arg Gly Arg Lys
       175                 180                 185
   CAG CGG CGA CAG CCC TGT ACT GTG TCT GAA ATT TTC CAT TTT TGT TGG      684
   Gln Arg Arg Gln Pro Cys Thr Val Ser Glu Ile Phe His Phe Cys Trp
   190                 195                 200                 205
   GTG CTT TTT ATA TAT GCA AAA GGT AAT TTC CCC ATG ATT AGT GAT GAT      732
   Val Leu Phe Ile Tyr Ala Lys Gly Asn Phe Pro Met Ile Ser Asp Asp
                   210                 215                 220
   TTG GTC AAT TCT TAT CAC CTG CTG CTG TGT GCT TTG GAC TTA GTT TAT      780
   Leu Val Asn Ser Tyr His Leu Leu Leu Cys Ala Leu Asp Leu Val Tyr
               225                 230                 235
   GGA AAT GCA CTT CAG TGT TCT AAT CGT AAA GAA CTT GTG AAC CCT AAT      828
   Gly Asn Ala Leu Gln Cys Ser Asn Arg Lys Glu Leu Val Asn Pro Asn
           240                 245                 250
   TTT AAA GGC TTA TCT GAA GAT TTT CAT GCT AAA GAT TCT AAA CCT TCC     876
   Phe Lys Gly Leu Ser Glu Asp Phe His Ala Lys Asp Ser Lys Pro Ser
       255                 260                 265
   TCT GAC CCC CCT TGT ATC ATT GAG AAA CTG TGT TCC TTA CAT GAT GGC     924
   Ser Asp Pro Pro Cys Ile Ile Glu Lys Leu Cys Ser Leu His Asp Gly
   270                 275                 280                 285
   CTA GTT TTG GAA GCA AAG GGG ATA AAG GAA CAT TTC TGG AAA CCC TAT     972
   Leu Val Leu Glu Ala Lys Gly Ile Lys Glu His Phe Trp Lys Pro Tyr
                   290                 295                 300
   ATT AGG AAA CTT TAT GAA AAA AAG CTC CTT AAG GGA AAA GAA GAA AAT    1020
   Ile Arg Lys Leu Tyr Glu Lys Lys Leu Leu Lys Gly Lys Glu Glu Asn
               305                 310                 315
   CTC ACT GGG TTT CTA GAA CCT GGG AAC TTT GGA GAG AGT TTT AAA GCC    1068
   Leu Thr Gly Phe Leu Glu Pro Gly Asn Phe Gly Glu Ser Phe Lys Ala
           320                 325                 330
   ATC AAT AAG GCC TAT GAG GAG TAT GTT TTA TCT GTT GGG AAT TTA GAT    1116
   Ile Asn Lys Ala Tyr Glu Glu Tyr Val Leu Ser Val Gly Asn Leu Asp
       335                 340                 345
   GAG CGG ATA TTT CTT GGA GAG GAT GCT GAG GAG GAA ATT GGG ACT CTC    1164
   Glu Arg Ile Phe Leu Gly Glu Asp Ala Glu Glu Glu Ile Gly Thr Leu
   350                 355                 360                 365
   TCA AGG TGT CTG AAC GCT GGT TCA GGA ACA GAG ACT GCT GAA AGG GTG    1212
   Ser Arg Cys Leu Asn Ala Gly Ser Gly Thr Glu Thr Ala Glu Arg Val
                   370                 375                 380
   CAG ATG AAA AAC ATC TTA CAG CAG CAT TTT GAC AAG TCC AAA GCA CTT    1260
   Gln Met Lys Asn Ile Leu Gln Gln His Phe Asp Lys Ser Lys Ala Leu
               385                 390                 395
   AGA ATC TCC ACA CCA CTA ACT GGT GTT AGG TAC ATT AAG GAG AAT AGC    1308
   Arg Ile Ser Thr Pro Leu Thr Gly Val Arg Tyr Ile Lys Glu Asn Ser
           400                 405                 410
   CCT TGT GTG ACT CCA GTT TCT ACA GCT ACG CAT AGC TTG AGT CGT CTT    1356
   Pro Cys Val Thr Pro Val Ser Thr Ala Thr His Ser Leu Ser Arg Leu
       415                 420                 425
   CAC ACC ATG CTG ACA GGC CTC AGG AAT GCA CCA AGT GAG AAA CTG GAA    1404
   His Thr Met Leu Thr Gly Leu Arg Asn Ala Pro Ser Glu Lys Leu Glu
   430                 435                 440                 445
   CAG ATT CTC AGG ACA TGT TCC AGA GAT CCA ACC CAG GCT ATT GCT AAC    1452
   Gln Ile Leu Arg Thr Cys Ser Arg Asp Pro Thr Gln Ala Ile Ala Asn
                   450                 455                 460
   AGA CTG AAA GAA ATG TTT GAA ATA TAT TCT CAG CAT TTC CAG CCA GAC    1500
   Arg Leu Lys Glu Met Phe Glu Ile Tyr Ser Gln His Phe Gln Pro Asp
               465                 470                 475
   GAG GAT TTC AGT AAT TGT GCT AAA GAA ATT GCC AGC AAA CAT TTT CGT    1548
   Glu Asp Phe Ser Asn Cys Ala Lys Glu Ile Ala Ser Lys His Phe Arg
           480                 485                 490
   TTT GCG GAG ATG CTT TAC TAT AAA GTA TTA GAA TCT GTT ATT GAG CAG    1596
   Phe Ala Glu Met Leu Tyr Tyr Lys Val Leu Glu Ser Val Ile Glu Gln
       495                 500                 505
   GAA CAA AAA AGA CTA GGA GAC ATG GAT TTA TCT GGT ATT CTG GAA CAA      1644
   Glu Gln Lys Arg Leu Gly Asp Met Asp Leu Ser Gly Ile Leu Glu Gln
   510                 515                 520                 525
   GAT GCA TTC CAC AGA TCT CTC TTG GCC TGC TGC CTT GAG GTC GTC ACT      1692
   Asp Ala Phe His Arg Ser Leu Leu Ala Cys Cys Leu Glu Val Val Thr
                   530                 535                 540
   TTT TCT TAT AAG CCT CCT GGG AAT TTT CCA TTT ATT ACT GAA ATA TTT      1740
   Phe Ser Tyr Lys Pro Pro Gly Asn Phe Pro Phe Ile Thr Glu Ile Phe
               545                 550                 555
   GAT GTG CCT CTT TAT CAT TTT TAT AAG GTG ATA GAA GTA TTC ATT AGA      1788
   Asp Val Pro Leu Tyr His Phe Tyr Lys Val Ile Glu Val Phe Ile Arg
           560                 565                 570
   GCA GAA GAT GGC CTT TGT AGA GAG GTG GTA AAA CAC CTT AAT CAG ATT      1836
   Ala Glu Asp Gly Leu Cys Arg Glu Val Val Lys His Leu Asn Gln Ile
       575                 580                 585
   GAA GAA CAG ATC TTA GAT CAT TTG GCA TGG AAA CCA GAG TCT CCA CTC      1884
   Glu Glu Gln Ile Leu Asp His Leu Ala Trp Lys Pro Glu Ser Pro Leu
   590                 595                 600                 605
   TGG GAA AAA ATT AGA GAC AAT GAA AAC AGA GTT CCT ACA TGT GAA GAG      1932
   Trp Glu Lys Ile Arg Asp Asn Glu Asn Arg Val Pro Thr Cys Glu Glu
                   610                 615                 620
   GTC ATG CCA CCT CAG AAC CTG GAA AGG GCA GAT GAA ATT TGC ATT GCT      1980
   Val Met Pro Pro Gln Asn Leu Glu Arg Ala Asp Glu Ile Cys Ile Ala
               625                 630                 635
   GGC TCC CCT TTG ACT CCC AGA AGG GTG ACT GAA GTT CGT GCT GAT ACT      2028
   Gly Ser Pro Leu Thr Pro Arg Arg Val Thr Glu Val Arg Ala Asp Thr
           640                 645                 650
   GGA GGA CTT GGA AGG AGC ATA ACA TCT CCA ACC ACA TTA TAC GAT AGG      2076
   Gly Gly Leu Gly Arg Ser Ile Thr Ser Pro Thr Thr Leu Tyr Asp Arg
       655                 660                 665
   TAC AGC TCC CCA CCA GCC AGC ACT ACC AGA AGG CGG CTA TTT GTT GAG      2124
   Tyr Ser Ser Pro Pro Ala Ser Thr Thr Arg Arg Arg Leu Phe Val Glu
   670                 675                 680                 685
   AAT GAT AGC CCC TCT GAT GGA GGG ACG CCT GGG CGC ATG CCC CCA CAG      2172
   Asn Asp Ser Pro Ser Asp Gly Gly Thr Pro Gly Arg Met Pro Pro Gln
                   690                 695                 700
   CCC CTA GTC AAT GCT GTC CCT GTG CAG AAT GTA TCT GGG GAG ACT GTT      2220
   Pro Leu Val Asn Ala Val Pro Val Gln Asn Val Ser Gly Glu Thr Val
               705                 710                 715
   TCT GTC ACA CCA GTT CCT GGA CAG ACT TTG GTC ACC ATG GCA ACC GCC      2268
   Ser Val Thr Pro Val Pro Gly Gln Thr Leu Val Thr Met Ala Thr Ala
           720                 725                 730
   ACT GTC ACA GCC AAC AAT GGG CAA ACG GTA ACC ATT CCT GTG CAA GGT      2316
   Thr Val Thr Ala Asn Asn Gly Gln Thr Val Thr Ile Pro Val Gln Gly
       735                 740                 745
   ATT GCC AAT GAA AAT GGA GGG ATA ACA TTC TTC CCT GTC CAA GTC AAT      2364
   Ile Ala Asn Glu Asn Gly Gly Ile Thr Phe Phe Pro Val Gln Val Asn
   750                 755                 760                 765
   GTT GGG GGG CAG GCA CAA GCT GTG ACA GGC TCC ATC CAG CCC CTC AGT      2412
   Val Gly Gly Gln Ala Gln Ala Val Thr Gly Ser Ile Gln Pro Leu Ser
                   770                 775                 780
   GCT CAG GCC CTG GCT GGA AGT CTG AGC TCT CAA CAG GTG ACA GGA ACA      2460
   Ala Gln Ala Leu Ala Gly Ser Leu Ser Ser Gln Gln Val Thr Gly Thr
               785                 790                 795
   ACT TTG CAA GTC CCT GGT CAA GTG GCC ATT CAA CAG ATT TCC CCA GGT      2508
   Thr Leu Gln Val Pro Gly Gln Val Ala Ile Gln Gln Ile Ser Pro Gly
           800                 805                 810
   GGC CAA CAG CAG AAG CAA GGC CAG TCT GTA ACC AGC AGT AGT AAT AGA      2556
   Gly Gln Gln Gln Lys Gln Gly Gln Ser Val Thr Ser Ser Ser Asn Arg
       815                 820                 825
   CCC AGG AAG ACC AGC TCT TTA TCG CTT TTC TTT AGA AAG GTA TAC CAT      2604
   Pro Arg Lys Thr Ser Ser Leu Ser Leu Phe Phe Arg Lys Val Tyr His
   830                 835                 840                 845
   TTA GCA GCT GTC CGC CTT CGG GAT CTC TGT GCC AAA CTA GAT ATT TCA      2652
   Leu Ala Ala Val Arg Leu Arg Asp Leu Cys Ala Lys Leu Asp Ile Ser
                   850                 855                 860
   GAT GAA TTG AGG AAA AAA ATC TGG ACC TGC TTT GAA TTC TCC ATA ATT      2700
   Asp Glu Leu Arg Lys Lys Ile Trp Thr Cys Phe Glu Phe Ser Ile Ile
               865                 870                 875
   CAG TGT CCT GAA CTT ATG ATG GAC AGA CAT CTG GAC CAG TTA TTA ATG      2748
   Gln Cys Pro Glu Leu Met Met Asp Arg His Leu Asp Gln Leu Leu Met
           880                 885                 890
   TGT GCC ATT TAT GTG ATG GCA AAG GTC ACA AAA GAA GAT AAG TCC TTC      2796
   Cys Ala Ile Tyr Val Met Ala Lys Val Thr Lys Glu Asp Lys Ser Phe
       895                 900                 905
   CAG AAC ATT ATG CGT TGT TAT AGG ACT CAG CCG CAG GCC CGG AGC CAG      2844
   Gln Asn Ile Met Arg Cys Tyr Arg Thr Gln Pro Gln Ala Arg Ser Gln
   910                 915                 920                 925
   GTG TAT AGA AGT GTT TTG ATA AAA GGG AAA AGA AAA AGA AGA AAT TCT      2892
   Val Tyr Arg Ser Val Leu Ile Lys Gly Lys Arg Lys Arg Arg Asn Ser
                   930                 935                 940
   GGC AGC AGT GAT AGC AGA AGC CAT CAG AAT TCT CCA ACA GAA CTA AAC      2940
   Gly Ser Ser Asp Ser Arg Ser His Gln Asn Ser Pro Thr Glu Leu Asn
               945                 950                 955
   AAA GAT AGA ACC AGT AGA GAC TCC AGT CCA GTT ATG AGG TCA AGC AGC      2988
   Lys Asp Arg Thr Ser Arg Asp Ser Ser Pro Val Met Arg Ser Ser Ser
           960                 965                 970
   ACC TTG CCA GTT CCA CAG CCC AGC AGT GCT CCT CCC ACA CCT ACT CGC      3036
   Thr Leu Pro Val Pro Gln Pro Ser Ser Ala Pro Pro Thr Pro Thr Arg
       975                 980                 985
   CTC ACA GGT GCC AAC AGT GAC ATG GAA GAA GAG GAG AGG GGA GAC CTC      3084
   Leu Thr Gly Ala Asn Ser Asp Met Glu Glu Glu Glu Arg Gly Asp Leu
   990                 995                1000                1005
   ATT CAG TTC TAC AAC AAC ATC TAC ATC AAA CAG ATT AAG ACA TTT GCC      3132
   Ile Gln Phe Tyr Asn Asn Ile Tyr Ile Lys Gln Ile Lys Thr Phe Ala
                  1010                1015                1020
   ATG AAG TAC TCA CAG GCA AAT ATG GAT GCT CCT CCA CTC TCT CCC TAT        3180
   Met Lys Tyr Ser Gln Ala Asn Met Asp Ala Pro Pro Leu Ser Pro Tyr
               1025                1030                1035
   CCA TTT GTA AGA ACA GGC TCC CCT CGC CGA ATA CAG TTG TCT CAA AAT        3228
   Pro Phe Val Arg Thr Gly Ser Pro Arg Arg Ile Gln Leu Ser Gln Asn
           1040                1045                1050
   CAT CCT GTC TAC ATT TCC CCA CAT AAA AAT GAA ACA ATG CTT TCT CCT        3276
   His Pro Val Tyr Ile Ser Pro His Lys Asn Glu Thr Met Leu Ser Pro
       1055                1060                1065
   CGA GAA AAG ATT TTC TAT TAC TTC AGC AAC AGT CCT TCA AAG AGA CTG        3324
   Arg Glu Lys Ile Phe Tyr Tyr Phe Ser Asn Ser Pro Ser Lys Arg Leu
   1070                1075                1080                1085
   AGA GAA ATT AAT AGT ATG ATA CGC ACA GGA GAA ACT CCT ACT AAA AAG        3372
   Arg Glu Ile Asn Ser Met Ile Arg Thr Gly Glu Thr Pro Thr Lys Lys
                   1090                1095                1100
   AGA GGA ATT CTT TTG GAA GAT GGA AGT GAA TCA CCT GCA AAA AGA ATT        3420
   Arg Gly Ile Leu Leu Glu Asp Gly Ser Glu Ser Pro Ala Lys Arg Ile
               1105                1110                1115
   TGC CCA GAA AAT CAT TCT GCC TTA TTA CGC CGT CTC CAA GAT GTA GCT        3468
   Cys Pro Glu Asn His Ser Ala Leu Leu Arg Arg Leu Gln Asp Val Ala
           1120                1125                1130
   AAT GAC CGT GGT TCC CAC TGA GGTTAGTCTC TTGTATTAAA CTCTTCACAA           3519
   Asn Asp Arg Gly Ser His  *
       1135                1140
   AATCTGTTTA GCAGCAGCCT TTAATGCATC TAGATTATGG AGCTTTTTTC CTTAATCCAG     3579
   CTGATGAGTT ACAGCCTGTT AGTAACATGA GGGGACATTT TGGTGAGAAA TGGGACTTAA     3639
   CTCCTTCCAG TGTCCTTAGA ACATTTTAAT TCATCCCAAC TGTCTTTTTT TCCCTACCAC     3699
   TCAGTGATTA CTGTCAAGGC TGCTTACAAT CCAAACTTGG GTTTTTGGCT CTGGCAAAGC     3759
   TTTTAGAAAT ACTGCAAGAA ATGATGTGTA CCCAACGTGA GCATAGGAGG CTTCTGTTGA     3819
   CGTCTCCAAC AGAAGAACTG TGTTTCAAGT TCAATCCTAC CTGTTTTGTG GTCAGCTGTA     3879
   GTCCTCATAA AAAGCAAAAC AAAAATTAGG TATTTTGTCC TAAAACACCT GGTAGGAGTG     3939
   TGTGATTTTT TGCATTCCTG ACAAAGGAGA GCACACCCAG GTTTGGAGGT CCTAGGTCAT     3999
   TAGCCCTCGT CTCCCGTTCC CTTTGTGCAC ATCTTCCCTC TCCCCATTCG GTGTGGTGCA     4059
   GTGTGAAAAG TCCTTGATTG TTCGGGTGTG CAATGTCTGA GTGAACCTGT ATAAGTGGAG     4119
   GCACTTTAGG GCTGTAAAAT GCATGATTTT GTAACCCAGA TTTTGCTGTA TATTTGTGAT     4179
   AGCACTTTCT ACAATGTGAA CTTTATTAAA TACAAAACTT CCAGGCTAAA CATCCAATAT     4239
   TTTCTTTAAT GCTTTTATAT TTTTTTAAAA TGTTAAAACC CCTATAGCCA CCTTTTGGGA     4299
   ATGTTTTAAA TTCTCCAGTT TTTTGTTATA TAGGGATCAA CCAGCTAAGA AAAGATTTTA     4359
   AGTCAAGTTG AATTGAGGGG ATTAATATGA AAACTTATGA CCTCTTCCTT TAGGAGGGAG     4419
   TTATCTAAAA GAAATGTCTA TTAAGGTGAT ATATTTAAAA ATATTTTTGG GTGTTCCTGG     4479
   CAGTTTAAAA AAATTGGTTG GAGAATTTAG GTTTTTATTA GTACCATAGT ACCATTTATA 4539
   CAAATTAGAA AATGTTATTT AACAGCTGAA TTATCTATAC ATATCTTTAT TAATCACTAT 4599
   TGTTCCAGCA GTTTTCAAGT CAAATTAATA ATCTTATTAG GGAGAAAATT CAATTGTAAA 4659
   TTGAATCAGT ATAAACAAAG TTACTAGGTA ACTTCATATT GCTGAGAGAA ATATGGAACT 4719
   TACATTGTTC AATTAGAATA GTGTTCTCCC CAAATATTTA TAAAACTTCT CAAGATACTG 4779
   CTACGTGTAA TTTTATATGA AGATAAGTGT ATTTTTCAAT AAAGCATTTA TAAATTAAAA 4839
   AAAAAAAAAA AAAA 4853
(2) INFORMATION FOR SEQ ID NO:2:
   (i) SEQUENCE CHARACTERISTICS:
      (A) LENGTH: 1140 amino acids
      (B) TYPE: amino acid
      (D) TOPOLOGY: linear
   (ii) MOLECULE TYPE: protein
   (xi) SEQUENCE DESCRIPTION: SEQ ID NO:2:
   Met Pro Ser Gly Gly Asp Gln Ser Pro Pro Pro Pro Pro Pro Pro Pro
    1               5                  10                  15
   Ala Ala Ala Ala Ser Asp Glu Glu Glu Glu Asp Asp Gly Glu Ala Glu
               20                  25                  30
   Asp Ala Ala Pro Ser Ala Glu Ser Pro Thr Pro Gln Ile Gln Gln Arg
           35                  40                  45
   Phe Asp Glu Leu Cys Ser Arg Leu Asn Met Asp Glu Ala Ala Arg Pro
       50                  55                  60
   Glu Ala Trp Asp Ser Tyr Arg Ser Met Ser Glu Ser Tyr Thr Leu Glu
   65                  70                  75                  80
   Gly Asn Asp Leu His Trp Leu Ala Cys Ala Leu Tyr Val Ala Cys Arg
                   85                  90                  95
   Lys Ser Val Pro Thr Val Ser Lys Gly Thr Val Glu Gly Asn Tyr Val
               100                 105                 110
   Ser Leu Thr Arg Ile Leu Lys Cys Ser Glu Gln Ser Leu Ile Glu Phe
           115                 120                 125
   Phe Asn Lys Met Lys Lys Trp Glu Asp Met Ala Asn Leu Pro Pro His
       130                 135                 140
   Phe Arg Glu Arg Thr Glu Arg Leu Glu Arg Asn Phe Thr Val Ser Ala
   145                 150                 155                 160
   Val Ile Phe Lys Lys Tyr Glu Pro Ile Phe Gln Asp Ile Phe Lys Tyr
                   165                 170                 175
   Pro Gln Glu Glu Gln Pro Arg Gln Gln Arg Gly Arg Lys Gln Arg Arg
               180                 185                 190
   Gln Pro Cys Thr Val Ser Glu Ile Phe His Phe Cys Trp Val Leu Phe
               195                     200                     205
   Ile Tyr Ala Lys Gly Asn Phe Pro Met Ile Ser Asp Asp Leu Val Asn
       210                 215                     220
   Ser Tyr His Leu Leu Leu Cys Ala Leu Asp Leu Val Tyr Gly Asn Ala
   225                 230                     235                 240
   Leu Gln Cys Ser Asn Arg Lys Glu Leu Val Asn Pro Asn Phe Lys Gly
                   245                     250                 255
   Leu Ser Glu Asp Phe His Ala Lys Asp Ser Lys Pro Ser Ser Asp Pro
               260                     265                 270
   Pro Cys Ile Ile Glu Lys Leu Cys Ser Leu His Asp Gly Leu Val Leu
           275                     280                 285
   Glu Ala Lys Gly Ile Lys Glu His Phe Trp Lys Pro Tyr Ile Arg Lys
       290                 295                     300
   Leu Tyr Glu Lys Lys Leu Leu Lys Gly Lys Glu Glu Asn Leu Thr Gly
   305                 310                     315                 320
   Phe Leu Glu Pro Gly Asn Phe Gly Glu Ser Phe Lys Ala Ile Asn Lys
                   325                     330                 335
   Ala Tyr Glu Glu Tyr Val Leu Ser Val Gly Asn Leu Asp Glu Arg Ile
               340                     345                 350
   Phe Leu Gly Glu Asp Ala Glu Glu Glu Ile Gly Thr Leu Ser Arg Cys
           355                     360                 365
   Leu Asn Ala Gly Ser Gly Thr Glu Thr Ala Glu Arg Val Gln Met Lys
       370                 375                     380
   Asn Ile Leu Gln Gln His Phe Asp Lys Ser Lys Ala Leu Arg Ile Ser
   385                 390                     395                 400
   Thr Pro Leu Thr Gly Val Arg Tyr Ile Lys Glu Asn Ser Pro Cys Val
                   405                     410                 415
   Thr Pro Val Ser Thr Ala Thr His Ser Leu Ser Arg Leu His Thr Met
               420                     425                 430
   Leu Thr Gly Leu Arg Asn Ala Pro Ser Glu Lys Leu Glu Gln Ile Leu
           435                     440                 445
   Arg Thr Cys Ser Arg Asp Pro Thr Gln Ala Ile Ala Asn Arg Leu Lys
       450                 455                     460
   Glu Met Phe Glu Ile Tyr Ser Gln His Phe Gln Pro Asp Glu Asp Phe
   465                 470                     475                 480
   Ser Asn Cys Ala Lys Glu Ile Ala Ser Lys His Phe Arg Phe Ala Glu
                   485                     490                 495
   Met Leu Tyr Tyr Lys Val Leu Glu Ser Val Ile Glu Gln Glu Gln Lys
               500                     505                 510
   Arg Leu Gly Asp Met Asp Leu Ser Gly Ile Leu Glu Gln Asp Ala Phe
           515                     520                 525
   His Arg Ser Leu Leu Ala Cys Cys Leu Glu Val Val Thr Phe Ser Tyr
       530                     535                     540
   Lys Pro Pro Gly Asn Phe Pro Phe Ile Thr Glu Ile Phe Asp Val Pro
   545                 550                 555                 560
   Leu Tyr His Phe Tyr Lys Val Ile Glu Val Phe Ile Arg Ala Glu Asp
                   565                 570                 575
   Gly Leu Cys Arg Glu Val Val Lys His Leu Asn Gln Ile Glu Glu Gln
               580                 585                 590
   Ile Leu Asp His Leu Ala Trp Lys Pro Glu Ser Pro Leu Trp Glu Lys
           595                 600                 605
   Ile Arg Asp Asn Glu Asn Arg Val Pro Thr Cys Glu Glu Val Met Pro
       610                 615                 620
   Pro Gln Asn Leu Glu Arg Ala Asp Glu Ile Cys Ile Ala Gly Ser Pro
   625                 630                 635                 640
   Leu Thr Pro Arg Arg Val Thr Glu Val Arg Ala Asp Thr Gly Gly Leu
                   645                 650                 655
   Gly Arg Ser Ile Thr Ser Pro Thr Thr Leu Tyr Asp Arg Tyr Ser Ser
               660                 665                 670
   Pro Pro Ala Ser Thr Thr Arg Arg Arg Leu Phe Val Glu Asn Asp Ser
           675                 680                 685
   Pro Ser Asp Gly Gly Thr Pro Gly Arg Met Pro Pro Gln Pro Leu Val
       690                 695                 700
   Asn Ala Val Pro Val Gln Asn Val Ser Gly Glu Thr Val Ser Val Thr
   705                 710                 715                 720
   Pro Val Pro Gly Gln Thr Leu Val Thr Met Ala Thr Ala Thr Val Thr
                   725                 730                 735
   Ala Asn Asn Gly Gln Thr Val Thr Ile Pro Val Gln Gly Ile Ala Asn
               740                 745                 750
   Glu Asn Gly Gly Ile Thr Phe Phe Pro Val Gln Val Asn Val Gly Gly
           755                 760                 765
   Gln Ala Gln Ala Val Thr Gly Ser Ile Gln Pro Leu Ser Ala Gln Ala
       770                 775                 780
   Leu Ala Gly Ser Leu Ser Ser Gln Gln Val Thr Gly Thr Thr Leu Gln
   785                 790                 795                 800
   Val Pro Gly Gln Val Ala Ile Gln Gln Ile Ser Pro Gly Gly Gln Gln
                   805                 810                 815
   Gln Lys Gln Gly Gln Ser Val Thr Ser Ser Ser Asn Arg Pro Arg Lys
               820                 825                 830
   Thr Ser Ser Leu Ser Leu Phe Phe Arg Lys Val Tyr His Leu Ala Ala
           835                 840                 845
   Val Arg Leu Arg Asp Leu Cys Ala Lys Leu Asp Ile Ser Asp Glu Leu
       850                 855                 860
   Arg Lys Lys Ile Trp Thr Cys Phe Glu Phe Ser Ile Ile Gln Cys Pro
   865                 870                 875                 880
   Glu Leu Met Met Asp Arg His Leu Asp Gln Leu Leu Met Cys Ala Ile
                   885                 890                 895
   Tyr Val Met Ala Lys Val Thr Lys Glu Asp Lys Ser Phe Gln Asn Ile
               900                 905                 910
   Met Arg Cys Tyr Arg Thr Gln Pro Gln Ala Arg Ser Gln Val Tyr Arg
           915                 920                 925
   Ser Val Leu Ile Lys Gly Lys Arg Lys Arg Arg Asn Ser Gly Ser Ser
       930                 935                 940
   Asp Ser Arg Ser His Gln Asn Ser Pro Thr Glu Leu Asn Lys Asp Arg
   945                 950                 955                 960
   Thr Ser Arg Asp Ser Ser Pro Val Met Arg Ser Ser Ser Thr Leu Pro
                   965                 970                 975
   Val Pro Gln Pro Ser Ser Ala Pro Pro Thr Pro Thr Arg Leu Thr Gly
               980                 985                 990
   Ala Asn Ser Asp Met Glu Glu Glu Glu Arg Gly Asp Leu Ile Gln Phe
           995                 1000                1005
   Tyr Asn Asn Ile Tyr Ile Lys Gln Ile Lys Thr Phe Ala Met Lys Tyr
       1010                1015                1020
   Ser Gln Ala Asn Met Asp Ala Pro Pro Leu Ser Pro Tyr Pro Phe Val
   1025                1030                1035                1040
   Arg Thr Gly Ser Pro Arg Arg Ile Gln Leu Ser Gln Asn His Pro Val
                   1045                1050                1055
   Tyr Ile Ser Pro His Lys Asn Glu Thr Met Leu Ser Pro Arg Glu Lys
               1060                1065                1070
   Ile Phe Tyr Tyr Phe Ser Asn Ser Pro Ser Lys Arg Leu Arg Glu Ile
           1075                1080                1085
   Asn Ser Met Ile Arg Thr Gly Glu Thr Pro Thr Lys Lys Arg Gly Ile
       1090                1095                1100
   Leu Leu Glu Asp Gly Ser Glu Ser Pro Ala Lys Arg Ile Cys Pro Glu
   1105                1110                1115                1120
   Asn His Ser Ala Leu Leu Arg Arg Leu Gln Asp Val Ala Asn Asp Arg
                   1125                1130                1135
   Gly Ser His  *
               1140
(2) INFORMATION FOR SEQ ID NO:3:
   (i) SEQUENCE CHARACTERISTICS:
      (A) LENGTH: 21 amino acids
      (B) TYPE: amino acid
      (C) STRANDEDNESS::
      (D) TOPOLOGY: linear
   (ii) MOLECULE TYPE: peptide
   (v) FRAGMENT TYPE: C-terminal
   (xi) SEQUENCE DESCRIPTION: SEQ ID NO:3:
   Glu Asn His Ser Ala Leu Leu Arg Arg Leu Gln Asp Val Ala Asn Asp
   1               5                   10                  15
   Arg Gly Ser His Cys
               20
(2) INFORMATION FOR SEQ ID NO:4:
   (i) SEQUENCE CHARACTERISTICS:
      (A) LENGTH: 561 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 312..551
   (xi) SEQUENCE DESCRIPTION: SEQ ID NO:4:
   CAGCCCTGTT GAATGTTCTC ACGGTGGGGA GGTACGTGTT TAAAATACGG GGAAGGTGCT      60
   TTTATTTCAC CCCTGGTGAA ACTAGGGGAG CTAATTTTTT TAAACATGAT TTTTGTCCCC     120
   CTTGAACCGC CGGCCTGGAC TACGTTTCCC AGCAGCCCGT GCTCAAGACT ACGGGTGCCT     180
   GCAGGCGGTC AGCGTCGTTT GCGACGGCGC AGACGCGGTG CGGGCGGCGG ACGGGCGGGC     240
   GCTTCGCCGT TTGAATTGCT GCGGGCCCGG GCCCTCACCT CACCTGAGGT CCGGCCGCCC     300
   AGGGGTGCGC T ATG CCG TCG GGA GGT GAC CAG TCG CCA CCG CCC CCG CCT      350
                Met Pro Ser Gly Gly Asp Gln Ser Pro Pro Pro Pro Pro
                 1               5                  10
   CCC CCT CCG GCG GCG GCA GCC TCG GAT GAG GAG GAG GAG GAC GAC GGC       398
   Pro Pro Pro Ala Ala Ala Ala Ser Asp Glu Glu Glu Glu Asp Asp Gly
        15                  20                  25
   GAG GCG GAA GAC GCC GCG CCG TCT GCC GAG TCG CCC ACC CCT CAG ATC       446
   Glu Ala Glu Asp Ala Ala Pro Ser Ala Glu Ser Pro Thr Pro Gln Ile
    30                  35                  40                  45
   CAG CAG CGG TTC GAC GAG CTG TGC AGC CGC CTC AAC ATG GAC GAG GCG       494
   Gln Gln Arg Phe Asp Glu Leu Cys Ser Arg Leu Asn Met Asp Glu Ala
                    50                  55                  60
   GCG CGG CCC GAG GCC TGG GAC AGC TAC CGC AGC ATG AGC GAA AGC TAC       542
   Ala Arg Pro Glu Ala Trp Asp Ser Tyr Arg Ser Met Ser Glu Ser Tyr
                65                  70                  75
   ACG CTG GAG GTGCGCTCGC                                                561
   Thr Leu Glu
   80
(2) INFORMATION FOR SEQ ID NO:5:
   (i) SEQUENCE CHARACTERISTICS:
      (A) LENGTH: 80 amino acids
      (B) TYPE: amino acid
      (D) TOPOLOGY: linear
   (ii) MOLECULE TYPE: protein
   (xi) SEQUENCE DESCRIPTION: SEQ ID NO:5:
   Met Pro Ser Gly Gly Asp Gln Ser Pro Pro Pro Pro Pro Pro Pro Pro
    1              5                    10                  15
   Ala Ala Ala Ala Ser Asp Glu Glu Glu Glu Asp Asp Gly Glu Ala Glu
               20                   25                  30
   Asp Ala Ala Pro Ser Ala Glu Ser Pro Thr Pro Gln Ile Gln Gln Arg
           35                   40                  45
   Phe Asp Glu Leu Cys Ser Arg Leu Asn Met Asp Glu Ala Ala Arg Pro
       50                   55                  60
   Glu Ala Trp Asp Ser Tyr Arg Ser Met Ser Glu Ser Tyr Thr Leu Glu
    65                  70                  75                  80
(2) INFORMATION FOR SEQ ID NO:6:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:6:
      ACGCTGGAGG TGCGCTCGC 19
(2) INFORMATION FOR SEQ ID NO:7:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:7:
      TCTTTTACAG GGAAATGAT 19
(2) INFORMATION FOR SEQ ID NO:8:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:8:
      AGAGCAGAGG TAACTATGT 19
(2) INFORMATION FOR SEQ ID NO:9:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO: 9:
      TTAATACCAG CTTAATCGA 19
(2) INFORMATION FOR SEQ ID NO:10:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:10:
      GAAACAGCGG TAGGTTTTC 19
(2) INFORMATION FOR SEQ ID NO:11:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:11:
      TCCCCCAAAG GCGACAGCC 19
(2) INFORMATION FOR SEQ ID NO:12:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:12:
      ATGCAAAAGG TAAGAAAAT 19
(2) INFORMATION FOR SEQ ID NO:13:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:13:
      AATCCTGCAG GTAATTTCC 19
(2) INFORMATION FOR SEQ ID NO:14:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:14:
      ATTTTAAAGG TAGGTTTGT 19
(2) INFORMATION FOR SEQ ID NO:15:
   (i) SEQUENCE CHARACTERISTICS:
      (A) LENGTH: 18 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:15:
      ACACCATAGG CTTATCTG 18
(2) INFORMATION FOR SEQ ID NO:16:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO: 16:
      GAAAAAAAGG TTTGTAAGT 19
(2) INFORMATION FOR SEQ ID NO:17:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO: 17:
      TTCATCATAG CTCCTTAAG 19
(2) INFORMATION FOR SEQ ID NO:18:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:18:
      AGAGAGTTTG TGAGTACTT 19
(2) INFORMATION FOR SEQ ID NO:19:
   (i) SEQUENCE CHARACTERISTICS:
      (A) LENGTH: 18 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:19:
      TTCCTATAGT AAAGCCAT 18
(2) INFORMATION FOR SEQ ID NO:20:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:20:
      TTTGACAAGG TGAGTTTAG 19
(2) INFORMATION FOR SEQ ID NO:21:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:21:
      TTTTCTTTAG TCCAAAGCA 19
(2) INFORMATION FOR SEQ ID NO:22:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:22:
      GATTCTCAGG TTAGTTTGA 19
(2) INFORMATION FOR SEQ ID NO:23:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:23:
      CCTTTTTTAG GACATGTTC 19
(2) INFORMATION FOR SEQ ID NO:24:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:24:
      GTGCTAAAGG TAATTGTGC 19
(2) INFORMATION FOR SEQ ID NO:25:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:25:
      ATTTCTACAG AAATTGCCA 19
(2) INFORMATION FOR SEQ ID NO:26:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:26:
      GATTTATCTG TGAGTAAAA 19
(2) INFORMATION FOR SEQ ID NO:27:
   (i) SEQUENCE CHARACTERISTICS:
      (A) LENGTH: 18 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:27:
      ATTTTATAGG GTATTCTG 18
(2) INFORMATION FOR SEQ ID NO:28:
   (i) SEQUENCE CHARACTERISTICS:
      (A) LENGTH: 19 base pairs
      (B) TYPE: nucleic acid
      (C) STRANDEDNESS:: double
      (D) TOPOLOGY: linear
   (ii) MOLECULE TYPE: DNA (genomic)
   (xi) SEQUENCE DESCRIPTION: SEQ ID NO:28:
      TTTTATAAGG TATTTCCCA 19
(2) INFORMATION FOR SEQ ID NO:29:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:29:
      TTTATTTCAG GTGATAGAA 19
(2) INFORMATION FOR SEQ ID NO:30:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:30:
      TGTGAAGAGG TGAAAATCA 19
(2) INFORMATION FOR SEQ ID NO:31:
   (i) SEQUENCE CHARACTERISTICS:
      (A) LENGTH: 18 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:31:
      TCTTCATAGG TCATGCCA 18
(2) INFORMATION FOR SEQ ID NO:32:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:32:
      TTGGAAGGAG TAAGTTTAA 19
(2) INFORMATION FOR SEQ ID NO:33:
   (i) SEQUENCE CHARACTERISTICS:
      (A) LENGTH: 20 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:33:
      TTGACCCCTA GGCATAACAT 20
(2) INFORMATION FOR SEQ ID NO:34:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:34:
      CTGTGCAAGG TAAGGAAGG 19
(2) INFORMATION FOR SEQ ID NO:35:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:35:
      CTGTCACTAG GTATTGCCA 19
(2) INFORMATION FOR SEQ ID NO:36:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:36:
      TTTAGAAAGG TAATTTTTC 19
(2) INFORMATION FOR SEQ ID NO:37:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:37:
      TATCTCCT_AG GTATACCAT 19
(2) INFORMATION FOR SEQ ID NO:38:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:38:
      ATGGCAAAGG TGAGTACCA 19
(2) INFORMATION FOR SEQ ID NO:39:
   (i) SEQUENCE CHARACTERISTICS:
      (A) LENGTH: 18 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:39:
      GTTTGCCAGG TCACAAAA 18
(2) INFORMATION FOR SEQ ID NO:40:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:40:
      CGGAGCCAGG TAACTACAT 19
(2) INFORMATION FOR SEQ ID NO:41:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:41:
      TTCTCTAAAG GTGTATAGA 19
(2) INFORMATION FOR SEQ ID NO:42:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:42:
      AAGATAGAAG TGGGATCTT 19
(2) INFORMATION FOR SEQ ID NO:43:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:43:
      CTGGCTGCAG CCAGTAGAG 19
(2) INFORMATION FOR SEQ ID NO:44:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:44:
      CAGGCAAATG TAAGTATGA 19
(2) INFORMATION FOR SEQ ID NO:45:
   (i) SEQUENCE CHARACTERISTICS:
      (A) LENGTH: 20 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:45:
      TTTTTAAACA GATGGGATGC 20
(2) INFORMATION FOR SEQ ID NO:46:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:46:
      CCTTCAAAGG TGAGCCTAA 19
(2) INFORMATION FOR SEQ ID NO:47:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:47:
      CCCACCATAG- AGACTGAGA 19
(2) INFORMATION FOR SEQ ID NO:48:
   (i) SEQUENCE CHARACTERISTICS:
      (A) LENGTH: 3865 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:48:
   GTAGGTTTTC TTGTTGGTTC ATCAGGAATA CACATTAGTC TGTGCTGCAG TGTTGATATT 60
   CTGCTAGGTT TTTTTTTTCT GGTTTTAAAA AAGAAATAAG ATTTAAAAAA TCTTTTTCCT 120
   CAGTCGTTTT CTTTTAATGA TGCTTCCGGG GCTTCACATT GTGGGTTAGC CATGAAGAGT 180
   GGCTTTCACA TATTGCTAAA TGTATACAGG TCTGTGTTTC TATAAACTAC ATGTGTCTTA 240
   TTTCATTTTA TTATTATTTA CCTCCTCAGT GATCCTTGTT CTGAAACCTT CCTTTTTCAT 300
   TTAAGCAACA AAAAATGCAG ACTGTACAAG TCAGACTTAG GGATTTTCAC CCTTTCGCCG 360
   CCTTGGAGAG TTCTGTATCT GTATCTGGAT ATATATATTT TTTATTGCGC AGGGGCCATG 420
   CTAATCAATG TATTGTTCCA ATTTTAGTAT ATGTGCTGCC GAAGGGAGCA CTGCCCTAGA 480
   TATAGATCAC TATATTAACC ACTATATTTT CTACTAGTGA TTATATAGAC TATTTTATGT 540
   CAAACTGAGT AATAAATAAT CCCCTTGAAA TGACTTCTCT ATGTATTTTG ATGTTTATAA 600
   TGAATTCAGA ATAGAGAGAC TGGATTGGGA AAAGACAGGA GAACTGAAAC TATTATGAAT 660
   TTGTGCTTTC TGATCACTTC TGCAAAGTCT ATAAGCATGC TCTGACTCAG TGTTTTCTAC 720
   CTTTCCTGAT AGATAAAGGC AGTTATGGAA TACACATTTT CCTTCTTTAT CATTGAAAGT 780
   TTTTTCATAA AGTAGAAATG AAAATTCTAA CAATTAAAAA AATGTTGACA AGAAAAGTAA 840
   AGGGAAAGGA GTTAAAATTA TTTGGCTAGA ATAAATAATG TTTGCTTCTC TTTAAATATA 900
   AAAGTTTTCC CAGACTGTGA AGGATGTTTA CATTAAGTGT AACCTTTTAA AAATAAAATG 960
   GAATGACAAA CCAGGAGGAA AAAAAATTTA AAAAAACTAG AACTATTTAC ATTTTAATAT 1020
   AGATGGCACC ACTGATACAG AAGCATCTGG TCTAGCTCAC TTACAGTTTT GGGGAATTGA 1080
   CTATTTAAAA TGAAGCATTC TGAGCCAGGC GGGTTGGCTC ACGCCTGTAA TCCCAGCACT 1140
   TTTATGAGGC TGAGGCAGGC GAATCACCTG AGTTCAGGAG TTCAATACCA GCCTGGCCAA 1200
   CGTGGCAAAA CCCCGTCTCT ACTAAAAATA CAAAAATTAG CTGTGCATGG TGGTGCATGC 1260
   CTATAATCCC AGCTACTCGG GAGGCTGAGT CAGTTGAATC CCTTGAACCG AGAAGCAGAG 1320
   GTTGTGAGCC AAGATCGTAC CATTGCATTC GAGCCTGGGC GACAGAATGA AACTCCATCT 1380
   CATAAATAAA TAAATAAACT AATAAAATGA CATATTCTCC TAGCACTTTG GGAGGCCGAG 1440
   GCAGGTGGAT TGCTGGAGGT CAGGAGTTCA AGACTAGCTT GGCCAATGTG CCAAAACCCC 1500
   ATTTCCATTA AAAATACAAA AATTAGGCAG GTATGGTGGT GTGTGCCTGT TGTCCCAGTT 1560
   ACTTGAGGGC TGAGGCAGGT GAATCACTTG AACCCAGGAG TCGGAGGTTT CAGTGAGCTG 1620
   CGATCGCGCC AATGCACTCC AGCTTAGGTG ACAGAGTGAG ACTTCGTCTC CAAATAAATA 1680
   AATAAAAAAT GAAGTATTCT AAAGGTTTGA ATAGAAGCTT TGTACTGAGT CTGAGTGAGG 1740
   CCAATGTGAT CATTTATGGG AAGATATCTT CTTTGTTTGG AGTATCTGGA AAATAATTTC 1800
   AGATTGCACT TGTTTTGCTA TTTCTTAGGA TATATATACT ACCTAATTCT AATTAAGAGA 1860
   ATTTTAAAAG GCCATGTGCA GTGGCTCACA CCTGATCCCC AGCACTTTGG GAGGCTGAAG 1920
   TGGACAGATC ACTTGAGCCC AGGAGTTTGA GACCAGCCTG GACAGTATGG CGAAACTTCA 1980
   TCTCCACAAA AAATACAAAA ATTAGCTTGG AGTGGTGGCG CACACCTGTG GTCCCAGCTA 2040
   CTGGGGAGGC TGGAGGTGGG GGGATCACTT GAGCCTGGGA GGTTGAGGCT GCAGTGAGCT 2100
   GTGCTCATAC CACTGTACTC CAGTTTGGGT GACAGAGCAA GACCTTGTTT CAAAAAAAAA 2160
   AAAAAAAAGT AAATCACTTT ATTAGAGATT TTACATTTTA ATCACTTTGT ATACTTTCTG 2220
   TTAGCTCTTT CTGTTAACTA TAGTCATAAT GTATAGCACT TACTGAGCAT TTACTTTGGG 2280
   GCAGGGACTC TTAAGACTTC AATATGTATT ACTTCAGTTA ATCCCTCTGA CAACCTTGTG 2340
   ATACTCATAC TATTGTTAGA TAGAGAAAAT TAACCGCAGA GAGGTTAAGT AATTTGGCCA 2400
   GGGTCGCACA ACCAAGCGTG GAGTTCTTAT TGAAACTGAC TGCGGGAACC CATGTGCTTT 2460
   ACTGTGACTA TATACTGCAT CTCTCACACA CTATCTGAAA ATGTGTCACT ATTTGTTTAG 2520
   CACTTATCCA CAGGAAATAC TGTCAGGTAT TATGTAGGAC ACAAGCATTT TTTAAAACAC 2580
   CAAACCCCAC AGTTTTTGTT TTCTGAGAGC TTACAGTACA GTCAGCGAGA TGAGGCAGGT 2640
   ATGAAGATTC CAGTGCATGC AATGCAGTGT GTTATAAAAG TCCCATGACT ACCAGAGGGA 2700
   ATACAGATGT AAAACTTAGG AGGAAAAGAA ATCACTCTGG ATGAGCCAGT CAGGTAAGTT 2760
   TACATGGAAT AAGTAGAAAT GGGTCTTGAA AGATGGGTAC GAGTTTGATA GGTGAATTTG 2820
   AAGATACAGA TAGCACCTTC TGTGTAGAGG AAACAAGAAA AGACAAAAGC AGTAAAGCAA 2880
   GAAGAAATGT GGGAGGTTAG TCAAGTTTTT TTTTCTAGAA TTCTCAAGTT GTAGAGCCAG 2940
   AATTAAGAGT AGCTTAAGTG TTAAGCTAAA AAAAATTGAA TTTTATTTTG GTAGGCAACT 3000
   AAAACTAGAA ATAGTTTATC ATGCGCCTAT GGTAGAGAGG ATACTTTTAA AAGCAGAACA 3060
   CTGACATTTA ATCCTTGCCA TGGAGTGGTG AACTAAGTAC AGTATTGTAC CCAAGTAGAG 3120
   TAATCTTTTG ACAGATGAAA TGACTAAGGC CCAGGTGAGC AAGTGTACCC TAGCTAATGG 3180
   CAGTGCTGGA ACTAAATCTA ATCTAATCTT CTCCACGGAA TTTCGTTCTT CTGGGCACCT 3240
   TGTTAGAATA AGGCTGTTGG GAGGTGGAGA CCACAGATTT CTTGTCTAAA AGTTGTCAGA 3300
   GGTTTTGGTA GAAAAGCCAA GCTTAAAGCA GGTCTGAAAC TTGGCAGACT ACTTGGCAAT 3360
   ATACAACAGG TACTCTTAAT GGATGGAAGT ATAAGGAATT ATAGGAAGCT CATAATTTAC 3420
   ATTAAAAAGG CCTTTTGTGA TTTGATATAG TCTGGAATAT CTTTAAGGAG GGAGGGAGGG 3480
   ATACAGGTCA TTAGCTATGA TAAAGGAGAA AAAAATAAGG ACATATCTGA CTGCATATAG 3540
   TGGTCCTGAA TCAGCATAGC ATTGCTGTGT CATCGAAAGA ACTATTTTTA TTCATTTTAT 3600
   TTTCCACCTC ACCTATCTTG CCTTCACAAA ACTTTAAAAG ATTCTTTAAG AATTTTCTTT 3660
   TCTTTGAGAT GGGCTCTTTC CCTGGTACCC AGCTATTTCC TACCAATATT TTGTTAAGGC 3720
   AGAACGTCCA CGTTTTCCAT GTGAAGCTGA ATCTGTTGTC TCTCCCTTTA ACTGTGGGTT 3780
   TTATTTTACA CCTGATTTAT AATCATTTGG GATTTTTTTT TCTGATCTTC TGGTGTCTCG 3840
   TGACTGGGGT TTTCTTCCCC CAAAG 3865
(2) INFORMATION FOR SEQ ID NO:49:
   (i) SEQUENCE CHARACTERISTICS:
      (A) LENGTH: 4576 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:49:
   GTAAGAAAAT AGTAATATTT ATTTAGATTT AATATGTCTA TTTACATTAC CAGGTATTAA     60
   TCTCGTCAAC TCCTAATATG TATCAGGAAA AGATTTCCAC TGAAAATTTT CTCAAGGGTT    120
   TTAATCCTAG ATTCTTTTTT AAGTATTGCC TTTCCATCAA AGGATCTATT GGATTTCTTT    180
   ACAATATCCA AATCTCTCTT ATTAAATGGA AAGTCCATTA ACTTCGTTGT ATACAACATC    240
   TTTCCTACCC AAAGCTACTC TCCTCAAATT ATGAGCTGAA AACACATAAT CCTGTATATG    300
   CTTGTATTGC GAACTCTATC TTCCATGAGA TGTATCTTAT TTAGTCTGAG CGCAATTACT    360
   GATCAACCTC AGAGCTGTTC AGATTTTTTT GTGTGTCTTG TTCACATAAG TATACTTAGT    420
   CAAATGCTTT TATATACTAT TTATTTTCTT TCCCTTTTTT CTTGTCTCAT TTAACCTACC    480
   CAAGGTCTGC ATTCAGTGAA ATACATGTCT CTATTATTTT TTGTCCTTTT TGTATTTATT    540
   TATTTATTTA TTTATTTGAG ATGGAATCTC ATTCTGTCTC CCAGGGCTAG ATTGTAGTGG    600
   CACAATCTCG GCTCACTGCA GGCTACACCT CCCAGGTTCA AGTAATTCTC CTGCCTCAGC    660
   CTCCCGAATA GCCGTGATTA CAGGCGCCCA CCACCATGCC CAGCTAATTT TTGTGTTTTC    720
   AGTAGAGATG GGGTTTCACC ATGTTGGCCA GGCTGGTCTC AAACTCCTGA CCTCAGGTGA    780
   TCTGCCTGCC CTGGCCTCCC ACAGTGCTGG GATTATAGGC ACGAGCCACT GCGTCCAGCA    840
   CCTTAGTATC TTTCTATGTA GAACGAATGC TCCCAGGTAG ATGGGAAAGT GCAGATATAT    900
   TATTATGTAG TCAGCTCCTG TATACCATGT GGCTTGGCCT TCGTCACTAA GATGGCTCAC    960
   TCTGAATGCA AAGTTATCAC AGAGTCTTAG GTGCTGGAAG GAGTTGCACA GGTATCACTG   1020
   AGACTCTCAT TATTAGATTA ACTAGCTTAA CTTACTTTAT TTTTTTTTGA GATGGAGTCT   1080
   CACTCTGTTG CCCAGGCTGG AGTGCAGTGG TGCGATCTCG GCCCACTGCA ACCTCTGCTG   1140
   CCCGGGTTCA AGCGATCTCC TGCCTCAGCC TCCCGAGTAG CTGGGATTAC AGGTGCCTGC   1200
   CACTGTGCCC GGCTAATTTT TTGTCGTTTT AGTAGACACG GAGTTTCACC ATCTTGGCCA   1260
   GGCTGGCCTT GAACTCCTGA CCTCGTGATC CACCTGCGTC AGCCTCCCAA AGTGCTGGGC   1320
   TTACAGGCGT GAGCCATCGC ACCCAGCCTA GCTTAACTCA GTTACTTTAT TTTCTATTTT   1380
   TATTTTTATT TTTGACACAG GATCTTGCTC TGTTGCCCAG GCTGGAGTGC AGTGGTATGA   1440
   TCTCTGCTCA CTGCAACCTC CGCCTCTTGT GTTCAAGTTG ATTCTTGTGG CTCAGCCTCT   1500
   TGAGTAGCTG GGATTGCAGG CATGCACCAT TATACCTGGC TAATTTTTGT ATTTTTAGTA 1560
   GTGTTGGGGT TTTGCCATGT TGGCCAGGGT GGTCTCGAAC TCCTGACCTC AAGTGATCTG 1620
   CCACCTCGGC CTCCCAAAGT GTTGGGATTA CAGGTGTTGA GCCACCATGC TCAATCAGCT 1680
   TAGTTACTTT AAAGATTAGG CAGCTGAGCC CAGAAACTAG CTGCTGGGAA CAAAGCTAAG 1740
   ATTGAACTCA GATCTCCTGG TTCCTGGTTC TTAGTTTCAT ACTGGCTGTG AAGGCCTCTG 1800
   GGAAGAATGT GTTACATTGT TGGTCTCCAG GTTTGATTTG TCCTGGTCCC TCTCTGGCTA 1860
   ATTAGGGTGA GAGCCGCCAT CCTTCCTTCC CTGAGCTGCA TGCTTGATTC AAGAGAAAAA 1920
   TCTTTCTTTT GTCATACATG ACACTGGCAT GTTTCTTTAA TGATGATAAA GGCGACATGA 1980
   TCAGTGGCAT GAAATAAAGG TTTTGGAGTA TATAAACCAT TTTTACAGCG GCTACAAATT 2040
   TTAGAATGTG TGACTGCTAT TATGTATGAT GGTAATCTTT TCATATGATT GTATTGGGCA 2100
   AGTATGTCTC ATTTCTAGGG TTTTTATCTG TTTTGTTTGT CTTTTATGGC ATATGTGTAC 2160
   TTAGAAGTAA ATATAGTTGG TACTATATAT AATATGTACA ATACAATAAA AAATAATTTC 2220
   ATTGTCCTTA TTTTGTTCTC ACTGGACCTG TTGGGGTGGT TTTTTCTCTG TAATTAACTC 2280
   AGTGTTTGAC TTTTATCTCA TTAATTCAGT TTATAATAAT TCCACCTTAA GAACCTTTGT 2340
   GGATTGGGCA TGTTGGCGTA TGCCTGGAAC CTAGCTACTT GGGAAGTTGA AGTGGGAAGC 2400
   GGAGGCTGCA GTGAGCTGAG ATTGCACCTC CAGTTTGGGC GAATTTGAGA CCGTGTTTCG 2460
   AAAAAAAAAA AAAAAAAAAA AGAAACTTGG TCCTTTCACA GTCCACCACT GTGATCTTTT 2520
   ATAATACACG ATGATCTTTT TCTAATAGTC ATTTAATTGC TTTAATTCAG TTCTCATTTA 2580
   TTTGGGGGAA AGGTGTACTC TTTTATAGCC ACCTTTCTAA TGACAAATAA GCCAACTCTG 2640
   GAGATGAAAC ATTTCTATTT ACTTGTTATC TTTGTTGATT AAAAGATAAA ATACCTCACA 2700
   AAGTCAGATT TATTTGTAAG GTCAGGATTT GAAATAGAAA ATACGTCATG TTGAGAGAGT 2760
   CCTAGAATTT AATTTAAATT AGATTCTGAT CTTTAGGGGC ATTTCAGCTT TTTATTAGAT 2820
   GTTACGAGTA CTGTTTTTTT TTTTTTTTTT TTTGCCTTCT ATGGCAAGTG CACACCAGTA 2880
   ACAAGTTTAG GCTTGTTGGT GTGATGGGCT TTGTAGCTTG AAATCAGTAG GTGCTACTTA 2940
   CTTACTTTTT TACACATGAG GAACCAAGTA TATTTTAATA TTAAACCTCT TTATAGGAGA 3000
   GCCAAGCAAG TTGGTTTGGC TGTATCAATG CGCAGTTTGA TGTGGTGATT ATCGTTTGCC 3060
   TGCTTTGGCA GAGGAGGATT TTTTTTTCTC TTTAGTTCAT TTAAGTTGAT TTGTTGAATG 3120
   TTTCCATCTA AACAAAAAAG AATTGCTTTG TATACGCTGA GGTAAGTGGT AACTTTCTTT 3180
   GGAGGAACAG AGAGAAAGGG AAACCTGAAA CAAAACTGCA GGTGTGTGTG TGTGTGTACA 3240
   TGTACACTTG GGTAGGCGTT AAGTGTGAAA TGCTGAGGTT TGGAAATAAT TCTTCATATG 3300
   TATGTTAGCT TATTTAAATT GAATTTATCT GATGATACAA GAATGTAAAA TCACCATGAA 3360
   GCATACATGT GCAGTGTTTA ACTAAAAAAG GATGGGCTTG AAGTTATAAA ATAACTAGAA 3420
   ATAATTCTTA ATTTCTAGAA AATTAAGATA ATAATAAAAT GGTTTAACTA CACGTAAAAA 3480
   TGTGTTCAGT GTTAGAGTTC AACCAGCACT GCAGAAAATT ACATGTTTCT GTCAGTTTAG 3540
   GTTTTTGATT TCTTATTTCC CTGTTACCAA GCATCAGCAA TTATTCTTGG GATTATTAGC 3600
   CCTGGAATTG AAAGATATTT AATGGTACTC CTGTTGCATT AATTTGTCTG AGTTTATGTA 3660
   GAAAAGTATT AAAAATGTTA CTGTTGGAGT CTGATAAAAA GTTCTGGTCT TTTAAAAATA 3720
   TGTGTATGAG AAATAGCATG AACTCAGGAG GCAGAGCTTG CAGTGAGCTG AGATCGTGCC 3780
   ACTGCACTCC AGCCTGGGCG ACAGTGAGAC TCCATCTCAA AAAAAAAAAA TGTATATGAG 3840
   AATAATTAAG TGAATTATTT TTTCGGCTGT CTCCTAAGTA TTTCTAATAA TTTTCATGAC 3900
   AGAAAAATGT TTTCATGCAA AACAATTTCC TTACAGTTTG AGATAATTTA TAAATGTTTT 3960
   GTGTTCAGAA TTTTCAAAGA AAAGACCAAT GATAAAGTTT TATTCAGCTA CTAGGTATTT 4020
   AATAAACACT TAATGATGAA TGGCATTTTT AGTAAAGTTA TAGTTTTCAC TAAGCTGTTA 4080
   GACATTTATT AATTTATTAA AGGCCAGGCA TGGTGGTTTA CACCTGTAAT CCTAGCACTT 4140
   TGGGAGGCCA AGGCAGAAGG ATCACTTGAG TCCAGGAGTT CAAGACCAGC CTGGGCAACA 4200
   TAGCAAGACT CCATCTCTAA AAAAAGTTTT TAAATTAGCC ATGTGTGGTG GCGTGTACCT 4260
   GTAATTTGCA GCTGCCCAGG AGGCTGAGAC AGGAAGCCCT TGAGCCCAAG AGGTTGAGGG 4320
   TGCAGTGAGC CATGATCATA CCACTGTACT CCAGCCTGGG TGACCCACCA AGACTCTGTC 4380
   TCTTGAAATA AATAAATAAA GAAATTTATT AAGATATTAG AGTAATATGT CGGATGTAAA 4440
   TTTGCCAAAA CACTTATTGT AATGAGTCAA TTTTGTACAA TTGTTTTGTA ATGTCATAAT 4500
   AAGAAAGGAA GAAATTTTTT AAAAATGTTA CAAAGTCAAT GCTAATTTAA CTCTGTAACT 4560
   GCTTATAATC CTGCAG 4576
(2) INFORMATION FOR SEQ ID NO:50:
   (i) SEQUENCE CHARACTERISTICS:
      (A) LENGTH: 1618 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:50:
   GTAGGTTTGT AAATCAAAGA TTTTTGGGCA ATCTGCGTTT CTGTGTTATG TTTACCCTTG 60
   GAGTTGTACA GGTTTCCTAG CATCAGTATT TTGAAGAGCT CCTGTCATTA CGGCTATCCA 120
   GGGTACTTAT AACTAAGAGT CAAGCTGCCT GTAAAAATAT TTTTGGATAA ACAGTTGCAG 180
   ATACCACAAA GTTTAAAGTC TTAAATGACA ACTTCAAGAA GTTTCTGAAA TATATACTCA 240
   ACAAGGAGAA GGCATTTAGA AACTCAGAGT TGCGAAGATG ACATTAAAGC CGATAATGTT      300
   TCCTACATTG GCAAACTTTG TGCCTGACAC ATTGTAGGAG ATCAAAAAGA ATTTGTTGAA      360
   AGAATCTTAC TTCAAATTTT GGTACAGAAG AATAGTTATG GTTCTAAAAT AAAGAAAATG      420
   AACTTTCATC TTTTAAACTA ACAGATATAT GGAAATGATG ATTTTGGCAT TGCATTTAAT      480
   AGAACTTAGG TATATAATTT CTATGAATGA TAAACAGTTA CAAGCCCAAA TTATGATTTA      540
   CAAAGCAAAT ATTAAAAAGT ATGTATAGAG TTAAAATAAA TATTGCTGCT GCTATTTGAG      600
   TAATATTGTA ATAGGATTCT GGGTGATTCT CAGTTTGGAG GTAATTTCAG TTAAAATTTC      660
   AGCTTGTCTA TCAAGGTAGA TTTTTAAAAT TAGTGGAGTT CAGTTGCTCC TGGTATGGTA      720
   AATTTAATGT TCCTCATCTT CTTTTCTGTT CTTTCTCTCA TTTCTATCAT AACTCCCTTG      780
   TATATTCCCA AAAAGCTGCT TCCTTTCACT TTTATCTTTT TTTGGTTTTA AATTAAAAAG      840
   AATTTTTTTT TTGGAGACAG GGTCTCACTC TGTCACCCAG GTTGGGATGC AGTGGTGAAA      900
   TCACAATTCA CTGCAGCCTC AATCTCCTGG GCTCAGATGA TCCTCTCATC TCAGCCTCCC      960
   AGGTAGCTGG GACTACAGAC ATACACCACC ACACCCAGTT AATTTTTTTG TATTTTTCAG     1020
   TATAGATGAG GTTTCACCAT GTTTCCTGGG TTGTCTCAAA CTCCTGGACT CAAGCGATGT     1080
   ACCCACCTTG GCCTCCCAAA GTGGATTATA GGAATGGAGC CACTATGCCC AACCTTTACC     1140
   TCTTTTATTT TTAGTTGATT TTTTTTCTTT TGTGCTGAGT CTAGGGCAAG AATAAATTGT     1200
   AAACTAGTAT GAAATACATC TAATACATTC AAATTAAAGA TATAAATATC TGAACAGTGT     1260
   AATTTTTTAA AGTGGTGTTT TTTGTTTAAA AGTAGACTTA CTTGCAAAGT TGTATTTTGT     1320
   GGTTTTTAGA TCTTAGTATC CTAAAATTTG ATTACCTAAA ATTTAAGTTT TAAGTTTCCC     1380
   TTAACCATCT CTACATAAAT AATTGAATAA CTGAAATCTT TCGAGTAATG ATACACTTTA     1440
   CTTCTATTTG CCATTTTTTG ACAAATTCTT AGTGTTGAAA TAGGCCCATA TATACTGTTT     1500
   CCTATACATT TGTATGCTAA GTGGTATACT GATTATACTC TATGTTTTAC ATTTTAGTTT     1560
   ATTACAAATT GGCTTATTGT GTGCTGATAT CTCTGTTTTG TGATTCTATA CACCATAG       1618
(2) INFORMATION FOR SEQ ID NO:51:
   (i) SEQUENCE CHARACTERISTICS:
      (A) LENGTH: 92 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:51:
   GTTTGTAAGT AGCAAAGAAA TAACGTGAAA ATGTTTTCTG GAGAAAAACT TGATTTAACA       60
   TGACGACTTA AGGATCTCTT CTTTCATCAT AG 92
(2) INFORMATION FOR SEQ ID NO:52:
   (i) SEQUENCE CHARACTERISTICS:
      (A) LENGTH: 889 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:52:
   GTGAGTACTT CTGTATAAAA TGTTTTAATA TTTTAAATTG TATACTTAGG AAACTTCAGA 60
   AGTTAGTGTT TTTATTGTTT GTACTCTGGA AACTGAGAAT ATGTTTTGTG AGAGAATACA 120
   GGGAAGCAAA AATTCTGTCA CCTAAATATA AGCACACTTT TTAAATGTGT TCAAAATTGT 180
   ATGGCTGTCT CCGAAGTTTC TTTAAGCTTC TGGATTATAA ATTCTGAAAT AAATTCTCTG 240
   GGAACTATAT GGGTGAAAAT TGATGATGTG TAAGTGTGGA AAGTCTTCAG GGGTGCCTAG 300
   AGCAGCTAGA CAGATAGTTA AGCTTCTCAC CGGAAGTTGC ACCTACCAGC AGCTGAAACA 360
   CTGTCAGCAA AAATACTTGT CCTGTGTGAT GGATGAGCTT GGGGATAGCA GGATTACATG 420
   TGATACTATC CAGTTTTTGT TTTGTTTTGT TTTTTGAGAT GGAGTCTCGC TGTGTCGCCC 480
   AGGCTGGAAT GCAGTGGCAT GATCTCGGCT CACTGCAACC TCTGCCTCCC AGGTTCAAGC 540
   GATTCTTCTG CCTCAGCCTC CTGAGTAGCT GTGAATACAG GCACGTGCCA CCATGCCCAG 600
   CTAATTTTTG TATTTTTAGT AGAGACAGGG TTTCACCATA TTGGCCAGGC TGGTCTCAAA 660
   CTCCTGACTT CGTGACCACC TGCCTCAGCC TCCCAAAGTG CTGGGATTAC AGACGGGAGC 720
   TACTGCACCC AGCTATACTA TCCAGTTCTT ATAACTACAA GTTACCCTAC CAAAGTTTAA 780
   CTTTCCAAAA AACTATTAGA ACTTTTAGTA AATAAAAAAA TGAAATAATT AATTGAAATG 840
   GCAGTTTCTG TGAGAGAGTA CATTTTGTCT GTATTTGTTT TTCCTATAG 889
(2) INFORMATION FOR SEQ ID NO:53:
   (i) SEQUENCE CHARACTERISTICS:
      (A) LENGTH: 4586 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:53:
   GTGAGTTTAG CCATGCCAGA AGAGTAGAAA TACCAGGAGC AGGTAAGCCA GGGGTTCTTT 60
   TTTATTTGGG TAATTTCATG TTTGTGTTTT ACTTGCCTAC AGTATGAAGG AGAAAATTCT 120
   CATCATACTT CTCTTAATTG AAAAAGGTAT CTCTATGATA TTTGCTTTGT TAATATCAAC 180
   TTTCATTCAT TTTAGTGAGG TCTGAGAAAA GAAATTAATA TAAATTTAAA ACAAATGTGT 240
   CATGCTGATA ATTGTTGGTT TTAAAAAGAT GGGCCAGTAA TATATGGTCT TATATGTAGT 300
   GAACATAGTG TAGGCATTTA GAAAGTGATA ATTGACCTGA CTGGGGCCTT CATTTAAGAG 360
   ACTGGAGTAA AATGAGGATC TACAGTCTTT AAGAAAATTC TTTCAAACTG AATTTCAGGA 420
   CCACGTGGTA TTATTTCTAA CAGACACTTA GAGTGATGCA GGCCAAGAGT TTCCCTCCTG 480
   CTATGTGGTG GAACAGAAAA CACCAAACTT CTGGAAAGTG CCACCAGGGG AAACACTGGG 540
   TAATCCAAGG GCCAGTTCAC CTGGATAGTG AGCTGCTTCA GACTTGAGAC TGGTCTGCTT 600
   ATTCATTCAA CAGATATTCC TAAAGCATTT TATATGTCAG GTTGTGTCCT GGACACTGGA 660
   GATAAAGCAG TGAACAAAAT AACCACGAGA ACCCTGTTCT AAAGAAGCTT ATATTCCAGT 720
   GTGGGGAGAT GGACAGGAGA TAAACAAGTA AATATATAGT ATGTTGGGTG ATGATAGATG 780
   AAGAAAATAG AGTAGTAATA CAAAATATTG AGGGGAGGGG AGAATGGGAT GGCTGGGCTG 840
   TGGTAGGTAA GGTGGTTGGG AACGGTGTCA CACACCAGAA GTAAGTGAGG AAGCAAGCCA 900
   TATGAATAGC TGGGTAAATG TATTTGAAGC TGAGAGCATA ACAAATGCAA AGCCATGAGG 960
   TTGGAACAGG ATTAGCTTTT TGGAGGAACA GTGAGAATGC TAGTGTGGTA GGAATAGAGT 1020
   GAGGGAAAAA GTGGTAAGAA GTGACGGGAG GCCAGGTGTG ATGGCTCATA CTTGTAATCC 1080
   TAGCACATTG GGAGACTGAG GCAGAAGACT GCCTGAGCCC AGGAGTTCAA GACTAGTCTG 1140
   GGCAACAAAG TGAGACCCCG TCTCTACATA AAATATTAAT ACAAAAAATA AGCTGGCCAT 1200
   GGTTGTGTCC ACCTGTGGGC CCAGCTACTT GCGAGGCTGA GTTAGGAGGA TTCGTTGAGC 1260
   CCAGGAGTTC CAGGCTGCAG TGAGCCGTGA TCGCGTCACT GCCCTCCAGC CTGGGTGACA 1320
   GAGCAAGAGC CTGTCTTTAA AAAAAAAGAA AAAAAGAAGA AGAAAAAGAA ATGCAGGGAA 1380
   GAGGGAACAA GAGAGCCAGA CAGACCGTGT AGGCTTTGGA AGCCATCGTA AGGACTTTTG 1440
   CTTCTGCTCT GATTGAGGTG AAAGCCATTA AGAGGGTTAT TAAGAGGAGT GACTGATTTA 1500
   CATTTTTAAA GGTCTTCTGG GAAAGTGGGA TTAGAGGCAA GGGTGGAAGT AGGGAGTTAA 1560
   GAAGCTATTG GAATGATTCT GGCAATAGTT TATGGTGGCT TGCTTCAGAA AATGGTTTGT 1620
   AGCTGGGCCA TATTTTGGAG ATGGCACCCA CAGGATTTAC CGAGGGTTTG TATCTAGGGT 1680
   ATGAGAAAAA GAGAACAGTG ATGTCTCCAG TTGGGTGAAT GATATAAAAG CTAAAATCCT 1740
   GACAAGTGCC TGTAATGTTG TAAGTTATCT GGCCCTGGCT CTCTCTGAAT TCATCTACTT 1800
   TCCTCCCTCC TCACCCACTT ATGCCACATT AACCTCCTTT TTTGTTCTTC AGATATGCCA 1860
   GGCATGCCTG CAACACAAAG CCTTTGCCTT TGCAATTCCC TCTGCCTAAA CTGTATTGCT 1920
   TCAAGAGATT CATGTGGCTT CCTTCTCACT TCATTCTGGT CTCTGATAAC CCAACTGCTA 1980
   TGTCAATAAT AACCACAACA TCCTCCCCAA CCCTCAGGAC TTCTTTTCCC CCTGACTCTG 2040
   CTTGCTAGTG TTTCTCTTCG TATTTATCAC TGTCTGACAG TAAGTACGGA CGTACGTACA 2100
   AAAGAATTGT TTATTACCTG TCTCCTTGCA TTAGAATATA AGCTTCACCA AGGCTGTGAC 2160
   CAGTGTTGTA TGCAGCGCTT GGCACATAGT AAACATTCGG GGAACATTTA CTACTGAAAT 2220
   TTATTAACCA GGGAACAAGT CTGGGGGAAC GGGAATCAAC AAGTTACGGT TATTACCATG 2280
   TTAAATTACA GATGTCTTTT AAGCATCCTA CTAGAGAAGT TGAATACACA CTTGAGGTAT 2340
   ACAAGACAGG AGTTCACAGT TCACACTACA GGTTAGGGGT TGTGTATATA TGTCCTGGGG 2400
   TCATCAGGGT GGGTACAGAT AGCCTTTTTT TTTTTTTTTT TTTTTTTTTT TTTTTTTTTT 2460
   TTTTTTTTTG AGATGGATCT CGCTCTTCAC CCAGGGTGGA GTGCAGTGGT GCAATCTTGG 2520
   CTGCAGCTGT GACCTGTGCC ACGGTGGGTT GCAAGGGATT CTCCTGCCTC AGCGTCGTGA 2580
   GTAGCTGGGA TTACAGGTGC CTGCCACCAT GCCCAGCTAA TTTTTTGTGA TTTTTGAGTA 2640
   GAAACGGCAT TTCACCATCT TGGCTAGTCT GATCTTGACT CCTGCCCTCA TGATCTTCCC 2700
   ACCTCGACTT CCTGAAGTGC TGGGATTATA GGCGTGAGCC ACCATACCCA GCCGTAGATG 2760
   GCTGTTAAAG CTATAAAATG AGGAGGGATT ACTTAGAGGT ATGAATTGAG AGAGAATACA 2820
   AGAGGTCTAA GGACAAAGCT CAGGGTCACT CCAAATTTTG TAAGTCTTCA TTTGGAGATG 2880
   GAACATCCTA ATATTTTTAA GATACCGACT TAATATTTGC ACCCAAGTTA AAGATCCTCT 2940
   TGATCAGAAT GAACAGGAAG CTTTAAGCTA AGCACAGTGC TACCAAGAAG CACCATGTTG 3000
   ACCTTGAGGA CTCTGGCAGG AAGCTGTTTG TGGTTGTCAC ACCTAGTTTC CTCTGTGAAA 3060
   CTACTGCTGC CTGTGGGTGA TGTGGTTATA TGCTGCTGGC TGCTGTTGAT TCTCCTGTTT 3120
   GTGTACAAGG TGTTTTTCCC TCCCAGTACC TCCCAATGTA GGCATCGGTT CATGCACAGT 3180
   GAAGTAGTTG CCTGCGAGAA ACCTTGTAAG GCAGGGAGCA GCCTTTTGAA TGCAATAATC 3240
   TACCCGAATC ATTTTAATGA CTTAATTATA GAATGAATTT CTTTGAGACA AAGTGAAAGT 3300
   CTTAGTTGTA TTACACTTTT AGACATAGAG GAGACATGTA GGTTTGTTTC TGTATACAGT 3360
   AAATTTCTGT GCTTTTCTAT ATCTTATGAA ACTTGAATAG TTGGCTCTGT TGCCAGGTGA 3420
   AAGTTTTGCT AGGTTTTTTA GGAAATTAGG ATGAGTACAT TTAAGACACA GGGAAATTTT 3480
   ATCTTGAATA GTAAAAGACA TTGTTAAGCT ATCGATTCCT TTCAGAGTTT ATTTGGAAAA 3540
   TCAGAGAGAT GTTTTACTGG CTCCTTTGAC ACCAAGTCAC ATCTTCTCCT AATTTATTGT 3600
   GAAGAATGTT GACATTAACT TATTTCTCTG AAGACCTGTC TACCTTAGGG GGCTGTTCTG 3660
   CATCAAGTTG CCTTTTTAGG GGATGTACAA CTTATTATCT GTCTCTGAAG CAAATATGAA 3720
   TATTTGGATG GTGGGTGTAT TAATTCATTT TAACACTGCT GATAAAGACA TGCCCCAAAC 3780
   TGGGGAACAA AAAGAGGTTT AATTGGACTT TACAGTTCCA CATGACTGGG GAGTCCTCAG 3840
   AATCATGGTG TGAGACGAAA GGCACTTCTT AGGTGGCGGT GGCAAGAGAA AAATGAGGCA 3900
   GAAGCAAAAG TGGAAACCCC TGATAAGACC GTCAGATCTC GCGAGACGTA TTCACTATCA 3960
   CAAGAATAGG ACGGGAAAGA CTGGCCTCCA TAATTCAATT ACCTCCCACT GGGTGCCTCA 4020
   CACAGCACAT GGGAATTCTG GGAAAAACAA TTCAATGGGA GGCTTCGATG CAGACATAGC 4080
   CAAACCATAT CAGTAGGCTT TTGTTAAATC ATGGATTTTT TTTGGAACCA AATTTAATCA 4140
   CAATTTTCTT TTATCTTTGA GTGTCTCCCA AAATAGCAGT AGATGGGAAT TGTGAAATTC 4200
   TGTTTCTCAG AGCTGAGAAT AATCTTAATT TTTCAGGTGA GCAGAATGCT TATCTTTGCC 4260
   TCCGAGCATA AGTTTTACAA GAGGGTATGT AGGGAGCTGT ACCTTATTTT AGAGTTTTAA 4320
   CTTTTAAGAG ACAAACTTTT AGTTAGCTAA AATACAAATT ATTCTTTCAC ACCTTCGTCT 4380
   TCACATGGAT ATTGGCGGCT CTTAATGCTG TTATGTTTAA ATTCCAAAGA ATGGTGACAT 4440
   TTGAGTCACT AAAATTTATT GATATTGTAA AGATAAAGTC TATCTGGCTT GAAGTCCCAT 4500
   TTGTGAAGTG AATTAAAGTC TTTCTGGCCT AAAATAATGT TCTTTAAAAA ATGTTTATTA 4560
   ATTCTGTGTA ATTTTTTTTT CTTTAG 4586
(2) INFORMATION FOR SEQ ID NO:54:
   (i) SEQUENCE CHARACTERISTICS:
      (A) LENGTH: 2127 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO: 54:
   GTTAGTTTGA GCCCTGTCTG CTTTCTAAGA TTTGGTTATT GACCATTTTC CAATTTCCTA 60
   TTCTTTCATT ATTAATGCCT TAATTCACCC ATGAATAATT TTTTATCAAT TGTATACTCA 120
   GTCCTGTTGT GAGTCTATAG AGGACCTAGC AATAAGATGT ATAAGTGGAA GATCTTCTTT 180
   CCTTAGATTT CTTTAATATA ATACAAGACA CAGTAACTAA TAACACCAGA CAGTGTAGAG 240
   TAAAACACAA AAGTGTCTTA TTGCCAACTG TTCTTTCAAG ATTTCAGGGA GTGGTGACGT 300
   GGCGGCGGGG GGAAGCTCAG TGATGATGGG AATAATTGTC AAAGGACTTT ATGAAGAGGG 360
   TTGACCTGAG GTAAGTTCTG AAGGGTGACT CAGATTTGCC AAGATTAATA GAGTTCCACA 420
   TGTTCATAAA GCAGGACAAA AACCACTGTA ACTTTTGTAA GCTCTATAAA ACATCCTTAT 480
   CCTGGAAAGG AAGTTGACTG CATTTAGCTC CTTTGATCTC CCTGAGACTG GTAGGAATAT 540
   CATTGAGTTT TAATTAAAAG CCCAGTAGGC TGAATCTCAT CATCTTATGC ATAACCTTTG 600
   GCAAGTTGAT TTGAAAAGCT ACCTCCAAGG TCCCTCTCAG TCCTAAAACC TTATGATATG 660
   ATAACGTTGA CCCAAAAGGA CCCCATTTCT TTTCTGATGA TGGTATATCA AGAAGACCCT 720
   ATATGTACAC ATAAGTAATT TCCCACTCAT AGCCAGGCTT CTTAAATGCC AACTACTTTT    780
   CCTTTAACAT TTCAGTGAAG TCTGCTTTAT TCATAAACTT GATTGTGATT TATACTCAAC    840
   AAGTTATATC TCTGTGGCCT CTTCCTGAGT CATGTTTTTC AGATGCACCT TGTTTGGCTT    900
   GAATTTAGAA GCATTTCGTA AATACATTTC AGAAGCCATC TTAATCTCTG TGTCTTCCAG    960
   ATCGCTTTAC AGTTTCTAAC TAGGCATAAC AGCATTTTAA ATCTTAGGGA CCATTAGTGG   1020
   GGTTAAATAA TTATTACCAG TAAATACTAG GTAAAATAAA GGGTGCTATT TTTGCTGAAA   1080
   GGTATGTGTG CGTGTGTTCC CAGAAAAATT CTGCTTGTAT ATGTATTCAG TAGTTATCTC   1140
   TAGCAGGACT GTAATTGATT TCTATTCTCT TTATAATTTT TTAAACTTGC TTCATTTTCA   1200
   CAAAGAATAT GTATATAATT ATATATATAT TTGTGATCAA GATAAAAACA GTTGTTACAA   1260
   AAAGCTTACA TGGTGATAAT TTGTATAATG CTTCTGGATT GAACATATAT TGCTCCCTAA   1320
   TAATAGAAAG ACTGAAGTAA ACCTCGTTGG CGGGAAAAAA ATGTAGAATG CCAGGAACAG   1380
   TTTATGTGAG TCTGTAGTAT GGGTTTTACA CCCCTTCATT CTATTTTCTT CCAGGTGTTC   1440
   TTAATGGGAG TTTTACTGTC CTCTAGGGAA ATAGTTAAGG GCAAGTTTGG GATAATCAGT   1500
   GACTGGGGAT GTGTAGGACA GGTGGGGGAC AGTCATAGAT ATCGAATGGG CCCAGGCCAA   1560
   GGTTGCTAAA CTTCCTGCAC TGAAAGGTGT ATCCCCGGCC GGGCGAAGTG GTTCATTCCT   1620
   GTAATCCTAA CACTTTGGGA GCCTGAGGCA AGTGGATCAC TTGAGGCCAG GAGTTCGAGA   1680
   CCAGCCTGGC CAACATGGTG AAACCCCATC TCTACTGAAA ATACAAAAAT TAGCTGGGCG   1740
   TGGTGGCAGG TGCCTGCAGT TCCAGCTACT TTGGAGGCTG AGGCAGGAGA ATCACTTGAA   1800
   CCTGGGAGGT GGAGGTTGCA GTGAGCCAAG ACTGCATCAC TGCATTCCAT CCTGGGTGAA   1860
   AGAGCGAGAC TCTGTCTCAA AAAAAATATA TATATATAAA AATAAAAGGT GTAGCTCCCA   1920
   CAAGAAAAGT TTTTTTTTTT TCATTCAAAC TGGTAATACC ACCACCTTTG AAAAGGAAGT   1980
   ATGGGATCTC TTGGATTAAT TTGGGAAGTG TATAGTTTCT GTTCAGAGTG TTTTATATTT   2040
   ACATGTTAGT GAAATTATAG AGACATTTTA TCCCCTTGTG ACTTGACAAG ACCTTTAAAT   2100
   TATGTTATTT CTCATTACCT TTTTTAG                                       2127
(2) INFORMATION FOR SEQ ID NO:55:
   (i) SEQUENCE CHARACTERISTICS:
      (A) LENGTH: 716 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:55:
   GTAATTGTGC TAAAGGTAAG GTTTAACATT GTTATTCTGC TTCCATGTTT GAAGTTTAAC 60
   TAAATGGAGT CATTTCTTAC TAACTAAGAA AGATGAGGAA AAGATTTATG ACTTTAGACT 120
   GGAGGCATGG ATATGGCTGT CCAATTTTTC TGGTCAACCA ACTGATTTCT GAGCCCTTCT 180
   CAGTAAGATA GAAATTTTAG AATGGTATCT TTATTATATT GGACTACTGA TGCTTCCCTA 240
   TCTGCAAATC TTTAGGTTTC CCTTGTAAAC TGGAAATTAA ATAGAAGTGT AGTGATTCTT 300
   CAACATATTG AGAATAAGGA CAGGAGATAT CACTGTTATG GGCGGAAACC TGGGCTAGGA 360
   ATTGTTTGCT GTCAGGAATT GGAACTAAGT AGGTGTGGAC TAGTAAGCCA ATTACATACC 420
   TCTTAGCATT GGTCTGTTTT GTTCCAACAT AGAGGAAAAA AAAGGGTGTT AGTCTTAAAT 480
   GATATTACAG TTCCTTATGT GCCAATTTCA TTTAATAATT TTAGAAAAAT GTGACTGTTA 540
   CCATGAAGAA AATTAAGGTA TCTTAGGGAT AATTAAAACA CCAATCATAA GAAGTGTGCA 600
   TATCTAAAGT ATTGGGTTGG TTTTGAATTT TATTTTGTGA GTAAAGGAGG AGGAATGGGC 660
   CTTTATTTTC TTTGTGTTCC AATTTTGTGG GGGTTTTTTT TTTATTATTT CTACAG 716
(2) INFORMATION FOR SEQ ID NO:56:
   (i) SEQUENCE CHARACTERISTICS:
      (A) LENGTH: 837 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:56:
   GTGAGTAAAA TAACCAATGT ATTGATCAGC ACAATGAAAC ATAATTTCCT TCCTGCCCTA 60
   TTCTGTGGGT TGTTTCCTTA CTTTATATAT AGTCTCCTTT CATACACAAA AGTTTTTAAT 120
   TTTGATGAAA TCCAATATAT TTTTTCACTA GTTGCCTGTG CTTTCGTTTC ATGTATGTAT 180
   GTATGTATGT ATTTACCTAT TCGAGATGGA GTCTCGCGCT GTCGCCAAGG CTGGAGTGTA 240
   GTGGCACGAT CTCGGCTCAA TGCAACCTCC GCCTCCTGGG TTCAAGCAAT TCTCCTGCCT 300
   CAGCCTCCCA AATAGCTGGG ATTATAGGCA TGTGCCACCA TGCCCAGCTC ATTTCTGTCT 360
   TTTTCGTAGA GATGGCGTTT AGTCATGTTG GGCAGGATGT TCTCGAACTC CAGACCTCAT 420
   GTGGACCACA TTCCTTGTGC TCCCAGAGTG CTAGTATTAC AGCTGTGAGC CACCCATGCC 480
   TTGCCTGTTG CCTGTGCCTT TGGCTCTTCA ATAACTTTTA TTTATAACAT CTTTGCCCTG 540
   TCATTGTTCT TCTAAGCATC AGTGTGTGTG TATTTTGGTT AGAGATGTAA TCTCTTTTAA 600
   GATACATTTT ATATAGGTAA GGTTTTAAAA TTCTCATACA TTCCTTTTAT ATATTTCCTC 660
   TACTAAAAAA TGGGCTTTAT TTATATAATT AAGAAAGGTT TTGTAAGAAA ATAAGGACAC 720
   ACTTTGCACT CACTCAGAAA ATGAGACTTT CTTTGGTATT TTCACTTAAG TTGCACTGGG 780
   TATGAAATGA CTTTTTAGAC TAAGTAGATG TTTCTAATGC TGTACTTTAT TTTATAG 837
(2) INFORMATION FOR SEQ ID NO:57:
   (i) SEQUENCE CHARACTERISTICS:
      (A) LENGTH: 1081 base pairs
      (B) TYPE: nucleic acid
      (C) STRANDEDNESS: double
      (D) TOPOLOGY: linear
   (ii) MOLECULE,TYPE: DNA (genomic)
   (xi) SEQUENCE DESCRIPTION: SEQ ID NO:57:
   GTATTTCCCA AAAAATATGA TACTAATGGG GATATTGTAG ATGAGACCAA CTTCCTGTTG 60
   TTAGTCATTT AGTTCAAGTT AACATCTAAG AACATTTATT CTGTTTCTAT TTACATAGTT 120
   AATCTCTACT TGTGGAGTAG AAAAGAAATA GAATCTTAAG ACCTATGTAA ATTCTTTTAA 180
   TATTGTATGA AAGATCTATT TTGGGTAAAA GCTTCGATTC CTCTCTATCT AATAAAAGTT 240
   TTTAGAATAC TGTGATTTTT ATGAGCTGAG AAGGCTTAAA AAAAGTAGCA CACATGTCAC 300
   TAGCTAATCT TGTATAGCAG CCTTTCCTTA TCTTATGAAA ATTAAATACC ATTGAAAATG 360
   TCAGAAAAAA AATAAAAAGT TGTCTTTCAT GTGTTACAGA GAGGCATAGA GTTAAAAGCA 420
   TTGATTTGGT AGCTAGTTCT TCCCCCTCCG GAGATGGAGT CTTGCTCTGT CGCCCAGCGT 480
   GGAGTGCAGT GGCGCCATCT CAGCTCACAG AAAGCTCCAC CTCCTGGGTT CACGCCATTC 540
   TCCTGCCTCA GCCTGCCGAG TAGCTGGGAC TACAGGCGGC CGCCACCACA CCCGGCTAAT 600
   TTTTTGTATT TTTAGCAGAG ACGGGGTCTA CACCGTGTTA GCCAGGATGG TACTCGATCT 660
   CCTGACCTCG TGATCCTGCC CGCCACGGCC CCCCAGAGTG CTGGGATTAC AGGCTGGTAG 720
   CTATTTCCTT GATACTGACT TAGCATATGA GTTTATGCTT AACTCTCATA AGATAGACGA 780
   AACTAATTTT TATAGTGGCA TAGATTAAAT GTTTAGAGAT TTTTATATGA AATTTTAAGA 840
   GTAATGTTTT TCAACCTCAA TGTACAAAAC ATGTATTTTA TTAAAAAATT TTGAAATACA 900
   TCACAATGTA AACCATTTTA TATAATTCAT AGTTTGAACT ATAATTATTT ACAAAGACAG 960
   TAAAAGGAAG AGCGGCTGTT TCAAAATAAT ACTTCAACTT GTAATTTTGA CTAATTTCTT 1020
   GTCTAAATAT TTAAAAAATA TTTAATAATT ATTCAGTGAA CCAAGACATT TTTTATTTCA 1080
   G 1081
(2) INFORMATION FOR SEQ ID NO:58:
   (i) SEQUENCE CHARACTERISTICS:
      (A) LENGTH: 1455 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:58:
   GTGAAAATCA ACATCTTTTT ATGAGAAAAA TACATCAATA TCTAATCTAT TAATAATCCT     60
   TTTGGGGATG GGAGGGTGGC AGTTAGGTTT AATATGTTAT AATTACACCT TGTTATGAGA    120
   AAAATCTTGG ACTGTAACGT CCCTCTCTAC CCACAAATTG GGAAGGTGCC AAGAGACCAA    180
   AGAATGACTC AGACAAGTCC AGCTCGGCAA GTACATAACG TCTATTAAGA CTTACATATG    240
   GAGGAGGCAG AGGTGGTGGG GAAAAATAAA AGACTTATAT ACAGGGTACT CCTAGGTAGC    300
   AGCAGGACAG CTCTAGAGAT CCTCGCTACC TCCCATCGCT AAGCTGCTTT TAAGCTAATT    360
   TTCTGGCTCT TTGCCTACTA TGTGTGTGCA CGATGGGACT GTTTTCCTTG GTAGTTTCTC    420
   AGATCTTCTC TGGGATGTTG GGGTTCTCAG GGACACCTGT TCCTTGGCTG GGCACCATGG    480
   CCTTGGCTCA CTGCCTAGCC TTCAGGGTTT AGGCAGCAGA CATACACCCT TAAGTAAGGT    540
   AGGTGACCTG TCACATTTCA CCCCATGTCA AAGAGGAAAC GAGTCAGATA ATTTGTGGTT    600
   GCCCTAAGAT TTTGGTGACA GAGTAAAAAT TCAGTGTTCT TTCTTGATTT CCTTACCAAG    660
   TTTCTTTCCC ATAGAGCAGT GGTCCATCCT TTTTGGCACC AAGGACCAGT TTCATGGAAG    720
   ACAATTTTTC CATGGACAGG GTTGGGGGTT GGAGAGATTT TGGGATGATT CATCTGCCTT    780
   ACATTTATTG CACACTTTAT TTCTATTATT ATTACGTGGT AATATATAAT GAAATAATTA    840
   TACAACTCAC CAAAATGTAG AGTCAGTGGG AGCCCTGAGC TTGTTTTCCT GCAACTAGAT    900
   GGTCCCATCT GGGGGCGGTG GGAGACAGTG ACAGATCAGC AGGCATTAGA TTCTCATAAG    960
   GAGCATGCAA CCTAGATCCC TTATGTGTGC AGTTCACAAT AGGGTTCACA CTCCTGTGAG   1020
   AATCTAATGC CACCACTAAT CTGACAGGAG GCCAGCACAG GCGGCAATGT GAGCGATGGG   1080
   GAGCAGCTTT ACATACAGAT GAAGCTTTGC TCGGATGCTC ACTGCCTGCT GCTCACCTCC   1140
   TGCTATGTTG CCCAGTTCCT AACAGGGTCC ATGGCCCAGG GGTTGGGGAC TCCTGCTTTA   1200
   GAGTGGTTGA TATTCAAACT CCTCTCCAAA CCAGTCAATG AAGTTTGACT CATATTTAGT   1260
   ATCCAATTAC AAGGTTTTGA ATTTTTTGAC TGCCAAAAGT TTTTTTTTTA ACTTTATTAT   1320
   TAAAATGGGA AAGACAGCTG ATTTTATTTA GATGGAATAA TTGTTAAGAT ACTTCTTCTG   1380
   CCTTAGATTA CTATTGTATT TGTAATTAAA GTGCTCGTTT GGATACTGGC ATTCTGTGTA   1440
   ACCAATTCTT CATAG                                                    1455
(2) INFORMATION FOR SEQ ID NO:59:
   (i) SEQUENCE CHARACTERISTICS:
      (A) LENGTH: 2741 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:59:
   GTAAGTTTAA CAATACTAGG AGAATATCTT GGGGCTTACT ATCTGGAAAT TTAAATTTCA     60
   TCTAACCCTA CAAGTGAAGT TAATAGGGTA TACATAGAAG AAAATATTCT ATGCATTTTG    120
   GTACCCATGG ATCACTTAAA AGAAGGGCCT TTAAAGACTA AGAACACAGG AAAATGCATG    180
   ATATAACAGG TATCTTTTAA AAAGGATAGA CTGCTTTATT TATTTATTTA TTTATTGAGA    240
   CAGAGTCTTG CTCTGTCACT CAAGCTGGAG TGCAGTGGCC CAATCTCAGC TCACTGCAAC    300
   CTCTGCCTGC CGGGTTCAAG CGATTCTCAT GCCTCAGCCT CCTGAGTAGC TGGGACTACA    360
   GGCATGCGCC ACCACGCCTA GCTAATTTTT GTATTTTTAG TAGAGAAGGG GTTTTGCCAT    420
   ATTGGCCAGG CTGGCCTTGA ACTCCTGACC TCAAGTGATC CGTCTACCTC GTTCTCCCAA    480
   AGTGTTGGAA TTACAGGCAT GGGCACCGTG CCCGGCTGAC TGCTGTATAT TTAATATGAT    540
   CCCTATTTTT AAAGTGTATG TTTATTTATG AGCATACAAA ATAGTGGAAA TGGAAAAACC    600
   AAACTGTTAA GATCATTGTT GGGTGAATGA ATTCCTGGTG ATTTCTGTAA AATTTTTAAG    660
   GCAAATACAT ATTACTTTTA AAATCAGAAA TAGAAAAGCC TTCTTAAAGA TAGAGCTGCA    720
   TGATCCAGTT AGGTATAGAC AAGCCAGTGA GTTAAGACAA CTGAGTATGT TCCACTTTGT    780
   TGAGCTGTGC TACCCTAGTT AATGTGACAT TAGTGCTGGC CCAAGAAATA CAGAAAAGGG    840
   CAGTTTTGCT ATCTATCTGG TTTATATTTT TTAGGCAGCT GCTTAGAAGA TCTGCAAGGT    900
   GAAAGGTTTT AGTTTACATA TGTGAGATAG AACTACTTTT TTAAAGAGCA ATTCAGTAAA    960
   TCCAGAGAGT TCTAAATCCT TGGATCCAAT TAAAAGAATA TTGTTATTTG TAGATCAGTT   1020
   TTATAATGTA ATTGATAAGA ACTGGCTATA GAAGGAATAC CAGTTTTAAA GTCAGGATTC   1080
   ACTCTAGGCT GGGCATGGTG GCTCATGCCT GTAATCCCAG CACTGTGGGA GACCTAGTGG   1140
   GGAGGATCAC TTGAGCCCCG GAGTTCAAGA CCATCCTGGG CAACATAGCA AGATACCATC   1200
   TCTACCCCCA ACCCCCCCAA AAAAATCACT CTAAGTGTAT ACTTAATACA CATGGATGAT   1260
   CCTTATGAAA AGTCCTCATT TTTGAAAGAT CTGAGAGCTG GTCTTTCTTA GTCTATTTTT   1320
   GTAGAATTTT CCGTTCCCTA ATCTACAGAT TAGGAAGACT TGACGTTAAC TTCATTTTCA   1380
   ATGTCTTACC ACTTGCTCAG TTTTCCTGAG ATCTCTTGAT ATTTTATGGA GGAGAAATGA   1440
   TCATAATCTA TTCTTTGCTG ATTCTGCAGC TTTGTACCAA ATACAAACTC AGTAAGTTTA   1500
   TTTACTTTTG TATCATCTGG AAATAGAAAT GTTAAGCCAC AGTTTGTTAG GATTTACTCC   1560
   TATCAGTACT TCTTACAAAC TTTGCTATGT ATATTTTAAA TTTTAAAAAC ACTCTGATGC 1620
   ACAGCTCTTA GAAGTGGACA CAGAAGAAGG AAGAAATGCT TCTCAAAAAT TCAGACATTG 1680
   GTGTGAATAC TTAAAAATAG ACTAAGCCAT AATGGGTTGT GTACCACTGA ATCATACACT 1740
   TAAAAATGGT TGAATGGTAA ATTTTATGTT ATATATATAA CCACAATTTT AAAAAACTAG 1800
   CCTGTAATAC CAGCATTTTG GGAGGCCAAG GCGGGTGGAT CACCTGAGGT CAGGAGTTCG 1860
   AGACCAGCCT GGCCAACATG GTGACCTCAT CTCTACTAGG GAGGCGGAAA GTAGCCATGC 1920
   CGTGTGGCAT ATGCCTCTAA TCCCAGTTAC TTGGGAGGCT GAGGCGCAAG AATCACTTAA 1980
   ACCCAGGAGG CAGAGGTTGT AGTGAACCGA GATCAGGCTA CTGCACTCCA GCCTGGGTGA 2040
   TAGAGTAAGA CTCTGTCAAA AAATAAATAG TAACAATTTG CCCCAAACCA TTGAATTGTA 2100
   TAATTTAAGT AGATGAAATT TATGGTATAT AAACTGTTTT AAAAAAATAA ATTATGCTTA 2160
   ACTGAATCCA AATCATGCAT GTCCACCTTG CTTAAGAACA TTATTGAGTT TTAATAATTT 2220
   TTTATATGTG GAAAAAGACA GAGATCCAAA TTGATAAAAC CGGTGGCGGC GGAATGCTCC 2280
   TAGATGACAT ACTACCAATC AGGTCCCCTT ATCAAGTAGT GGCTCTGTAG TAAAATCACA 2340
   TCTTACATGA GTGGTAGGTA GAAAGTGGAT ATGATAGAAA ATATTATAGA AAAATATAAT 2400
   ATAGAAAAAT AGGGTAATTC CTTAAATTGC CCCTAAATCA TGAAGGTTCT TTAGTAGTGG 2460
   AAGACAGAGT CAGGTCTGAT TTGGGAAAGG GGGCGTGGAG AAAGGAACAC TGCAAGACAC 2520
   AAAATTCCGT TTTAAAATTT TGCTCTCAGT AGTGTTCACT GAACACGAAT GAAAGTTCAC 2580
   TAATGAATAT AGGTAAGATT AGACTTCTGT AATTCTTGTT TGCTTTTTGA ATTATGAAGT 2640
   ATTTCAAACA CTGTAGTTAT TTTTTAACAT AAGAGCTTGG ACGGAAGTCA GATCTGAGTC 2700
   TCCTTGAGTT AAATGCTTTG TTTGATTTGT TTTGACCCTA G 2741
(2) INFORMATION FOR SEQ ID NO:60:
   (i) SEQUENCE CHARACTERISTICS:
      (A) LENGTH: 197 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:60:
   GTAAGGAAGG CAGAGTTGGA TATTGAGTTC CTTCTCTGTG GCATGTATTG AAAAGTTACC 60
   CGAGGTTTGG CTAGAGTGAC ATAGGGGACA GAGGAGTGAT GGGGAGAGAG GGTTTGGGAG 120
   AGCAGAAATT GTAAACCTCT GCCCGGAGAA CCTCTTATTA TCAACATTTT CTTCATGCTT 180
   TTTTTCTCTG TCACTAG 197
(2) INFORMATION FOR SEQ ID NO:61:
   (i) SEQUENCE CHARACTERISTICS:
      (A) LENGTH: 82 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:61:
   GTAATTTTTC ACATACCTTA TCAGAGCATG AGCTTGGGAA ATACAAGTGT TAAACAAAGT    60
   TTGAAATGTT TTTATCTCCT AG                                             82
(2) INFORMATION FOR SEQ ID N0:62:
   (i) SEQUENCE CHARACTERISTICS:
      (A) LENGTH: 1079 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:62:
(2) INFORMATION FOR SEQ ID NO:63:
   (i) SEQUENCE CHARACTERISTICS:
      (A) LENGTH: 659 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:63:
   GTAACTACAT TTTCTCTATG GGCTGCAAAA TAAAGCTTAT AGTCTGTGAT GAATACAAAA 60
   AATTACCCAT AGTTGACTCT GTGGCCTTTT TTCCAAGATA AACACCTGGG ACTCTACTTA 120
   AGGAAGTTTC TACTTTAATC TTTATTCTTG ATGTCACATG TTGATTAAGG TCTCTTTTCC 180
   TCAAAAGGCA ACAATGTTAA ATATTTCATT GCCTTCTTAA TTCAGAAAAA TCACAAGATA 240
   GGAATTAAGA AGTTACTTGG TTTCTATGTC ACCTTTCATT CTGGTTTAGT AAACATACTG 300
   TAGGTTTAAC CAAGAGAATG TCACATGGAA ATTTAAAACC CACTTCGACT TTATTACCAT 360
   TCATCTCTGA GAGGCAAATC GGCCAGATCT GTGTATCTTA CTTAGAATGA CTTGACATTA 420
   TGGTTGGGTG CTGTCACTGC AGTGTAGTAC TGCAGGTAGT ACTTGGCATG TGATGCTAGA 480
   TGGGCTCTGA TTGAATCCTG GATCTGTTAT AATTTGAGTT ATGTTTCTCA ACCTGTTCTG 540
   AGGACAACTA TTGCTATACA GGTTATTGTG AAAACCAAGT AACATATGTG AAGGTCCTAT 600
   CACCAAGGGT GTGCTCAACA AATACTAGTT TATGTCCCCT CCTCATTGTT TCTCTAAAG 659
(2) INFORMATION FOR SEQ ID NO:64:
   (i) SEQUENCE CHARACTERISTICS:
      (A) LENGTH: 572 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:64:
   GTGGGATCTT TGTGAACTAC AAGACAAAAT TAGGAGCTTT TCTTACTTTT TAGGCCTTGA 60
   AGAAGTAACT AAGCATTACT AAATGAAATA ACTATAGAAA CTATGAAAGT GTTTTATAGA 120
   TCAGTAAACC ATATTCTAGC TGGCAAAACT GTCCATTACA TAGCTTTGGG GCACAATATT 180
   ATGTAACATA TTTCTCCAGG AGAATTAGAG CTTTCAGGGA GGAATCTGCT TGCCTGAGTT 240
   CCAGAAAGGT CTGATATGTC AATTGGAACC ATGCTATGGA AATACCATCC CCTGCCTGTC 300
   TGCTTTGTAC CACTTAGTAC AGGGCTTAGG TCCTAGAAAA TTTGGTGTAA CTTATTAATG 360
   GACACTACTC AGAAAGCCCT TGCTATGGTT ATGGCATAGG GAGAAAGTTA ATATCCTAGC 420
   TGAGCTTTGC TTTTTGGTGT GAAGAACAGA GTGCCTATTC ACTGTTATTA GCAAGTAGTG 480
   CAGGTAGCTG TTCCCTTTCT CCTACTTTTA AAAAATTAAA ACAGTCACTA TTAGCAGCCT 540
   TTGTTCGACA GCCTTGGTTC TCCTGGCTGC AG 572
(2) INFORMATION FOR SEQ ID NO:65:
   (i) SEQUENCE CHARACTERISTICS:
      (A) LENGTH: 901 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:65:
   GTAAGTATGA CAGGGATTAT TTCATACTTT TCTCACTCAT GAGTGTTGAG GAATCATTTA 60
   TGATTTATAT ATGGACCATT CACCTGGTCC GTATATAAAC TAGTTTTGGC CAGGTGTGGT 120
   GGCTCACACC TGTAATCCTA GCACTTTGGG AGGCCGAGGA GGGTAGATCA CTTGAGGTCA 180
   GGAGTTCAAG ACCAGCCTGG CCAACGTGGC AAAACCCAGT CTCTACTAAA CATACAAAAA 240
   TGAGCTGGGC GTGGTGGCAC ACACTTGTAA TCCCAGCTAC TCTGGGGGCT GAGGCAGGAG 300
   AATTGTCTGT ACATGGAAGG CGGCGGCTGT AGTGACCTGA CATTGTGCCA CTGCACTCCA 360
   GCTTGGGTGA CAGAACAAGA CTCTGTCTCA TCACTAAGCT AGCTCTACAA ACACTTCTCT 420
   TATGTACAAT GAGGAAGTCT GTAATCTACC TAACCAATAT AAATTCTACT GTTGTCAAGC 480
   ATCAACCGAG TAAGATTGTA TTTGGAGTCC CCGCAAAGTA TAGTAGTACA AGAGGCAGGC 540
   TACATGGGTT CAAATTTCCC AGTACTTAAC AGTGGTGGTA ACCCTGCAAA TCATTAAATT 600
   TTCTCTGTAC CTCATTTCCT CATATATAAA ATGGGAATAT AACTAGTTCC TAGCATATGG 660
   GGTTGTTGTA AGGATGACAT GACATAATGT ATAAAAATTG CTTACAATAA TAACTGGCAC 720
   AAACTAAGCA CTTAAGGTTT GCTATTAGAA TATTTTTCTT TAGGTTAAGT TATTGCTAAA 780
   ACATCACTCT GTCATTCATA AAACTACTGG TTTAGCACAC CTCTTCACTC AATAATCATT 840
   TTCAGTAAAA ATAATTATAA ATTTTTTTTC TTAGAATTAC TGATTTTTTT TTTTTAAACA 900
   G 901
(2) INFORMATION FOR SEQ ID NO:66:
   (i) SEQUENCE CHARACTERISTICS:
      (A) LENGTH: 4220 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:66:
   GTGCGCTCGC GGGCGGAGGG GCGCTTCCGG CCTAGTTGGT GTGAACCGGT GCCTTCCGAG      60
   CCGTGTCGCG CGCCTCGAGA GACTCTCGGG CGGGTTGCGG GCTCCCAGCC CCGAGAGGGG     120
   TGGGGACTCC CTCTGCGCTA TTCCGAGGCT CTTAGCCGCT CCGAGGGTTA ACCCGCTCTC     180
   GCCGGGCTTT CCTGCGGCTT CCGAATGGGG AACGTGTCTT GCCCTAAAGT AGCACAGCAA     240
   GGTTGAGATC GCGTTGGGGC CCCGTTGAGG AAAATGGGTG TGTGTGGTCC ATCTGACCCC     300
   CCGCCCGTCT TGTTAGTAGA ATGAACTAGT GTCGTTGTCA AGACCACACG GACAAGGGGA     360
   GGGGACTTGC CCTTATTTGC ACCGCGATTA ACCGGGTTGT GGCACCTGGG TCTCCACGCG     420
   TCTCCGTCTG TTCGCTTCCC CCTGTTAACC AAATTGCCTT TGCCCTGGCG TTGCGGGCGT     480
   TTGAGTCAAC GTGCTGATGC GTTTTGGGCT GTGTTTACGT CTGTGTAAAC AAATTAATAC     540
   TCATTTCCCC CCAGGCCATA TGAAATGAGC CCACCGCCGA CCCGGATGTT TACACATGCC     600
   CCCATTTGTC ACTACGATCA GGACTGTGGC TACCTCCAGG GCTTTTTGGT CACCCCGCGC     660
   ATTGCACAGG ACTCCTGTTG TCGTCGCCAT CCGGGTGTGT TAGGTCGCAG CCTTCGGCAC     720
   AGGGCTTGCA CCATGACAAA AATGGCCATT CTAGCCAGTG AGTGTCAGCT TTGTATGCAC     780
   CTCCCCTTCA TGGGCCAATG GGAAGTGACA CGGAAGTACG GATTGTTTAT CACCTGTTTG     840
   ACTGTGTGTG TGGCATTTAA ACCTGAGGCC ATTTGATTTC TCAAGTCGTT TTATAATTAA     900
   TTTGTACAAA GAGTCGGGCA AATACGTCCA GGATGCAAAG CCTAACGAAG GTATTATTTA     960
   AATATGATGT TTTTGGCTAT GTGTACTGAT GACTGAGGTT ATTTTTAATT TGTATTTGCA    1020
   TTAATACAAT TTTAATTCAA TTACTAGTTC CCTCTTTGAA TTGTTAGGTC TGCACAACAT    1080
   ACTGTATGGT GGCTTTACAA CCCGACAGAC CTGAAACCGC TGAAAAAGTT CAGTATGGTG    1140
   ATCTCTAAAC TGGAGATATT TGTGTTTACC TCACAGAGCT GTTCTGAAGA TTAAATAAGG    1200
   CAATAATGTA GTTTCTGGCA CATAAAGCAC CCATATGGAC AGTGTTTTCA AGTTTACTAA    1260
   GCTCTTTGTA TATTTACATG ATCTGGCTGA GTAAGCTATG TTCCTATTCA TCTCTCAGTG    1320
   CCTTTCTGTA GTCTGGCAAA GAGAAGGACT GGTTGGCTTT TTATGTTGTT TTTTGTTTTT    1380
   TGGGTTTTTT TTTGGTAAAT GGCCTTAAAG GCTTCCAAAC AAGCTCTTAT TTTACCCTCA    1440
   AGATAATCCT GTAAATCAGA TAGAACAAGC ATTATCGCCA TTTATTTGAG GTATTTCAAC    1500
   TCATAGCAGT TAAGTTGTAT GAAGTCTAGT GATACATGAG CAAGTATCAC GTAATAGCTG 1560
   GTTAGTAAAT TATTTTTGAA ATCATGTTTG ATTACTCAAT TCTTTTGATT ACTGAGACTT 1620
   TAGTTTCAGC TTCTTAGCCC AGTTTATCAG TAAATGATTT ACTCAGTAAA ATATTCATCA 1680
   AATATTTCTT GAGCACCTAT TACTTGCTAC ACATTGTTCT AGGTGCTGGA TATAGAGCAC 1740
   AAACTGCTCT TGTGGGGCTT ACAGTGAGGT ACGCTGTGAC AATATGGGAT GTCATTCTCA 1800
   TGGGAGTGCA AGGGTAAAAT AAAGCTCTTA TGATGTTTAA TACAGAATAC TGGTTATGGA 1860
   ATTTTAACTT GATTTCTTGT ATTTTCTGTG CATTTTTAAC CTGTAACTCA TTCTCACAGT 1920
   CCTCAGCCAA GAAAATGCAG CCTCTGAGAC TGTTAAGTAA TTTCCCCACT GTGTTATAGC 1980
   TACTGTATGG CAGAGCCGGA ATTTGAAACC AGATCTATTT GACCCTAGAA GATGTGACCA 2040
   TGAGATGTTA ATTTTGAGGA TAACTTTTTT AGTATTATGG AATTTTCAAC ATATATTTTT 2100
   TAGGACCAAA GATAAACTAG GCACAGAGTC TACTCTTTGC ATAAATTATT TAAAAGAGCT 2160
   TCGCGCTCCA TTTTGTCATC TAAGCACTGT AAAATTCTCA CAAGACTAAT TCTTCTTTTT 2220
   AGGAACGATA TAGTTGTAAA CTTTCTATTT TTTTTCTTTT TTTTTTCTCC CTCCACCATC 2280
   CAAGTAGTTG TGAATTTTCT AGAGCCAAAA TAGAACACTA TAGATTATCT TTTAAACCCT 2340
   TTATTGAAGC AGAGGATAAT GCTGTGACCG ACTTAACTTT ATGCTTTCTA AGAGATATTG 2400
   ATATAGTAGA GAAATGCAGT AGTTATGCAT CTCCGTTTGC TTTTACATCA TAAATCAAGA 2460
   ATATTATGAA ACCATCTCCC AGAGATATAT GTGATACACA GATCTTGGCT GTTTTTTTTT 2520
   TTTACAAAAG TAACATCTAT GCTATTGATA CATATAAGTG GGTTTGTAAG ACAGTCTATG 2580
   TGTAAATGTG AAAAAAGGAA GAATTTCCAG TTCTTCTCAT TTTCATTTAG ACCAGTAATG 2640
   AATACAGTGA AGCTAAAGGA CATCTTCCAT CCTTCCTCGC TTTTATAGGG AGAGGAAAGT 2700
   TGTATCACTT CTTGAGTAAA AAGAATTGTG ACGATCTTTT ACAAACAATG CCTTAAAAAT 2760
   TATTATTTTT GAATGATATG TGGTAGTGGG ATCCACAATA GTCTCATTTG GTTATACAAA 2820
   TAAATTTTAT GTATTCATGT ATGTGTTTTG ATTAGGTATA AAATTAGTGG CTGAATATCC 2880
   ATTCAAGCTT AATTTTGTAT TTCTATCACT TTTGTAGATT TTGAGCAAGA TTAAAAATAT 2940
   AAACAATAGG CCAGGCGCAG GGGCTCACGC CTGTAATCCC AGCACTTTGG GAGGTCTAGG 3000
   TGGGCGAGTC ACGAGGTCAG GAGATCAAGA CCATCCTGGC TAACACATTG AAACCCAGTC 3060
   TGCTACTAAA AATACAAAAA ATTAGCTGAG CGTGGTGGTG GGCACCTGTA GTCCCAGCTA 3120
   CTCAGGAGGC TGAGGCAGGA GAATGGTGTG AACCTGGGAG GCAGAGCTTG GAGTGAGCCA 3180
   AGATGGAGCC ACTGTACTCC AGCCTGGGTG ACACAGTGAG ACTCCATCTC AAAAAAAATA 3240
   AAAAATAAAT AAAAATAAAC AATAATATTG TTTGCATTAC TATGGCTATA TAGCAAATTG 3300
   CCTTAAAACT TAGGGGCAGA AAGCAATTTG TTTTGGTCAC AGGTTCTGTG AGTAAGGAAT 3360
   TCAGGCTGGG GACAGTGTGG ATGTCATGTT TCTGCGTCAA AATGACTGGT ACCTCACCTG 3420
   GAAGACTTGA GCAACTAGGT ACTGGCACAG CTGGAGCTCG TTGGGCATCT CTGTATGTTT 3480
   GTTCCATGTG GTCTCACCAG CATGGTGATC CAGGGTAGGT AAATTGTTAC ATGCTGGTTC 3540
   AGGACTCCGA AGGCACATGT CCTAAGAGAG AGAACCAAGT GGAATCTATA GTGCGTTGTA 3600
   TAATCTTTTA GAATTACATA GTTTCAGTTG TACCTGTGCA ATTATTGATA GAGACAGTTA 3660
   ATCAGTGTGA GGGAACACAG ACCCTTGCCC AGGTCCAAGG TGAGGGAACC CTCTGTACCT 3720
   GTCAGTGGAA TAATGTTAAT GTCACATTAT AAGAAGAGCC TGACGGGGCT GGGTAGAGTG 3780
   GCTCACACCT GTAATCCCAG CACTTTGGAA GACCAAGGCG GATGGATCAC TTGAGGCCAG 3840
   GAGTTCAAGA CCAGCCTGGG CGACATGACA AAACCCTGTC TCGACCAAGA AAACATAGAA 3900
   TTAGCCAGGT ATGGTGGCGC ACTTCTGTAG TCCCAGCTAC TTGGGAGACT GAGGTAGGAG 3960
   GAGTGCTTGA ACCTGGGAGG TGGAGGTTTC AGTGAGCCAA GATTGCGCCA CTGCACTCCA 4020
   GCCTGGGTGA CAGAGCAAGA TTCCATCTCC GAGAGAAAAA AAAAAAAAAA AAAAAAAGAG 4080
   CGTATGAGAT AGGGTCATCA TTGAAACTAA GTTTCCCACA AAAATATAAA CAACACTTTC 4140
   AATTTAAACA TACTTTTAAA AATATTGAAA TATTTATATG TAGCTTTTTA ACTGAAAATC 4200
   AATTTTCTTT TCTTTTACAG 4220
(2) INFORMATION FOR SEQ ID NO:67:
   (i) SEQUENCE CHARACTERISTICS:
      (A) LENGTH: 3507 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:67:
   GTAACTATGT TAGAGTTTGA CAAGTAGAGT ATGGCTAATG TAAGCTCATA AATCATAGTG 60
   ATAGTAAGAA TTATCTCTGC TCATCATTTC CTGAGCATTT GTACCTGTGG ACTGGCGAAA 120
   TTAGATGCTA AAACTAGCAT CTAATGATTT TCCTCCTCTA TATCACAGTT AATATCCATT 180
   ATATTTTACT TCTTTGGTGA AAATATTTAA ATTTTAATGT TTTAGGCACT TGTATGGCAG 240
   AATTTATTTT TAAAGTTTAG GACATTGTGT AATATTGGGA GAAATGAAGG ATATTGAGAA 300
   ACTTTAGGAG ATACTCCAAG TTGAAAAGGT AAATAAAATA TTATTTGCTA TTATACTTAG 360
   CAAATATGTG CACAGGACTT GTGGTCTTAA TATAAATGGA ACATGTAAGT ATTTCTCAGT 420
   TTCCTGTTTG GAGGATAAAT GACATGATTA TAATCCATTT TAGAAAGGGT CAAATATGTT 480
   TAAAAGAAGA GGCAGAAATT GCTTTATCTG TTGTGTAATT AAATTGATTA CATTTATTTT 540
   TTGTGCCTTT TAGGTGAATT TTCTTACATG GCTTATTAAA GATAAGTGGA AAAATGATGT 600
   TTAGCATTTT GGGGGAAATT ACCACTGTCA AAATTTATGG AGTTAATGGT TAAAAAATCA 660
   CTTACTAAAT AAAAAAATTA ACTGGGTGTG GTTGTGCATA CCTGCAGGCC TAGCTACTTG 720
   GGAGGCTGAG ATGGGAGGAT CACTTGAGCC CTGAATGATG GAGCAGCACT GCACTCCAGC 780
   CTGGGCCACA GAGCAAGACC TTGTCTCCAA AAAAAAAAAA AAAAAAGAAG GTTACTATTA 840
   AAATAATTAG CAGGCTGGGG GCGGTGGCTC ACACTTGTAA TCCCAGTAAT CCCAGCACTT 900
   TGGAGGCCAA GGTGTGTGGA TCACTTGAGG TCAAGAATTG GAGATCAGCC TGGCCAATAT 960
   GGTGAAACCC CGTCTCAACT AAAAATACAA AAATTAGCCG AGTGTGGTGA CATGCGCCTG 1020
   TAATCTTAGC TACTCAGGAA GCTGAGTCAG GAAAATCACT TGAGCCCAGG AGGCACAGGT 1080
   TGCAGTGAGC ACTATTGCAC TCCAGCCTGG GTGACAAGAG CGAGACTCCA TCTCAAAACA 1140
   AATAAATAAA ATAAAATAAT TCACAATGTC ATGTTTTAGC TGACATTGTG AATTTTAGTA 1200
   ATCTTTTTTT AACCTTTAAC TCCATCCTGA GTTACATTGA CCAAAGAAAT CAGTATCTAG 1260
   AATTATATCA GGGAACTACT AACAGGGTTA ATAAAATGAA TAAAGAACAT GACTTCACAA 1320
   AGGTTATAAT TCACATAGCT AATAGATACA GGAAGAGATA TTCACTGTCA CTAATAAAGA 1380
   CTTTCAAAGT AGAAAGATAA CATTTCATTC TGTTTTTTTT GAGATGGAGT CTTGCTGTTT 1440
   CACCCAGGCC AGGGTGCAGG GGCGTGATCT CAGCTCATTG CAGCGTGTGC GTCCCAGGTT 1500
   CAAATGATTC TCCCGCTGTG GCCTCCCAAG TAGCTGGGAT TACAGATGCG CACCACCACA 1560
   CCTGGCTAAT TTTTTGTATT TTTAGTAGAG ACGGGTTTCA CCATGTTGGC CAGGCTGGTT 1620
   TCCAACTCCT GACCTCAGGT GATCCACCCG CCTTGGACTC CCAAAGTGCT GGCATTACAG 1680
   GTGTGAGCCA CCATGCCTGG CCAACATTTT ATTCTTATCA TTGGGAAAAT TTGAAGTCTG 1740
   GTATACCAAG TTTGGTCACT GTACAGGGAA ACAGGAACTC TATTTTTTTT ATTTTTCAGT 1800
   TCTTTTTTTT TTTTTTTTTT TTTTTTTGAG ATGGAGTCTC ACTCTGCTGC CCAGGCTGGA 1860
   GTGCAGTAGC TCAATCTCTA CTCACTGCAA CCTCCACTTC CCAGGTTCAG GTGATTCTCA 1920
   TGCTTCAGCC TCCCGGAGTA GCTGGGATAA AGGCACATAC CACTATACCT GACTAATTTT 1980
   TGTATTTTTT GTGGAGACCA GGTTTCACCG TGTTGACCAG GCTAGTCTCG AACTCCTGAC 2040
   CTCAAGTGAT CTACCTGCCT CGGTCTCCCA AAGTGCTGGG ATTACAGGCA TGAGCCACTG 2100
   CGCTCAGGCA GGAACTCTAT ATTGCTGGTG TACATTGGTG AGAGTCAAAA TTGACACAAC 2160
   TACTTTACTA GCAAATTTGG TGGTATTTAG TAATATTGAA GGTGCACATT CTCTTACTGT 2220
   ACTTCTTGGA GTAGTCCCCA AAGAAACTCC TGCACACATG TATAAGGATG TTTTCATTAC 2280
   AACATGTTTT GTTATCATGG AATATTAGAA ACAACCTAAA TTTCCATTGG TTGGGGAGTG 2340
   AATGCAAAAA GTCATTGTAT GTTCATATGA AAGAATGTTT TTAGCAATTA AAATGAATAT 2400
   ATCTTACATA TCAACATTAA TGTCAGAAAC ATTATTGAGT GTGAAAAAGC AAGTTGCAGA 2460
   ATACCACTGA AGTATGATAG CATTTATATA AAATGTAAAA ACACGTAATA AGATATTGCT 2520
   TATTGTTTAC ACATACATGT GTATGTGTAG TAAGTGTGAA AACATAGGAA GGATTAAGAC 2580
   CAACTTTGGA ATGGTTTTTA TCTTTGGGGT AGAAGGGTAA GGATGGGATT AGGGAGGAGT 2640
   ATAAAATGGT AATTTTGACT GTTTCTTTTT CTTTTTCTTT TTCTTTTTTG AGACAGAGTC 2700
   TCGCATTGTC GCCAGGCTGG AGTGCAGTGG CGTGATCTCG GCTCACTGCA ACCTCCGCCT 2760
   CCCAGGTTTA AGTGATTTTC CTGCCTCAGC CTCCTGAGTA GCTGGGATTA CAGGTGCCCG 2820
   CCACCACGCC CAGCTAATTT TTTGTATTTT TAGTAGAGAT CGGGTTTTAC CATGTTGGCC 2880
   ATGCTGGTTT CAAACTCCTG ACCTTGTGAA TCTCCCACCT CGGCCTCCCA AAGTGCTGGG 2940
   ATTACAGGTG TGAGCTACTG CGCCTAGCCT TGACTGCTTT TATAGTGTTG CTAGTTTAAA 3000
   AAAAAATCTG AAGTGGCAGG AGGAGGTGGC TCACACCTGT AATCACAGTG TTCTAGGAAG 3060
   CCAAAGTAGG AGGATCACTC AAGCCCAGGA GTCTGCGGTG AGCTGTGATC TTGCCACTGA 3120
   ACTCCAACAT GGGTGATAGA ACGAAACCCT ATCTCTTACA AAAACAAAAA CGACAAAATT 3180
   TATTTAATAT ATTAACATTT AAAAAATCTG GCAGTGAACC AACGTGAATG TTGGTTAGGT 3240
   TACTCTTGTT AATTTTGGTT TGTATTTTCA AATATTTCAT AGTTAACAAA TACTTTAGGT 3300
   AACCTAAACA AAATGGATTA GGAGGATCAG AGGAATATAC CAATCTGTAA GAAATTAAGC 3360
   TAGTCAGAGA CATGAGTTGT GATTTTATTT CACTGTCTAA AAGTAATATA ATTTAATGCG 3420
   ATAATATTGA TTTACTTTTG AATACTTACT TTTGTATACT TTAGCCTTAT GTTAATTATG 3480
   AAATATCTTG TTTGTCTTTA ATACCAG 3507
(2) INFORMATION FOR SEQ ID NO:68:
   (i) SEQUENCE CHARACTERISTICS:
      (A) LENGTH: 9837 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:68:
   GTGAGCCTAA CATCAATCTT GGCCTTTACT AACCTCAAAA TGCTTCAGAT GCTAGAAACA 60
   GGGTTTGTGC TAAGCTTAGG CACTCATTAG AGTGATGAGA GCTGCCAGGG AGCAGTGATC 120
   AGTCAGTCCT CATGAAGCAA AACCCAGGGT TGTTTTGTTT TTTGCCTTTT TTGAGGGGGA 180
   GGGGGTGGAA TTTAAGGGTG GGAAACAGGG CAAGGGATTT TGATTCTTTT TATTCCCTCT 240
   CCTATTTGTA CATTTTGGTG TAAACCTGAA ATTGATTTCT TACCAAAGGC CTGTTTCTGG 300
   GACAGGCAGT GTCCTCAGGA GTCTGGCTAA TGGGAGAAGT TGACATTTTT GACATTGCAG 360
   TTCAATAGTC ATATTAGCAC AGATGTATGT GGCAACAGCC ACCTCATTCT AAGAAGGGGA 420
   AGGAAGCTTG AGTCAGGCCT TAATGTTGAA AAGTCAGGGA GCTGTTGAGG TATGGAAGGG 480
   CACTCAGCAG GAAGCAGGTT AAGGGGAAGA AAACAGTGTC CTTGAGGCAG ACAGTGATTC 540
   AAAGCTTAAT TACGGGCATC ATGCTATGTT AGCGAGTGGA ACTGGATTGT GACGGCCCTT 600
   ACATAATGAG ATTTTTATTG ATAAAGGTTG CTTAGAGGCT GGGCGTTGTG GCTCACACCT 660
   GTACTCCCAA CACTTTGGGA GGCCACAGTG GGCAGATCAC CTGAGGTCAG GAGTTCATGA 720
   CCAGGCTAGT CAACACGGTG TAAACCTCAT CTCTATTAAA AATACAAAAA TTAGCTGGGT 780
   GTGGTGGAAT GCACCTGTAA TCCCAGCTAC TCGGGAGGCT AAGGCAGGAA AATAGCTTGA 840
   ACCCAGGAGG TGGAGGTTGC AGTGAGCAGA GCATTGCGCC ATTGCACTCC AGCCTGGGTG 900
   ACAAAAGCGA AACTCACTGT CTCAAAAAAA AAAAAAAACC GGTTGCTTAG AAATACACAT 960
   TTTTTTTTGG CCTGAACTCT TCAAAAAAAG GTCAGTATGG TAAGAGGACG GGGAAGGTTT 1020
   CGTAGAGGAG ACTAGGGAGA CACGACATCC AAATGCAATG CATGATTCTT GACCCTGCAT 1080
   AGGAAATCGT CGTTATAAAG GACATTTTGA GGAAAATTTG AATGTGGGCT TTAGTGTATT 1140
   TTTTTTTTTA AAGTTTCTTT GGTGTTGATG ATGTCTAGCA GATTATGTAG GAGACTGTGC 1200
   TGAAAAGTAT TCAGAGGTAA AGTGTCCCAG TGTCTGCAGC TTACTTTCAA ACGGGTTGGT 1260
   TGCAATATAT TTAGGTAGGG AGAGAGTGAA AGTAACTCTT AGACATTAAT GATTGATAAG 1320
   TGGCTGTTCA GTGTACTATT TTTTTCAACT CTTTGTAGGC TTGCAATCTT TTAAAAAGTT 1380
   GAGGAAAACA GTCCGGGTGC AGTGCCTCAC GCCTGTAATC CCAACATTTT GGCAGGCTGG 1440
   GATGGGAAAA TTGCTTGAGG CCAGAATTTG GAAAACGGCT CAGGCAACAT AAAACCCCAT 1500
   CCCTACAACA AATAAAAATT AGCTGAGCAT GGTGCCATGC ACCTGTAGTT GTATCTACTC 1560
   AGGAGGCTGA GCCCAAAATT TCAAGGCTGC GGTGAGCTAT GGTCGTGCCA CCACACTCCA 1620
   GCCTGGGCAA TAAATTGAGA AACCCTGTCT GTTTGGAAAA AAAAGTTGAG GAAAACAATT 1680
   AAACAATAAC AGCAAAAATC TGTTATAAAA TGTAATAATG GGCCAGGTGT GGTGGCTCAT 1740
   GCCTGTAATC CCACCACTTT GGGAGGCCGA AATGGGTGGA TCACCTGAGG TCAGGAGTTC 1800
   AAAATCAGCT TGGCCAACAT GGTGAAACCC CATCTCTGCT AAAATTACAA AAAAATTAGC 1860
   TGGGTGCGGT GGCGCACACC TGTAATCCCA GATACTCAGG AGGCTGAGGC AGGAGAATCG 1920
   CTTGAACCCA GGAGGCGGAG GTTGCAGTGA GCCGAGATCG TGCCACTACA CTCCAGCCTG 1980
   GGCAACAGAG CCAGACTCTG TCTCAAAAAA AAAAAAAAGT TTAATTCACG CAGAGCCAGC 2040
   TGAACGGCAG ACAGGAGTTT GGTTATTCAA ATCAGCCTAC CAGAAAATTC GGAGACTGGG 2100
   GTTTTTAAAG AATGACTTGG CGGGTAGGGG GCCAGGGATT GGCGAATGCT AATTTGTCAG 2160
   GTGGGAGGTG AAATCACAGG GGGTTGAAGT GGGCTCTTGC TGTCTTCTGT TACTGAGTGG 2220
   AATTGCAGAA CTTGTTGAGC CAGATTATGG TCTGAGTGGC GCCAGCTAGT GCATTGGAAT 2280
   GCGCGGTCTG AAAAGTATCT CCAGCACCAA TCTTAGGTTT TACAATAGTG ATGTTATCCC    2340
   TGAGAGCAAT TGGGGAGGTC AGGAATCTTA TAGCCTCTGG CTGCAAGCCT CCTAAATCAT     2400
   AATTTCTAAT CTTGTGGCTA ATTTGTTAGT TCTACAAAGG CAGACTGATC CCCAGGCAAG     2460
   AATGGGGTTT GTTTTTGGAA AGGACTGTTA CAATCTTTGT TTCAAAGTGA AATTAGAAAT     2520
   TAAATTCCTC CTGTAGTTAG TTAGGTCTTC GCCCAGGAAT GAACAAGGGC AGCTCGGAAG     2580
   TGAGAAGCGT GGAGTCATTT AGGTCAGATC CCTTGCACTG TCATAACTTT CTCACTGTTA     2640
   GGATTTTTGC AAAGGCAGTT TCGTGAACGT ACAGAGACAG GCCCTTGCTA TTATCCCTAT     2700
   TTTTTAGATA AGGATATCCA GGCGATGAGG AAGTTTTACT TCTGGGAACA GCCTGGATAC     2760
   GAAACCTTCA CACGTCAGTG TCTTTTGGGA CATTTTCTCG TCAGTACAGC CCTGTTGAAT     2820
   GTTCTCACGG TGGGGAGGTA CGTGTTTAAA ATGCGGGGAA GGTGCTTTTA TTTCACCCCT     2880
   GGTGAAACTA GGGGAGCTAA TTTTTTTAAA CATGATTTTT GGCCCCCTTG AACCGCCGGC     2940
   CTGGACTACG TTTCCCAGCA GCCCGTGCTC AAGACTACGG GTGCCTGCAG GCGGTCAGAG     3000
   TCGTTTGCGG CGGCGCAGGC GCGGTGCGGG CGGCGGACGG GCGGGCGCTT CGCCGTTTGA     3060
   ATGGCTGCGG GCCCGGGCCC TCACCTCACC TGAGGTCGGC CGCCCAGGGG TGCGCTATGC     3120
   CGTCGGGAGG TGACCAGTCG CCACCGCCCC CGCCTCCCCC TCCGGCGGCG GCAGCCTCGG     3180
   ATGAGGAGGA GGAGGACGAC GGCGAGGCGG AAGACGCCGC GCCGCCTGCC GAGTCGCCCA     3240
   CCCCTCAAAG CCGAATTCTG CAGATATCCA TCACACTGGC GGCCGCTCGA GCATGCATCT     3300
   AGAGGGCCCA ATTCGCCCTA TAGTGAGTCG TATTACAATT CACTGGCCGT CGTTTTACAA     3360
   CGTCGTGACT GGGAAAAACC CTGGCGTTAC CCAACTTAAT CGCCTTGCAG CACATCCCCC     3420
   TTTCGCCAGC TGGCGTAATA GCGAAGAGGC CCGCACCGAT CGCCCTTCCC AACAGTTGCG     3480
   CAGCCTGAAT GGCGAATGGA CGCGCCCTGT AGCGGCGCAT TAAGCGCGGC GGGTGTGGTG     3540
   TTACGCGAGC GTGACCGCTA CACTTGCCAG CGCCCTAGCG CCCGCTCCTT TCGCTTTCTT     3600
   CCCTTCCTTT CTCGCCACGT TCGCCGGCTT TCCCCGTCAA GCTCTAAATC GGGGGCTCCC     3660
   TTTAGGGTTC CGATTTAGTG CTTTACGGCA CCTCGACCCC AAAAAACTTG ATTAGGGTGA     3720
   TGGTTCACGT ATTGGGCCAT CGCCCTGATA GACGGTTTTT CGCCCTTTGA CGTTGGGAGT     3780
   CCACGTTCTT TAATAGTGGA CTCTTGTTCC AAACTGGAAC AACACTCAAC CCTATCTCGG     3840
   TCTATTCTTT TGATTTATAA GGGATTTTGC CGATTTCGGC CTATTGGTTA AAAAATGAGC     3900
   TGATTTAACA AAAATTTAAC GCGAATTTTA ACAAAATTCA GGGCGCAAGG GCTGCTAAAG     3960
   GAAGCGGAAC ACGTAGAAAG CCAGTCCGCA GAAACGGTGC TGACCCCGGA TGAATGTCAG     4020
   CTACTGGGCT ATCTGGACAA GGGAAAACGC AAGCGCAAAG AGAAAGCAGG TAGCTTGCAG     4080
   TGGGCTTACA TGGCGATAGC TAGACTGGGC GGTTTTATGG ACAGCAAGCG AACCGGAATT     4140
   GCCAGCTGGG GCGCCCTCTG GTAAGGTTGG GAAGCCCTGC AAAGTAAACT GGATGGCTTT     4200
   CTTGCCGCCA AGGATCTGAT GGCGCAGGGG ATCAAGATCT GATCAAGAGA CAGGATGAGG     4260
   ATCGTTTCGC ATGATTGAAC AAGATGGATT GCACGCAGGT TCTCCGGCCG CTTGGGTGGA 4320
   GAGGCTATTC GGCTATGACT GGGCACAACA GACAATCGGC TGCTCTGATG CCGCCGTGTT 4380
   CCGGCTGTCA GCGCAGGGGC GCCCGGTTCT TTTTGTCAAG ACCGACCTGT CCGGTGCCCT 4440
   GAATGAACTG CAGGACGAGG CAGCGCGGCT ATCGTGGCTG GCCACGACGG GCGTTCCTTG 4500
   CGCAGCTGTG CTCGACGTTG TCACTGAAGC GGGAAGGGAC TGGCTGCTAT TGGGCGAAGT 4560
   GCCGGGGCAG GATCTCCTGT CATCCCACCT TGCTCCTGCC GAGAAAGTAT CCATCATGGC 4620
   TGATGCAATG CGGCGGCTGC ATACGCTTGA TCCGGCTACC TGCCCATTCG ACCACCAAGC 4680
   GAAACATCGC ATCGAGCGAG CACGTACTCG GATGGAAGCC GGTCTTGTCG ATCAGGATGA 4740
   TCTGGACGAA GAGCATCAGG GGCTCGCGCC AGCCGAAACT GTTCGCCAGG CTCAAGGCGC 4800
   GCATGCCCGA CGGCGAAGGA TCTCGTCGTG ACCCATGGCG AATGCCTGCT TGCCGAATAT 4860
   CATGGGTGGA AAAATGGCCG CTTTTCTGGG ATTCATCGAA CTGGTGGCCG GGCTGGGTGT 4920
   GGCGGACGCT ATCAGGACAT AGCGTTGGCT ACCCGTGATA TTGCTGAAGA GCTTGGCGGC 4980
   GAATGGGCTG ACCGCTTCCT CGTGCTTTAC GGTATCGCCG CTCCCGATTC GCAGCGCATC 5040
   GCCTTCTATC GCCTTCTTGA CGAGTTCTTC TGAATTGAAA AAGGAAGAGT ATGAGTATTC 5100
   AACATTTCCG TGTCGCCCTT ATTCCCTTTT TTGCGGCATT TTGCCTTCCT GTTTTTGCTC 5160
   ACCCAGAAAC GCTGGTGAAA GTAAAAGATG CTGAAGATCA GTTGGGTGCA CGAGTGGGTT 5220
   ACATCGAACT GGATCTCAAC AGCGGTAAGA TCCTTGAGAG TTTTCGCCCC GAAGAACGTT 5280
   TTCCAATGAT GAGCACTTTT AAAGTTCTGC TATGTGGCGC GGTATTATCC CGTATTGACG 5340
   CCGGGCAAGA GCAACTCGGT CGCCGCATAC ACTATTCTCA GAATGACTTG GTTGAGTACT 5400
   CACCAGTCAC AGAAAAAGCA TCTTACGGAT GGCATGACAG TAAGAAGAAT TATGCAGTGC 5460
   TGCCATAACC ATGAGTGATA ACACTGCGGC CAACTTACTT CTGACAACGA TCGGAGGACC 5520
   GAAGGAGCTA ACCGCTTTTT TGCACAACAT GGGGGATCAT GTAACTCGCC TTGATCGTTG 5580
   GGAACCGGAG CTGAATGAAG CCATACCAAA CGACGAGCGT GACACCACGA TGCCTGTAGC 5640
   AATGGCAACA ACGTTGCGCA AACTATTAAC TGGCGAACTA CTTACTCTAG CTTCCCGGCA 5700
   ACAATTAATA GACTGGATGG AGGCGGATAA AGTTGCAGGA CCACTTCTGC GCTCGGCCCT 5760
   TCCGGCTGGC TGGTTTATTG CTGATAAATC TGGAGCCGGT GAGCGTGGGT CTCGCGGTAT 5820
   CATTGCAGCA CTGGGGCCAG ATGGTAAGCC CTCCCGTATC GTAGTTATCT ACACCGACGG 5880
   GGAGTCAGGC AACTATGGAT GAACGAAATA GACAGATCGC TGAGATAGGT GCCTCACTGA 5940
   TTAAGCATTG GTAACTGTCA GACCAAGTTT ACTCATATAT ACTTTAGATT GATTTAAAAC 6000
   TTCATTTTTA ATTTAAAAGG ATCTAGGTGA AGATCCTTTT TGATAATCTC ATGACCAAAA 6060
   TCCCTTAACG TGAGTATTCG TTCCACTGCA GCGTCAGACC CCGTAGAAAA GATCAAAGGA 6120
   TCTTCTTGAG ATCCTTTTTT TCTGCGCGTA ATCTGCTGCT TGCAAACAAA AAAACCACCG 6180
   CTACCAGCGG TGGTTTGTTT GCCGGATCAA GAGCTACCAA CTCTTTTTCC GAAGGTAACT 6240
   GGCTTCAGCA GAGCGCAGAT ACCAAATACT GTTCTTCTAG TGTAGCCGTA CGTAGGCCAC 6300
   CACTTCAAGA ACCTCTGTAC CACCGCCTAC ATACCTCGCT CTGCTAATCC TGTTACCAGT 6360
   GGCTGCCGCC AGTGGCGATA AGTCGTGTCT TACCGGGTTG GACTCAAGAC GATAGTTACC 6420
   GGATAAGGCG CAGCGGTCGG GCTGAACGGG GGGTTCGTGC ACACAGCCCA GCTTGGAGCG 6480
   AACGACCTAC ACCGAACTGA GATACCTACA GCGTGAGCTA TGAGAAAGCG CCACGCTTCC 6540
   CGAAGGGAGA AAGGCGGACA GGTATCCGGT AAGCGGCAGG GTCGGAACAG GAGAGCGCAC 6600
   GAGGGAGCTT CCAGGGGGAA ACGCCTGGTA TCTTTATAGT CCTGTCGGGT TTCGCCACCT 6660
   CTGACTTGAG CGTCGATTTT TGTGATGCTC GTCAGGGGGG CGGAGCCTAT GGAAAAACGC 6720
   CAGCAACGCG GCCTTTTTAC GGTTCCTGGC CTTTTGCTGG CCTTTTGCTC ACATGTTCTT 6780
   TCCTGCGTTA TCCCCTGATT CTGTGGATAA CCGTATTACC GCCTTTGAGT GAGCTGATAC 6840
   CGCTCGCCGC AGCCGAACGA CCGAGCGCAG CGAGTCAGTG AGCGAGGAAG CGGAAGAGCG 6900
   CCCAATACGC AAACCGCCTC TCCCCGCGCG TTGGCCGATT CATTAATGCA GCTGGCACGA 6960
   CAGGTTTCCC GACTGGAAAG CGGGCAGTGA GCGCAACGCA ATTAATGTGA GTTAGCTCAC 7020
   TCATTAGGCA CCCAGGCTTT ACACTTTATG CTTCCGGCTC GTATGTTGTG TGGAATTGTG 7080
   AGCGGATAAC AATTTCACAC AGGAAACAGC TATGACCATG ATTACGCCAA GCTATTTAGG 7140
   TGACACTATA GAATACTCAA GCTATGCATC AAGCTTGGTA CCGAGCTCGG ATCCACTAGT 7200
   AACGGCCGCC AGTGTGCTGG AATTCGGCTT AAAGGTAGGC GGATCTGGGT CGACTCTAGG 7260
   CCTAAATGGC CATTTAGGTG ACACTATAGA AGAGCTCGAG GACAACAGAA AATCTTAGTG 7320
   AACATGTTTT ATGGGAAAAT TTTATATACA ACATCAAAAG CACAATCCGT AAAATACTGT 7380
   TAAAATGGAT TTTATCAAAA TGAATAATTT CTGCTATTTG AGACACTGTT AAGAGAATTA 7440
   AAAAACCAGC CATAGACTAT TAGAAAATCT GTACACGTTC CATATCTGAT GAAGCATTTG 7500
   TATATCTACA GTATCTAAAG AATTCTCAAA ATTCAGTAGG AAAACCACCA AATGTAAAAG 7560
   TGGGCAAAAG ATTTGAACAC ACTTCACCCA TTACATGCCT GTTAGAATGG CTAAAATCCA 7620
   AAAAGTGACA AATCGTAAGT TCTGACAACA ATGTGGAACA ATTTTACATA TTGCTGGTGT 7680
   GAACGCAAAA TGGCATCGCC ACTGTGGAAA GTTGTTTCTT AAACATACCA TTATACAACC 7740
   AGCAATCTCA TTCCTAGGTA TTTACACAAA TGAAATGGAA ACTTATGTTT AGACAAAATC 7800
   ACGTACATGA CTGTTTATAG TGACTTTCTT CCTAATTGCC AAAAAGTGGG AAACAACCCA 7860
   AACGTCCTTC AGCTGGTGAA TGCATATAAA TAAGCTGTGG TGCATCCAGA CAATCGACTG 7920
   CTACTTTGCA ATAAAAAGGA ACTGATATAT TCAATGTAGA TAAATCTCAA ATGCATCAAT 7980
   GCTTAAGTGA AAGACACTGG ATTCAGTAGG CTACTTATGA TTCCATTTCT GTGACATTGT 8040
   GGAAAAGGCA AAACTATTGG ACAAGAACAT CAGTGGTGGT TTGGGATAGG CTGACAAGGG 8100
   AGTATGAGGG ATTTTTTCAG AGGAACAGTT TTATCCGACT GTAGGTATTT CTAGCACAGA 8160
   ATTGGGAGTC TGTCCAGTAA AATGATAGCG ATTATTAGAC TCTTGGTTGG AGAAAGATTT 8220
   GTCATCTTGA CGTAATAGGT GATAGCTGAA ACTTACGGGG AGAATATTAC AAAGCAAGGA 8280
   GGGGGAGAAT ATTCCCAAGC AAGAAGTAGC TTATGTCTAG AACCAATCTA TAACGTACTA 8340
   ACATTTAGAC TACTATGAGG GGATAATTAT CAAATACTAT ACAAGATCAG TTAAGATGAA 8400
   GACTGATCAT TAGTGATACT TGACAGAGCA GTGTCAGTGC ACTGGTATGA CTTGTTGAGA 8460
   AATAAATTAT GGTAGCATTG CTTATACACA ATTAACGATG TATACAGTAA GACAGTGTGA 8520
   GAAATATTCA AGCAAATGGG AGACCGCAGA GATACCAAAT GCAGACCAGA CTCTTAGGAG 8580
   GCAAGAAGGG GGCTAGAAAA AGAATTGAAG GAAAGCTTTC TTCAGATGCT TAAGATTTTG 8640
   TGGCCAGGTG CAGTGGCTCA TGCCTGTTCC CAGCACATTA GGAGGCCCAA AGCAGGAGGA 8700
   TTGCTTGAGC CCAGGAATTC AAGACCAGCT TGGACAACAT AGTGCAACCC CATTTCTATT 8760
   GGTAATTAAA AAAAAAAAAA AATGAAAAAC ACTTGTGAAG GTACATCTGT TGATAATAAA 8820
   GAACACTGAT TTTCATTAAA ACCCCCAAAA CATTTATTAC TTTAAAGAAT AAAAATAACA 8880
   AGTGTCATGA TAAAATATGT CTGGGATTTG TTTTAAAATA ATCTGGGGAA TGGAAGTGAA 8940
   TCAGAGTATA AATCAAGCAA GGCTGGCCAA ACATGCTGAA GTAGAGGAAT AGGTATGTGA 9000
   GGATGCATTA TGCTTCTCTA CTTTTGTATG TTTACAATTT CCCTATAATA GATATCTGTG 9060
   AATTTGCTTA GTATGCTTTC TGTAAGCAAA CATGGATGAA GCAGCACATG AAAAAGAATT 9120
   TTAACCAACA AACTAGCAGA AATAATGTGA CAGACGACTT TTAGAGGCTT TGGAGAAACT 9180
   GAATGCTAAA GGTGCTGTAC AGCCAGCCCC AGTCTTTCTG ACATTCTGGC AGTGTCTTTC 9240
   TCAATTGCAG CTCCTCATCT GAGCCACTGT CCAGAAAATA ATTTGAGTAA CTTTAATCCT 9300
   CAATTCTCCC AAGGATAGTA CCATTCTAGA TCTTACTAAT TTATTAGCTA CAATGGATAC 9360
   CTTAGGGGGG GATTAAGGCC TACTTTTCTA GTGAAATCCC AGTTGAGAAT GGCTGCTAAA 9420
   AACTGAGTAA CATTAGACTG AAAGAAAGGG AATATTGTAT AAAGTTGTAC TTTGAAAAAG 9480
   AGAAAAAGAT GTGTCTAAGT GACTATCAGA TAGCAATGTA ATGCTCCCTA ATTGTAAAAA 9540
   AAATCACAAA TTTGTGAACT CACGAATTAT AGACATGTAT AATTGACCTA CAGGTCAAGA 9600
   AGTGCCTGTG GAAGAGCTTG TTAAAAATAG AACTACTCAG CCCCTTCTCA AATAGCCATC 9660
   GGCCTCAGCC ATCTGGAAAG TAAAGTTGGC AGGTTATGTA ACTTAGTGTT TCTTTTACTC 9720
   TGTAGATGTG TTCAAACTCT TCCAGGTAAA CTGCTTAACT CATTTGAGAT TCTTTGACTA 9780
   ATACTGAGCT ATGTGCATTT GCATTTTGAA AAATTATGTA TCTTTTTCCC ACCATAG 9837
(2) INFORMATION FOR SEQ ID NO:69:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:69:
      CTCTGTAACT GCTTATAATC CTG 23
(2) INFORMATION FOR SEQ ID NO:70:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:70:
      CTAGGAAACC TGTACAACTC C 21
(2) INFORMATION FOR SEQ ID NO:71:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:71:
      GGCTTATTGT GTGCTGATAT C 21
(2) INFORMATION FOR SEQ ID NO:72:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:72:
      AGAGATCCTT AAGTCGTCAT G 21
(2) INFORMATION FOR SEQ ID NO:73:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:73:
      CAGTTTCTGT GAGAGAGTAC A 21
(2) INFORMATION FOR SEQ ID NO:74:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:74:
      GGCTTACCTG CTCCTGTATT T 21
(2) INFORMATION FOR SEQ ID NO:75:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:75:
      GAGGAGGAAT GGGCCTTTAT T 21
(2) INFORMATION FOR SEQ ID NO:76:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:76:
      AACCCACAGA ATAGGGCAGG A 21
(2) INFORMATION FOR SEQ ID NO:77:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:77:
      GGATACTGGC ATTCTGTGTA AC 22
(2) INFORMATION FOR SEQ ID NO:78:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:78:
      ATTTCCAGAT AGTAAGCCCC A 21
(2) INFORMATION FOR SEQ ID NO:79:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:79:
      AGCTTGGACG GAAGTCAGAT C 21
(2) INFORMATION FOR SEQ ID NO:80:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:80:
      TCTAGCCAAA CCTCGGGTAA C 21
(2) INFORMATION FOR SEQ ID NO:81:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:81:
      AATTGTAAAC CTCTGCCC 18
(2) INFORMATION FOR SEQ ID NO:82:
   (i) SEQUENCE CHARACTERISTICS:
      (A) LENGTH: 18 base pairs
      (B) TYPE: nucleic acid
      (C) STRANDEDP1ESS: single
      (D) TOPOLOGY: linear
   (ii) MOLECULE TYPE: other nucleic acid
   (xi) SEQUENCE DESCRIPTION: SEQ ID NO:82:
      ATTTCCCAAG CTCATGCT 18
(2) INFORMATION FOR SEQ ID NO:83:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:83:
      AGCATGAGCT TGGGAAAT 18
(2) INFORMATION FOR SEQ ID NO:84:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:84:
      TGAAGACCTA TCTTTGCC 18
(2) INFORMATION FOR SEQ ID NO:85:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:85:
      GTTCACAGAG CTCCTCACAC T 21
(2) INFORMATION FOR SEQ ID NO:86:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:86:
      AGGCCACAGA GTCAACTATG G 21
(2) INFORMATION FOR SEQ ID NO:87:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:87:
      AGGTCCTATC ACCAAGGGTG T 21
(2) INFORMATION FOR SEQ ID NO:88:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:88:
      GCTTAGTTAC TTCTTCAAGG C 21
(2) INFORMATION FOR SEQ ID NO:89:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:89:
      GTAGCTGTTC CCTTTCTCCT A 21
(2) INFORMATION FOR SEQ ID NO:90:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:90:
      CCTCAACACT CATGAGAGTG A 21
(2) INFORMATION FOR SEQ ID NO:91:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:91:
      TGGTTTAGCA CACCTCTTCA C 21
(2) INFORMATION FOR SEQ ID NO:92:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:92:
      GCTTAGCACA AACCCTGTTT C 21
(2) INFORMATION FOR SEQ ID NO:93:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:93:
      TTCGCCGTTT GAATTGCTGC 20
(2) INFORMATION FOR SEQ ID NO:94:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:94:
      ACCGGTTCAC ACCAACTAGG 20
(2) INFORMATION FOR SEQ ID NO:95:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:95:
      GAGATAGGGT CATCATTGAA AC 22
(2) INFORMATION FOR SEQ ID NO:96:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:96:
      CATTAGCCAT ACTCTACTTG T 21
(2) INFORMATION FOR SEQ ID NO:97:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:97:
      GCTAATTTAA CTCTGTAACT GC 22
(2) INFORMATION FOR SEQ ID NO:98:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:98:
      CACTGCAGCA CAGACTAATG TGT 23
(2) INFORMATION FOR SEQ ID NO:99:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:99:
      TCTCTCCCTT TAACTGTGGG TTT 23
(2) INFORMATION FOR SEQ ID NO:100:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:100:
      GGAGTTGACG AGATTAATAC CTG 23
(2) INFORMATION FOR SEQ ID NO:101:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:101:
      CATGACGACT TAAGGATCTC TT 22
(2) INFORMATION FOR SEQ ID NO:102:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:102:
      CTCAGTTTCC AGAGTACAAA C 21
(2) INFORMATION FOR SEQ ID NO:103:
   (i) SEQUENCE CHARACTERISTICS:
      (A) LENGTH: 22 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:103:
      GTGAATTAAA GTCTTTCTGG CC 22
(2) INFORMATION FOR SEQ ID NO:104:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:104:
      ATCTTAGAAA GCAGACAGGG C 21
(2) INFORMATION FOR SEQ ID NO:105:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:105:
      GAGACATTTT ATCCCCTTGT G 21
(2) INFORMATION FOR SEQ ID NO:106:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:106:
      TCCATGCCTC CAGTCTAAAG T 21
(2) INFORMATION FOR SEQ ID NO:107:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:107:
      CACTTAAGTT GCACTGGGTA 20
(2) INFORMATION FOR SEQ ID NO:108:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:108:
      CAACAGGAAG TTGGTCTCAT C 21
(2) INFORMATION FOR SEQ ID NO:109:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:109:
      TAAAAGGAAG AGCGGCTGTT T 21
(2) INFORMATION FOR SEQ ID NO:110:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:110:
      TTAAACCTAA CTGCCACCCT C 21
(2) INFORMATION FOR SEQ ID NO:111:
   (i) SEQUENCE CHARACTERISTICS:
      (A) LENGTH: 20 base pairs
      (B) TYPE: nucleic acid
      (C) STRANDEDNESS:: single
      (D) TOPOLOGY: linear
   (ii) MOLECULE TYPE: other nucleic acid
   (xi) SEQUENCE DESCRIPTION: SEQ ID NO:111:
      CTGAGCTATG TGCATTTGCA 20
(2) INFORMATION FOR SEQ ID NO:112:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:112:
      AAGGCTGCTG CTAAACAGAT 20
(2) INFORMATION FOR SEQ ID NO:113:
   (i) SEQUENCE CHARACTERISTICS:
      (A) LENGTH: 2461 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO:113:
   TGCCCGCCTT GGCCTCCCAA CGTGTAGGGA TTACAGGCGT GAGTCACCGC GCCTTGCCAA 60
   ATTATTTATT ATTATTTTTT GGAGACAGGG TCTCTGTTGC CCAAGCTGTA GTGGTATGGC 120
   CACAGTTCAC TGCAGACTCC CCAGGATTAG GCGTTCCTCC CACCTCAGTC TCCCAAGTAG 180
   CTAGGATTAC AGGCGTCTAC CACCACTCTG GGTTAATTTT TCTATTTTTT GGAGAGACAG 240
   GGTTTCACTA TGTCGCCCAG GCTGGACCTC GAACTCCTGT CTCAAGCAGC CCCCCCACCT 300
   CGCCTCCCAA AGTGCTGGAT TTACAGGTGT GATCCACAAC GTCCAGCCTA TATACTTAAG 360
   ATACTTCTAA ACCATTTGTG TTCAACTTCT GTTCTTGCCC CATAGTCACC TTGAGACTCA 420
   TCACTTAGCC AACTCCAAAA GCATTGCTGA TTACTGTGAA TTTTACTAAG GTTTTCTTAA 480
   GAGGGTTCCA TTGTCTCAAA ATTGTTCCTG AAATATCCTG TTACCTGTCT ACCTGATTTT 540
   CTCCTATCTT CAGAGTTCCA TTTCCTGTCC TCCCGCCTGT CATTATACCT TCCATAAGCC 600
   CCTACTTTTG TCCCAGCACT TTTCCCTCTG TCAGTTTACA TATCCCACCA AGCAAAACAA 660
   AAATAGCAAA ACAGTAATGC CTTCTGAATC CTCAAATTGC TCAATCCTCA GATTGCTCCT 720
   CAATCTGGAA AATGTTTTAT ATCAAGCCCA TTTATAAATC AAGGATTGGC AATTTAAAAA 780
   ATTAAAATAA AGAAAGGAGA ATTGGAAATA AAATGAATTG GCTGGGCACG GTGGCTCACG 840
   CCTGTAATCC CAGAACTTTG GGAGGCCGAG GTGGGTGGAT CACTTGAGGT CAGGAGTGCG 900
   AGACCAGCCT GGCCAACATG GTGAAACCCT GCCTGTTCTG AAAATCCAAA AATCAGCTGG 960
   GTGCGGCGGC GCACACCTGT AATCCCAGAT ACTCAGGAGG CTGAGGCAGG AGAATCGCTT 1020
   GATCCCAGGA GGCGGAGGTT GCAGCGAGCC GAGATCGTGC CACTACACTC CAGTCTGGCC 1080
   AACAGAGCCA GACTCTGTCT CACAAAAAAA AAAAAGTTTA ATTCACGGAG AGCCAGCTGA 1140
   ACGGCAGACA GGAGTTTGGT TATCCAAATC AGCCTACCAG AAATTGGAGA CTGGGGTTTT 1200
   TAAAAGAATG ACTTGGCGGG TAGGGGCCCA GGGATTGGCG AATGCTAATT TGTCAGGTGG 1260
   GAGGTGAAAT CACAGGGGGT TGAAGTGGGC TCTTGCTGTC TTCTGTTACT GAGTGGAATT 1320
   GCAGAACTTG TTGAGCCAGA TTATGGTCTG AGTGGCGCCA GCTAGTGCAT CGGAATGCGC 1380
   GGTCTGAAAA GTATCTCCAG CACCAATCTT AGGTTTTACA ATAGTGATGT TATCCCTGAG 1440
   AGCAATTGGG GAGGTCAGGA ATCTTATAGC CTCTGGCTGC AAGCCTCCTA AATCATAATT 1500
   TCTAATCTTG TGGCTAATTT GTTAGTTCTA CAAAGGCAGA CTGATCCCCA GGCAAGAATG 1560
   GGGTTTGTTT TTGGAAAGGA CTGTTACAAT CTTTGTTTCA AAGTGAAATT AGAAATTAAA 1620
   TTCCTCCTGT AGTTAGTTAG GTCTTCGCCC AGGAATGAAC AAGGGCAGCT CGGAAGTGAG 1680
   AAGCGTGGAG TCATTTAGGT CAGATTCCTT GCACTGTCAT AACTTTCTCA CTGTTAGGAT 1740
   TTTTGCAAAG GCAGTTTCGT GAACGTACAG AGACAGGCCC TTGCTATTAT CCCTATTTTT 1800
   TAGATAAGGA TATCCAGCCG ATGAGGAAGT TTTACTTCTG GAACAGCCTG GATACGAAAC 1860
   CTTCACACGT CAGTGTCTTT TGGACATTTT CTCGTCAGTA CAGCCCTGTT GAATGTTCTC 1920
   ACGGTGGGGA GGTACGTGTT TAAAATACGG GGAAGGTGCT TTTATTTCAC CCCTGGTGAA 1980
   ACTAGGGGAG CTAATTTTTT TAAACATGAT TTTTGTCCCC CTTGAACCGC CGGCCTGGAC 2040
   TACGTTTCCC AGCAGCCCGT GCTCAAGACT ACGGGTGCCT GCAGGCGGTC AGCGTCGTTT 2100
   GCGACGGCGC AGACGCGGTG CGGGCGGCGG ACGGGCGGGC GCTTCGCCGT TTGAATTGCT 2160
   GCGGGCCCGG GCCCTCACCT CACCTGAGGT CCGGCCGCCC AGGGGTGCGC TATGCCGTCG 2220
   GGAGGTGACC AGTCGCCACC GCCCCCGCCT CCCCCTCCGG CGGCGGCAGC CTCGGATGAG 2280
   GAGGAGGAGG ACGACGGCGA GGCGGAAGAC GCCGCGCCGT CTGCCGAGTC GCCCACCCCT 2340
   CAGATCCAGC AGCGGTTCGA CGAGCTGTGC AGCCGCCTCA ACATGGACGA GGCGGCGCGG 2400
   CCCGAGGCCT GGGACAGCTA CCGCAGCATG AGCGAAAGCT ACACGCTGGA GGTGCGCTCG 2460
   C 2461
(2) INFORMATION FOR SEQ ID NO:114:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:114:
      ACCTCAGGTG AGGTGAGGGC CCGG 24
(2) INFORMATION FOR SEQ ID NO:115:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:115:
      GTGTGCCATT TATGTGATGG CAAAG 25
(2) INFORMATION FOR SEQ ID NO:116:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: other nucleic acid
   (xi) SEQUENCE DESCRIPTION: SEQ ID NO:116:
      GTATACCATT TAGCAGCTGT CCGCC 25